(19) Europäisches Patentamt / European Patent Office / Office européen des brevets

(11) EP 1 690 937 B1

(12) **EUROPEAN PATENT SPECIFICATION**

(45) Date of publication and mention of the grant of the patent:
**17.10.2012 Bulletin 2012/42**

(21) Application number: **04793197.7**

(22) Date of filing: **29.10.2004**

(51) Int Cl.:
***C12N 15/09*** *(2006.01)* ***C12N 5/16*** *(2006.01)*
***A61K 39/00*** *(2006.01)* ***A61K 48/00*** *(2006.01)*
***A61P 35/00*** *(2006.01)*

(86) International application number:
**PCT/JP2004/016089**

(87) International publication number:
**WO 2005/042737 (12.05.2005 Gazette 2005/19)**

(54) **METHOD OF CONSTRUCTING TRANSGENIC DENDRITIC CELL**

VERFAHREN ZUR KONSTRUKTION EINER TRANSGENEN DENDRITISCHEN ZELLE

METHODE DE CONSTRUCTION D'UNE CELLULE DENDRITIQUE TRANSGENIQUE

(84) Designated Contracting States:
**AT BE BG CH CY CZ DE DK EE ES FI FR GB GR HU IE IT LI LU MC NL PL PT RO SE SI SK TR**

(30) Priority: **04.11.2003 JP 2003374808**
**24.06.2004 JP 2004187028**

(43) Date of publication of application:
**16.08.2006 Bulletin 2006/33**

(73) Proprietor: **Dnavec Research Inc.**
**Tsukuba-shi,**
**Ibaraki 305-0856 (JP)**

(72) Inventors:
- **OKANO, Shinji**
**Fukuoka-shi,**
**Fukuoka 812-0041 (JP)**
- **YONEMITSU, Yoshikazu**
**Fukuoka-shi,**
**Fukuoka 813-0043 (JP)**
- **SUEISHI, Katsuo**
**Fukuoka-shi,**
**Fukuoka 815-0073 (JP)**
- **SHIBATA, Satoko**
**Fukuoka-shi,**
**Fukuoka 812-0061 (JP)**
- **HASEGAWA, Mamoru,**
**c/o DNAVEC RESEARCH INC.**
**Tsukuba-shi,**
**Ibaraki 305-0856 (JP)**

(74) Representative: **Vossius & Partner**
**Siebertstrasse 4**
**81675 München (DE)**

(56) References cited:
**EP-A1- 1 186 667**

- **JIN CH ET AL: "Recombinant Sendai virus provides a highly efficient gene transfer into human cord blood-derived hematopoietic stem Cells" GENE THERAPY, MACMILLAN PRESS LTD., BASINGSTOKE, GB, vol. 10, no. 3, February 2003 (2003-02), pages 272-277, XP002983185 ISSN: 0969-7128**
- **CREMER I ET AL: "Inhibition of human immunodeficiency virus transmission to CD4+ T cells after gene transfer of constitutively expressed interferon beta to dendritic cells" HUMAN GENE THERAPY, vol. 11, no. 12, 10 August 2000 (2000-08-10), pages 1695-1703, XP002406342 ISSN: 1043-0342**
- **STEITZ J ET AL: "Depletion of CD25+ CD4+ T cells and treatment with tyrosinase-related protein 2-transduced dendritic cells enhance the interferon alpha-induced, CD8+ T-cell-dependent immune defense of B16 melanoma" CANCER RESEARCH, AMERICAN ASSOCIATION FOR CANCER RESEARCH, BALTIMORE, MD, US, vol. 61, no. 24, 15 December 2001 (2001-12-15), pages 8643-8646, XP002396933 ISSN: 0008-5472**
- **STEINMAN R M: "DENDRITIC CELLS AND IMMUNE-BASED THERAPIES" EXPERIMENTAL HEMATOLOGY, NEW YORK, NY, US, vol. 24, no. 8, July 1996 (1996-07), pages 859-862, XP000999716 ISSN: 0301-472X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 690 937 B1

- **JIN, CH. ET AL.: 'Recombinant Sendai virus provides a highly efficient gene transfer into human cord blood-derived hematopoietic stem Cells' GENE THER. vol. 10, no. 3, February 2003, pages 272 - 277, XP002983185**
- **JONULEIT, H. ET AL.: 'Efficient transduction of mature CD83+ dendritic cells using recombinant adenovirus suppressed T cell stimulatory capacity' GENE THER. vol. 7, no. 3, 2000, pages 249 - 254, XP002983186**
- **SUMIMOTO, H. ET AL.: 'Rapid and efficient generation of lentivirally gene-modified dendritic cells from DC progenitors with bone marrow stromal cells' J IMMUNOL METHODS vol. 271, no. 1-2, 2002, pages 153 - 165, XP004393934**
- **LUNDQVIST, A. ET AL.: 'Nonviral and viral gene transfer into different subsets of human dendritic cells yield comparable efficiency of transfection' J IMMUNOTHER. vol. 25, no. 6, 2002, pages 445 - 454, XP002983187**
- **OKANO, S. ET AL.: 'Recombinant Sendai virus vectors for activated T lymphocytes' GENE THER. vol. 10, no. 16, August 2003, pages 1381 - 1391, XP002973825**
- **SASAKI, K. ET AL.: 'Efficient and stable Sendai virus-mediated gene transfer into primate embryonic cells with pluripotency preserved' GENE THER. vol. 10, 2003, pages 1381 - 1391, XP002973825**


Remarks:

The applicant has requested a correction according to Rule 88 EPC.

## Description

### Technical Field

The present invention relates to a method for introducing genes into dendritic cells. The methods of the present invention can be used in the production of vaccines against cancers, infectious diseases, and the like.

### Background Art

The dendritic cell (DC) is one of the antigen-presenting cells (APCs) present in peripheral blood, skin, lymphatic organs, and thymus, and is widely distributed in lymphatic and non-lymphatic tissues (see Steinman, R. M., Ann. Rev. Immunol. 9:271 (1991); Banchereau, J.B. and Steinman R.M., Nature 392:245 (1998)). The dendritic cell has strong antigen-presenting ability and expresses antigen peptides on class I and II on the dendritic cell surface, which activate CD4 and CD8 T cells, respectively. Through this activation, the cell induces an *in vivo* immune response against specific antigens (e.g., antigens of pathogenic microorganisms, tumor-related antigens, transplantation antigens, *etc*.).

Gene alterations in dendritic cells produce a variety of clinically beneficial effects. For example, the production and use of a mature dendritic cell expressing a molecule (CD80, CD86, and the like) that induces a costimulatory signal required for T cell response enables strong activation of adaptive immunity against an antigen (for example, vaccines against viruses and tumors). Alternatively, immune tolerance to an antigen (for example, acquired insensitivity to transplantation antigens and antigens causing autoimmune diseases) can be induced by using dendritic cells devoid of a costimulatory signal-inducing molecule or a dendritic cell expressing a molecule that gives suppressive stimulus. Reported examples of such applications include: immunostimulation by introducing the interleukin (IL)-12 gene into dendritic cells (Gene Therapy 7:2113-2121 (2000)), and removal of antigen specific T cells by introducing the Fas ligand gene into dendritic cells (J Immunol. 2000: 164; 161-167). With respect to tumor immunotherapy, immunity against tumor can be induced through gene transfer of a tumor antigen into dendritic cells.

Non-Patent Document 1: Steinman, R. M., 1991, Ann. Rev. Immunol. 9:271
Non-Patent Document 2: Banchereau, J.B. and R.M. Steinman, 1998, Nature 392:245
Non-Patent Document 3: Akiyama, Y. et al., 2000, Gene Therapy, 7: 2113-2121
Non-Patent Document 4: Min, W.P., 2000, J. Immunol., 164: 161-167
Non-Patent Document 5: Hsu, F.J. et al., 1996, Nat. Med. 2, 52-58
Non-Patent Document 6: Nestle, F.O., et al., 1998, Nat. Med. 4, 328-332
Non-Patent Document 7: Camporeale, A., et al., 2003, Cancer. Res. 63, 3688-3694
Non-Patent Document 8: Bon, L. A., et al., 2003, Nat. Immunol. 4, 1009-1015
Non-Patent Document 9: Xia, D.J., et al., 2002, Gene Therapy, 9, 592-601
Non-Patent Document 10: Mullins, D.W. et al., 2003, J. Exp. Med. 198, 1023-1034
Non-Patent Document 11: Okada, T. et al., 2003, Gene Therapy 10, 1891-1902
Non-Patent Document 12: Nakahara, S. et al., 2003, Cancer Res. 63, 4112-4118
Non-Patent Document 13: Teitz-Tennenbaum, S. et al., 2003, Cancer Res. 63, 8466-8475
Non-Patent Document 14: Imboden, M. et al., 2001, Cancer Res. 61, 1500-1507
Non-Patent Document 15: Goldszmid, R. S. et al., 2003, J. Immunol. 171, 5940-5947
Non-Patent Document 16: Strome, S.E. et al., 2002, Cancer Res. 62, 1884-1889

### Disclosure of the Invention

Problems to Be Solved by the Invention

The present invention provides a method for producing a mature dendritic cell, consisting of contacting a Sendai virus vector with a $CD11c^+$, HLA-class $II^+$, $CD1a^+$, $CD3^-$, $CD19^-$, $CD20^-$, $CD56^-$, $CD14^-$ and $CD15^-$ immature myeloid dendritic cell, thereby inducing maturation of the immature dendritic cell only by contacting with the Sendai virus vector.

The present disclosure also provides a method for producing gene transferred dendritic cells. Furthermore, the present disclosure provides a use of the dendritic cells that are introduced with genes according to the methods disclosed herein. Moreover, the present disclosure provides dendritic cells that are introduced with genes by the methods disclosed herein. In addition, the present disclosure provides a viral vector for gene transfer into dendritic cells. The present disclosure also provides a method for activating dendritic cells.

Means to Solve the Problems

The present inventors discovered that Sendai virus vector is an excellent vector for gene transfer into dendritic cells. Sufficient gene transfer efficiency can be achieved through only a short contact time between the minus-strand RNA viral vector and dendritic cells, and the expression was detectable for a long period of time. Furthermore, dendritic cells can be activated through mere infection with the minus-strand RNA viral vector.

Although gene transfer into dendritic cells is expected to be applicable to various types of immunotherapy, previous techniques for gene transfer into dendritic cells were complicated and/or their introduction efficiency was unsatisfactory. The present invention has demonstrated that foreign genes can be introduced into dendritic cells by a very simple procedure using a

Sendai virus vector. Efficient gene delivery into dendritic cells can be achieved by the method of the present invention, and thus the method is expected to be applicable to gene alteration of dendritic cells for immunotherapy.

Specifically, the present invention relates to a method for producing a mature dendritic cell as described in each of the claims.

More specifically, the present invention relates to:

(1.) A method for producing a mature dendritic cell, consisting of contacting a Sendai virus vector with a $CD11c^+$, HLA-class $II^+$, $CD1a^+$, $CD3^-$, $CD19^-$, $CD20^-$, $CD56^-$, CD $14^-$ and CD $15^-$ immature myeloid dendritic cell, thereby inducing maturation of the immature dendritic cell only by contacting with the Sendai virus vector.

(2.) The method according to (1), wherein the produced mature dendritic cell is a gene transferred mature dendritic cell.

(3.) The method according to (1), wherein the vector comprises a cytokine gene.

(4.) The method according to (3), wherein the cytokine is interferon $\beta$.

(5.) The method according to(1), wherein the cell is a human cell.

Effects of the Invention

Dendritic cells have high ability for immune induction. Thus, dendritic cell (DC) vaccines useful for immunotherapy against cancers, infectious diseases, and the like can be produced by introducing a desired antigen gene or an immune activating gene into dendritic cells through the present method. For example, to present tumor antigens on dendritic cells for tumor immunotherapy, a method of mixing dendritic cells with the lysate of tumor cells, a peptide pulse method, a method of introducing tumor antigen genes into dendritic cells, and the like may be used. Among them, the method of introducing tumor antigen genes into dendritic cells can be expected to prolong the duration of *in vivo* tumor antigen presentation as compared with the tumor lysate and peptide pulse methods, and also has the advantage of being free from HLA restriction (in the case of peptide: a certain peptide derived from an antigen is used; however, due to the requirement for the binding with HLA, the region of the antigen used for the peptide varies depending on the type of HLA).

The liposome method, electroporation, and the like are available to introduce plasmids as vectors for introducing genes into dendritic cells. However, these methods are considered to be impractical due to their low introduction efficiency (Cancer Gene Ther 4:17-25s (1997)). Practical vectors include the following three kinds of vectors:

(i) adenoviral vectors (J. Immunotherapy 25:445-454 (2002); Gene Therapy 7:249-254 (2000)),
(ii) retroviral vectors (J. Leuko. Biol., 263-267 (1999); Br. J. Haematol. 108:817-824 (2000)), and
(iii) lentiviral vectors (J. Gene Med. 3:311-320 (2001); J. Immunol. Meth. 153-165 (2002); Mol. Ther. 283-290 (2002); Cancer Gene Therapy 9:715-724 (2002)).

Of the above, the retroviral vectors of (ii) can only be introduced into cells of the growth phase. In addition, these vectors require cytotoxic reagents, such as polybrene, that assist their introduction and their introduction is substantially time consuming. Therefore, they are disadvantageous in that they tend to lower the cell viability. Furthermore, the introduction efficiency of these vectors into dendritic cells induced from peripheral blood is much worse. Thus, in general, the differentiation of the cells to dendritic cells is induced after the introduction of the vectors into CD34-positive cells. Since this method requires bone marrow cells, cord blood, or peripheral blood immobilized with G-CSF, it is very invasive to patients. In addition, the introduction of retroviral vectors into dendritic cells fails to elevate the activation state of dendritic cells. Furthermore, retroviral vectors integrate viral nucleic acids into the genome of the cells, which raises concerns about damage to the genome. Moreover, the introduction efficiency is generally low, and thus requires selection of the vector-transferred cells via sorting or the like.

Vector introduction using the lentiviral vectors of (iii), like retroviral vectors, is very time consuming. This, in turn, can lower cell viability. The lentivirus is also known to transfer genes into resting cells. However, in general, due to postentry restriction, the efficiency achieved by the introduction into dendritic cells that are differentiated to some extent and have lost their proliferating activity is very low (several %) (J. Virol. 75; 5448-5456). Furthermore, the introduction efficiency into dendritic cells induced from peripheral blood is much lower. Therefore, the lentiviral vectors can only be used through a method wherein dendritic cells are obtained *in vitro* after the introduction of vectors into CD34 positive stem cells; this method is quite invasive to patients since it requires bone marrow cells, cord blood, or peripheral blood immobilized with G-CSF. Recent vector modification techniques attempt to overcome this problem, and, today, in the case of SIV, gene transfer into peripheral blood-derived monocytes and differentiated dendritic cells can be achieved by retaining vpx (which promotes nuclear localization of proviral DNA) in the helper construct or by inserting a DNA-flap sequence (which also promotes nuclear localization of proviral DNA) with HIV (Mol. Ther. 283-290 (2002)). However, it is still necessary to infect the virus at an early stage of the differentiation of monocytes to dendritic cells and therefore, the differentiation of the dendritic cells may be disturbed by the inserted gene. In addition, the introduction efficiency varies from donor to donor. Furthermore, the introduction of vector cannot elevate the activation state of the dendritic cells. Similarly to the retroviral vector described above, use of the lentiviral vectors involve risks of genome damage and development of malignant tumors, which is a barrier to practical use of the vector at present. Moreover, due to the low introduction efficiency, selection is generally required or higher titers of the vectors are required to improve the introduction efficiency, which hampers the clinical application of the vectors. In addition, the vector requires auxiliary manipulations (such as centrifugation) or assisting reagents (such as polybrene), as well as longer reaction time due to the complicated introduction procedures. The cost needed for vector purification is an additional problem.

Meanwhile, due to the introduction efficiency (about 80%) and its ability to directly transfer genes into differentiated cells, the adenovirus of (i) is the currently preferred vector for gene transfer into dendritic cells. However, the introduction efficiency is time-dependent and it requires about 72 hours to gain maximal introduction efficiency(J. Immunotherapy 25:445-454 (2002)). Furthermore, this vector requires 10 to 100 times higher titer as compared with the minus-strand RNA virus. A more critical problem is that this vector, at MOIs allowing high gene transfer efficiency, has an immunosuppressive effect, which attenuates the mixed lymphocyte reaction (MLR) of allo T cells (in particular, at high DC:T ratios) (Gene Therapy 2000; 7; 249-254). In addition, due to dilution of episome, it is sometimes difficult to perform the step for differentiating dendritic cells from stem cells, such as CD34 positive cells, after gene transfer.

Requirements to achieve gene introduction into dendritic cells include high introduction efficiency, technical stability for gene transfer, convenience, clinical safety, and maintenance of the T cell activating ability of the dendritic cells. However, none of the presently available vectors satisfy all these requirements. In contrast, when the Sendai virus vector was used, a very short contact period resulted in a gene introduction efficiency of nearly 100% and the suppression of allo T cell response was relatively mild to retain the T cells stimulating ability of the cell. Thus, the Sendai virus vector appears to meet all requirements described above, considering that the vector has the ability to express proteins in the cytoplasm, is safe, and requires neither nuclear transfer nor gene insertion to the genome. The gene transfer of the vectors of (i) to (iii) described above do not alter the activation state of dendritic cells. In contrast, the gene transfer of the Sendai virus vector induces dendritic cell activation, and thus, when used for immunostimulation (e.g., tumor immunity, *etc*.), the post-transfer step for the activation with cytokines and the like can be omitted, which is expected to contribute to maintenance of cell viability, reduction in cost, and further reduction in the time required for *ex vivo* manipulation. It was also herein confirmed that activated T cells, in particular, tumor specific cytotoxic T cells and the like, required for T cell transfer therapy could be efficiently and easily induced *ex vivo* in a short period by using dendritic cells gene transferred with the Sendai virus vector. In addition, comparable to the lentiviral vectors, when dendritic cells were differentiated after the gene transfer of the vector into stem cells, the introduction efficiency reached nearly about 70%. These characteristics expand the scope of clinical applications of the Sendai virus vector.

## Brief Description of the Drawings

Fig. 1 depicts graphs showing phenotypes of dendritic cells derived from mononuclear cells in monocyte-enriched peripheral blood cells. Viable cells recognized by PI were gated, and the expression of CD11c and HLA-class II (DR, DP, and DQ) was observed using anti-CD11c-PE-conjugated antibody and anti-HLA-class II (DR, DP, and DQ) FITC-conjugated antibody (the left matrix). Furthermore, a gate was selected for cells positive for both CD11c and HLA-class II (DR, DP, and DQ), and the expression levels detected with: (1) anti-CD14-APC-conjugated antibody; (2) anti-CD1a-APC-conjugated antibody; and (3) anti-CD80-biotin-conjugated antibody (secondarily stained with streptavidin-APC) relative to that of CD11c are shown in dot plots (the three matrices on the right). In the Examples, "Class II" indicates a result obtained using an antibody recognizing all of HLA-DR, DQ, and DP, and "HLA-DR" indicates a result obtained using an antibody specifically recognizing HLA-DR.

Fig. 2 depicts graphs showing the expression of GFP and costimulatory molecules in DCs introduced with SeV-GFP.

Fig. 3 depicts graphs showing the introduction efficiency of SeV-GFP into human monocyte-derived dendritic cells and the activation of the dendritic cells (day 2 after infection).
Fig. 4 depicts graphs showing the introduction efficiency of SeV-GFP into human monocyte-derived dendritic cells and the activation of the dendritic cells (day 4 after infection).
Fig. 5 depicts graphs showing the introduction efficiency of SeV-GFP into human monocyte-derived dendritic cells and the activation of the dendritic cells (day 8 after infection).
Fig. 6 depicts a graph showing alterations in DC count after SeV-GFP introduction.
Fig. 7 depicts graphs showing the duration of GFP expression after SeV-GFP Fig. 8 depicts graphs showing the effect of LPS stimulation on the introduction efficiency of SeV-GFP into human DCs.
Fig. 9 depicts graphs showing the effect of LPS stimulation on the introduction efficiency of SeV-GFP into human DCs.
Fig. 10 depicts graphs showing the result of examination on the incubation time for gene transfer into DCs.
Fig. 11 depicts graphs showing gene transfer into DCs derived from cord blood.
Fig. 12 depicts graphs showing gene transfer into DCs derived from cord blood.
Fig. 13 depicts graphs showing the expression of costimulatory molecules after gene transfer (as compared with LPS stimulation).
Fig. 14 depicts graphs showing the expression of costimulatory molecules after gene transfer (as compared with LPS stimulation).
Fig. 15 depicts graphs showing the expression of costimulatory molecules after gene transfer (as compared with LPS stimulation).
Fig. 16 depicts graphs showing the phagocytic ability after gene transfer.
Fig. 17 depicts graphs showing the phagocytic ability after gene transfer.
Fig. 18 depicts graphs showing cytokine production in monocyte-derived DCs after the introduction of the minus-strand RNA viral vector.
Fig. 19 depicts graphs showing the expression of marker proteins on the dendritic cells after introduction of the minus-strand RNA viral vector.
Fig. 20 depicts graphs showing the expression of marker proteins on the dendritic cells after introduction of the minus-strand RNA viral vector.
Fig. 21 depicts graphs showing the allo-T-cell stimulating ability of DCs introduced with SeV GFP.
Fig. 22 depicts the results of *in vitro* induction of MART-1 specific CTLs by introducing the minus-strand RNA viral vector.
Fig. 23 depicts the growth curve for subcutaneously injected B16 melanoma cells.
Fig. 24 depicts the results of $^{51}Cr$ release assay for YAC-1 target cells.
Fig. 25 depicts the results of $^{51}Cr$ release assay for TRP2 peptide + EL-4.

## Exemplary Mode for Carrying Out the Invention

The present invention provides a method for producing a mature dendritic cell, consisting of contacting a Sendai virus vector with a $CD11c^+$, HLA-class $II^+$, $CD1a^+$, $CD3^-$, $CD19^-$, $CD20^-$, $CD56^-$, $CD14^-$ and $CD15^-$ immature myeloid dendritic cell, thereby inducing maturation of the immature dendritic cell only by contacting with the Sendai virus vector.

This method may include the step of contacting a Sendai virus vector carrying a gene desired to be introduced with said immature dendritic cell. The present inventors discovered that the Sendai virus vector was capable of introducing a gene into dendritic cells with very high efficiency. In addition, the introduction of the vector activated the dendritic cell leading to differentiation into mature dendritic cell without cytokine stimulation. The resulting mature dendritic cell retained the ability to activate T cells. The present method is useful for the presentation of desired antigens by dendritic cells, or to express desired cytokines or other physiologically active factors in the dendritic cells. Dendritic cells that are genetically modified by the present method have high ability to activate the immune system, and can be suitably used to prevent or treat infectious diseases, cancers, and other desired diseases on which immune induction is expected to cause beneficial effects. The contact between the vector and dendritic cells can be achieved *in vivo* or *in vitro,* for example, in a desired physiological aqueous solution, such as culture solution, physiological saline, blood, blood plasma, serum, and body fluid.

The introduction efficiency of the Sendai virus vector is significantly higher in non-activated (immature) dendritic cells than in mature dendritic cells. Thus, the Sendai virus vector is contacted with immature dendritic cells as defined in the claims or to mix the vector with a cell fraction containing such immature dendritic cells. Such methods are also included in the method of the present disclosure for introducing genes into dendritic cells. Dendritic cells are activated by the contact with bacteria, lipopolysaccharide (LPS), double-stranded RNA, or the like. When dendritic cells to be introduced with genes are separately activated by such a method, the vector may be introduced after activation. However, to prevent the reduction in the efficiency of vector introduction, the activation treatment is preferably carried out not before the vector introduction but after gene transfer using the Sendai virus vector (or simultaneous with the contact of the Sendai virus vector to the dendritic cells).

The achievement of highly efficient gene delivery through simple techniques is an important superiority of the Sendai virus vector-mediated gene delivery to the dendritic cells. The gene delivery into dendritic cells mediated by retroviral vectors and the like has low efficiency, and sometimes requires a toxic agent, such as polybrene, to stimulate gene transfer. On the other hand, superior gene delivery can be achieved with the Sendai virus vector, without any requirement for a special agent, by simply adding the vector to a solution containing immature dendritic cells as defined in the claims. Furthermore, the Sendai virus vector mediated gene delivery into dendritic cells can achieve the highest efficiency within a very short exposure time (for 30 minutes or less). Considering clinical situations, these characteristics simplify *ex vivo* and *in vivo* genetic alterations of dendritic cells, and can minimize manipulation-dependent adverse effects, such as loss of cell viability.

For the contact of the vector with dendritic cells, MOI (multiplicity of infection: the number of infecting viruses per cell) is preferably within the range of 1 to 500, more preferably within the range of 2 to 300, even more preferably within the range of 3 to 200, still more preferably within the range of 5 to 100, and yet more preferably within the range of 7 to 70. The contact between the vector and dendritic cells requires only a short time, which may be, for example, 1 minute or longer, preferably 3 minutes or longer, 5 minutes or longer, 10 minutes or longer, or 20 minutes or longer, for example, within the range of about 1 to 60 minutes, more specifically within the range of about 5 to 30 minutes. Of course, the contact time may be longer, for example, for several days or longer. The contact can be achieved *in vivo* or *ex vivo.* For example, the present method is suitably used in *ex vivo* gene transfer where immature dendritic cells as defined in the claims removed from the body are contacted *ex vivo* with the minus-strand RNA viral vector, and returned into the body after vector introduction.

One of the characteristics of the present method is the sustained long-term expression of introduced gene in dendritic cells after the gene transfer. According to the present method, the expression of the introduced gene is detected in the dendritic cells for 2 days or more, for example, for 3 days or more, 5 days or more, 10 days or more, 14 days or more, 30 days or more, 50 days or more, and even 60 days or more after the infection of the vector into the cells.

The present invention also involves selectively introducing a gene into immature dendritic cells, defined in the claims, which includes the step of allowing the Sendai virus vector carrying the gene to coexist with a cell population composed of mature and immature dendritic cells. The phrase "selectively ... into immature dendritic cells" means that the gene is introduced into immature dendritic cells at a significantly high rate as compared with mature dendritic cells. Specifically, the ratio of the vector-introduced immature dendritic cells to the total immature dendritic cells is significantly higher than the ratio of the vector-introduced mature dendritic cells to the total mature dendritic cells. For example the present disclosure provides a method which includes the step of adding a Sendai virus vector carrying a desired gene to be introduced to a cell population composed of mature and immature dendritic cells. Since the Sendai virus vector preferentially transfers genes into immature dendritic cells as compared to mature dendritic cells, a gene can be selectively introduced into immature dendritic cells through this method.

A dendritic cell (DC) is a cell which takes a dendritic morphology in the mature state and has the ability to activate T cells by presenting an antigen. The dendritic cells include a group of bone marrow-derived cells with dendritic morphology distributed in various organs and tissues in the body, and a group of cells resulting from *in vitro* differentiation of bone marrow- or blood-derived stem cells using cytokines or the like, that are equivalent to the cells with dendritic morphology distributed in various organs and tissues in the body. Specifically, the dendritic cells include, for example, lymphocytic dendritic cells (including cells which induce Th2 or immune tolerance), myeloid dendritic cells (generally used dendritic cells, including immature and mature dendritic cells), Langerhans cells (dendritic cells important as antigen-presenting cells in the skin), interdigitating cells (distributed in the lymph nodes and spleen T cell region, and believed to function in antigen presentation to T cells), and follicular dendritic cells (important as antigen-presenting cells for B cells; the cells present antigens to B cells by presenting antigen-antibody complexes or antigen-complement complexes on the surface via the antibody receptor or the complement receptor). The dendritic cells used in the method of the invention express CD11c and, preferably, highly express MHC class I and class II.

An immature myelord dendritic cell used in the method of the invention is a cell with dendritic morphology and that is positive for the surface markers CD11c, HLA-class II (HLA-DR, -DP, or -DQ), and CD1a and is devoid of the expression of T cell marker (CD3), B cell markers (CD 19, CD20), NK cell marker (CD56), neutrophil marker (CD15), and monocyte marker (CD14).

The immature dendritic cells for use in the present invention refer to dendritic cells having low T cell activating ability. Specifically, the immature dendritic cells may have an antigen-presenting ability that is lower than 1/2, preferably lower than 1/4 of that of dendritic cells which maturation had been induced by adding LPS (1 $\mu$g/ml) and culturing for two days. The antigen-presenting ability can be assayed, for example, by allo T cell-activating ability (e.g., a mixed lymphocyte test: allo T cells and dendritic cells are cultured in a mixed culture with a T cell:dendritic cell ratio of 1:10, or preferably with varied ratios; $^3$H-thymidine is added 8 hours before terminating cultivation, and the T cell growth capacity is assayed based on the amount of $^3$H-thymidine incorporated into the DNA of the T cells. See Fig. 21; Gene Therapy 2000; 7; 249-254) or by the ability to induce specific cytotoxic T cells (CTLs) using a peptide (e.g., a known class I-restricted peptide of a certain antigen is added to dendritic cells; the dendritic cells are co-cultured with T cells obtained from peripheral blood of the same healthy donor from whom the dendritic cells had been obtained (with 25 U/ml or preferably 100 U/ml of IL-2 on day 3 or later) (preferably stimulated three times during 21 days, more preferably twice during 14 days by dendritic cells); the resulting effector cells are co-cultured with $^{51}$Cr-labeled target cells (peptide-restricted class I positive tumor cells) at a ratio of 20:1, 10:1, 5:1, or 2.5:1, preferably 100:1, 50:1, 25:1, or 12.5:1, for four hours; and $^{51}$Cr released from the target cells is quantified. See Fig. 22; Arch Dermatol Res 292:325-332 (2000)). Furthermore, the immature dendritic cells preferably have phagocytic ability for antigens, and more preferably show low (for example, significantly low as compared to mature DCs induced by LPS as described above) or negative expression of receptors that induce the costimulation for T cell activation. On the other hand, the mature dendritic cells refer to dendritic cells that have strong antigen-presenting ability for T cell activation or the like. Specifically, the mature dendritic cells may have an antigen-presenting ability that is half or stronger, preferably equivalent to or stronger than the antigen-presenting ability of dendritic cells in which maturation has been induced by adding LPS (1 μg/ml) and culturing for two days. Furthermore, the mature dendritic cells preferably have weak or no phagocytic ability for antigen, and more preferably show high expression of receptors that induce the costimulation for T cell activation. The activation of dendritic cells refers to the transition from immature to mature dendritic cell; and the activated dendritic cells encompass mature dendritic cells and dendritic cells in the process of the transition, wherein the expression of CD80 and CD86 that induce costimulatory signals are elevated upon the activating stimuli. In CD11c positive dendritic cells, CD83 positivity serves as an indicator of mature dendritic cells.

For example, mature dendritic cells may preferably be cells whose expression of CD40, CD80, CD86, and HLA-class II is strongly positive. More preferably, mature dendritic cells express CD83. An immature dendritic cell can be distinguished from a mature dendritic cell, for example, using markers selected from the group consisting of CD80, CD83, and CD86. The immature dendritic cell is weakly positive for these markers, preferably negative, while the mature dendritic cell is positive.

As described above, immature dendritic cells generally have a high phagocytic ability. When dendritic cells are added with LPS (1 μg/ml) and cultured for two days, they become activated and their phagocytic ability is reduced. The phagocytic ability can be detected by measuring the amount of small molecules taken up into dendritic cells or the proportion of uptaking cells. The phagocytic ability is preferably determined by the amount of small molecules taken up into dendritic cells. For example, using colored beads with a size of about 1 μm, the uptake of beads into dendritic cells can be measured. Quantitation is performed by subtracting the positive background at 4°C. A high phagocytic ability indicates an ability wherein the amount of small molecules taken up into dendritic cells is 4 times or more, more preferably 5 times or more, and even more preferably 6 times or more than that taken up into dendritic cells stimulated with LPS (1 μg/ml) for two days as described above. Alternatively, the proportion of cells taking up small molecules is twice or more, and more preferably 3 times or more. A low phagocytic ability is indicated when the amount of small molecules taken up into dendritic cells is less than four times, more preferably less than twice, and more preferably less than 1.5 times to that taken up into dendritic cells stimulated with LPS (1 μg/ml) for two days. Alternatively, when measured as the proportion of cells that take up small molecules, the proportion is less than twice, and more preferably less than 1.5 times.

Discrimination of mature dendritic cells is routinely performed by those skilled in the art, and the respective markers described above and methods for measuring their expression are also well known to those skilled in the art. For example, CD11c is an adhesion glycoprotein of about 150 kD (p150, integrin alpha chain). CD11c binds to CD18 to form a CD11c/CD18 complex, which is capable of binding to fibrinogen and has been reported to function as a receptor for iC3b and ICAM-1. In addition, it has been reported that CD11c/CD18 can function as an adhesion molecule that binds to receptors on stimulated epithelia (Knapp, W. et al., eds., 1989, Leucocyte Typing IV: White Cell Differentiation Antigens, Oxford University Press, New York; Barclay, N.A. et al., eds., 1993, The Leucocyte Antigen Facts Book, CD11 Section, Academic Press Inc., San Diego, California, p. 124; Stacker, S.A. and T.A. Springer, 1991, J. Immunol. 146:648).

CD1a is a polypeptide of about 49 kD, which binds to beta2 microglobulin. CD1a is structurally similar to an MHC class I antigen and is assumed to function in antigen presentation (Knapp, W. et al., eds., 1989, Leucocyte Typing IV: White Cell Differentiation Antigens, Oxford University Press, New York; Schlossman, S. et al., eds., 1995, Leucocyte Typing V: White Cell Differentiation Antigens. Oxford University Press, New York; Hanau, D. et al., 1990, J. Investigative Dermatol. 95: 503; Calabi, F. and A. Bradbury., 1991., Tissue Antigens 37: 1).

CD14 is a glycosylphosphatidylinositol (GPI)-anchored single-chain glycoprotein of 53 to 55 kD expressed in dendritic reticulum cells and some types of Langerhans cells. CD14 was identified as a surface receptor having high affinity to a complex of LPS and serum LPS-binding protein (LPB) (McMichael, A.J. et al., eds., 1987, Leucocyte Typing III: White Cell Differentiation Antigens, Oxford University Press, New York; Knapp, W. et al., eds., 1989, Leucocyte Typing IV: White Cell Differentiation Antigens, Oxford University Press, New York; Schlossman, S. et al., eds., 1995, Leucocyte Typing V: White Cell Differentiation Antigens. Oxford University Press, New York; Wright, S.D. et al., 1990, Science 249:1434).

CD40 is a type I integral membrane protein of 45 to 48 kD (type I integral membrane glycoprotein). CD40 is frequently used as a cell marker (Schlossman, S. et al., eds., 1995, Leucocyte Typing V: White Cell Differentiation Antigens. Oxford University Press, New York; Galy, A.H.M.; and H. Spits, 1992, J. Immunol. 149: 775; Clark, E.A. and J.A. Ledbetter, 1986, Proc. Natl. Acad. Sci. 83: 4494; Itoh, H. et al., 1991, Cell 66: 233; Barclay, N.A. et al., 1993, The Leucocyte Antigen Facts Book., Academic Press).

CD80 is a transmembrane glycoprotein of about 60 kD, and is a member of the Ig supergene family. CD80 is a ligand for CD28 and CD152 (CTLA-4) expressed in T cells (Schlossman, S. et al., eds., 1995, Leucocyte Typing V: White Cell Differentiation Antigens. Oxford University Press, New York; Schwarts, R.H., 1992, Cell 71: 1065; Azuma, M. et al., 1993, J. Exp. Med. 177: 845; Koulova, L. et al., 1991, J. Exp. Med. 173: 759; Freeman, G.J. et al., 1998, J. Immunol. 161: 2708; Behrens, L. et al., 1998, J. Immunol., 161(11):5943; Guesdon, J.-L. et al., 1979, J. Histochem. Cytochem. 27: 1131-1139).

CD83 is a transmembrane protein of about 45 kD, and is a member of the Ig superfamily. CD83 has a short extracellular domain of V-type Ig and a C-terminal cytoplasmic tail. CD83 is mainly expressed in follicular dendritic cells, circulating dendritic cells, interdigitating dendritic cells in lymphatic tissues, *in vitro*-produced dendritic cells, and dendritic cells of the thymus (Zhou, L-J., and T.F. Tedder, 1995, J. Immunol. 154. 3821; Zhou, L-J. et al., 1992, J. Immunol. 149: 735; Summers, K.L. et al., 1995, Clin Exp. Immunol. 100:81; Weissman, D. et al., 1995, Proc. Natl. Acad. Sci USA. 92: 826; Hart, D.N.J., 1997, Blood 90: 3245).

CD86 (B70/B7-2) is a cell surface protein of about 75 kD, which is a second ligand for CD28 and CTLA-4 and plays an important role in costimulation of T cells in early immune response (Azuma M. et al., 1993, Nature 366: 76; Nozawa Y et al., 1993, J. Pathology 169: 309; Engle, P. et al. 1994., Blood 84: 1402; Engel, P. et al., CD86 Workshop Report. In: Leukocyte Typing V. Schlossman, S.F. et al. eds., 1994, Oxford University Press; Yang, X.F. et al., 1994, Upregulation of CD86 antigen on TPA stimulated U937 cells, 1994, (abstract). American Society of Hematology, Nashville, TN; Guesdon, J.-L.et al., 1979, J. Histochem. Cytochem. 27: 1131-1139).

CCR7 is also called BLR-2, EBI-1, and CMKBR7, which is a seven-transmembrane G protein-coupled receptor, and is a receptor of the CC chemokines, MIP-3beta/Exodus 3/ELC/CCL19 and 6Ckine/Exodus 2/SLC/TCA4/CCL21 (Sallusto, F. et al., 1999, Nature 401:708-12; Lipp, M. et al., 2000, Curr. Top. Microbiol. Immunol. 251:173-9; Birkenbach, M.et al., 1993, J. Virol. 67:2209-20; Schweickart, V. L. et al., 1994, Genomics 23:643-50; Burgstahler, R. et al., 1995, Biochem. Biophys. Res. Commun. 215:737-43; Yoshida, R. et al., 1997, J. Biol. Chem. 272:13803-9; Yoshida, R. et al., 1998, J. Biol. Chem. 273:7118-22; Yoshida, R. et al., 1998, Int. Immunol. 10:901-10; Kim, C. H. et al., 1998, J. Immunol. 161:2580-5; Yanagihara, S. et al., 1998, J. Immunol. 161:3096-102).

DR, DP, and DQ exist as HLA-class II antigens, and can be collectively detected using antibodies that bind to all three antigens (Pawelec, G. et al., 1985, Human Immunology 12:165; Ziegler, A. et al., 1986, Immunobiol. 171:77). HLA-DR is one of the human MHC class II antigens, which is a transmembrane glycoprotein consisting of an alpha chain (36 kDa) and a beta subunit (27 kDa). In epidermal Langerhans cells, the protein is co-expressed with CD1a antigen. CD1a plays a principal role in cell interaction for antigen presentation (Barclay, N.A. et al., 1993, The Leucocyte Antigen Facts Book. p. 376. Academic Press).

Dendritic cells of nonhuman mammals can also be specified using the products of homologous genes of the above-described marker genes as indicators. Antibodies to such markers are commercially available, for example, from BD Biosciences (BD PharMingen), and detailed information is available at the websites of the company or its distributors.

For dendritic cell markers, also see the references by Kiertscher *et al.* and Oehler. (Kiertscher SM, Roth MD, Human CD14+ leukocytes acquire the phenotype and function of antigen-presenting dendritic cells when cultured in GM-CSF and IL-4, J. Leukoc. Biol., 1996, 59(2):208-18; Oehler, L. et al., Neutrophil granulocyte-committed cells can be driven to acquire dendritic cell characteristics., J. Exp. Med., 1998, 187(7):1019-28). For further details regarding flow cytometry, see the references by Okano *et al.* and Stites *et al.* (Okano, S. et al., Recombinant Sendai virus vectors for activated T lymphocytes. Gene Ther., 2003, 10(16):1381-91; Stites, D. et al., Flow cytometric analysis of lymphocyte phenotypes in AIDS using monoclonal antibodies and simultaneous dual immunofluorescence., Clin. Immunol. Immunopathol., 1986, 38:161-177). The expression of each of the markers may be determined, for example, using as a threshold the fluorescence intensity that makes a positive rate of 1% or less when stained with an isotype control antibody, wherein the fluorescence equal to or above the threshold is deemed positive, and the fluorescence below deemed negative.

Dendritic cells or precursor cells thereof can be prepared according to or based on known methods. For example, the cells can be isolated from blood (for example, peripheral or cord blood), bone marrow, lymph nodes, other lymphatic organs, spleen, and skin. Dendritic cells to be used in the context of the present invention are preferably obtained from blood or bone marrow. Alternatively, dendritic cells to be used in the present invention may be skin Langerhans cells, veiled cells of afferent lymphatics, follicular dendritic cells, spleen dendritic cells, and interdigitating cells of lymphatic organs. The dendritic cells used in the present invention include dendritic cells selected from the group consisting of $CD34^+$-derived dendritic cells, bone marrow-derived dendritic cells, monocyte-derived dendritic cells, splenic cell-derived dendritic cells, skin-derived dendritic cells, follicular dendritic cells, and germinal center dendritic cells. $CD34^+$-derived dendritic cells can be differentiated from hematopoietic stem cells, hematopoietic progenitor cells, or the like, obtained from cord blood, bone marrow, or the like, using granulocyte colony stimulating factor (G-CSF), granulocyte macrophage colony stimulating factor (GM-CSF), tumor necrosis factor (TNF)-alpha, IL-4, IL-13, stem cell factor (SCF), Flt-3 ligand, c-kit ligand, combinations thereof, or the like. For example, peripheral blood monocytes can be differentiated into immature dendritic cells using GM-CSF and IL-4, and then differentiated into mature dendritic cells by stimulating with TNF-alpha.

When dendritic cells are selected (or enriched) from a composition including dendritic cells and other cells, it is preferable to perform so-called negative selection which removes cells other than the dendritic cells. Through the negative selection process, precursors of DC-granulocytes (J. Exp. Med., 1998, 187: 1019-1028; Blood, 1996, 87: 4520-4530) remain and thus, it is considered that not only DCs differentiated from adhesive $CD14^+$ cells but also DCs differentiated from precursors can be recovered together. This is expected to reduce the cytotoxicity resulting from vector introduction.

For example, by removing T cells, NK cells, B cells, and the like, using antibodies specific thereto, dendritic cells can be enriched. Specifically, for example, it is preferable to obtain cells with low or negative expression of a surface marker selected from CD2, CD3, CD8, CD19, CD56, and CD66b, or any combinations thereof. More preferred are cells in which the expressions of CD2, CD3, CD8, CD19, CD56, and CD66b are all low or negative. Therefore, it is preferable to remove cells expressing these markers using antibodies against the markers (Hsu et al., Nature Med. 2:52 (1996)). The negative selection can be performed using polyvalent antibodies as shown in the Examples. Alternatively, a similar selection can also be performed using beads or the like for magnetic cell separation (MACS). The use of beads is preferred for large scale cell preparation, such as collection of mononuclear cells through blood cell separation or the like. For example, dendritic cells prepared by negative selection from monocytes that were enriched from a cell solution obtained from the body can be preferably used in the context of the present invention.

When dendritic cells differentiated from peripheral blood monocytes obtained from adhesive cells are selected before introduction of the minus-strand RNA virus, the efficiency of vector introduction is sometimes reduced. To prevent the reduction of the proportion of immature dendritic cells, before the contact with the Sendai virus vector, cell culture is preferably carried out without the step of selecting cells adhering to a solid support (for example, culture container such as culture dish or bottle); however, the dendritic cells used in the context of the present invention are not limited thereto. Specifically, the present disclosure provides a method which excludes the step of selecting cells adhered on the solid support within 24 hours before contact of dendritic cells with the Sendai virus vector. More preferably, excluded is the step of selecting cells adhered to the solid support within 2, 3, 5, or 7 days before the contact of dendritic cells with the Sendai virus vector.

The method preferably excludes the step of selecting $CD14^+$ cells before the contact with the minus-strand RNA viral vector, but it is not limited thereto. Specifically, the present invention provides a method which excludes the step of selecting $CD14^+$ cells within 24 hours before the contact of dendritic cells with the Sendai virus vector. More preferably, the method excludes the step of selecting $CD14^+$ cells within 2, 3, 5, or 7 days before the contact of dendritic cells with the Sendai virus vector.

Specific methods for isolating dendritic cells are described in, for example, Cameron et al., Science 257:383 (1992); Langhoff et al., Proc. Natl. Acad. Sci. USA 88:7998 (1991); Chehimi et al., J. Gen. Virol. 74:1277 (1993); Cameron et al., Clin. Exp. Immunol. 88:226 (1992); Thomas et al., 1993; J. Immunol. 150:821 (1993); and Karhumaki et al., Clin. Exp. Immunol. 91:482 (1993). The isolation of dendritic cells by flow cytometry is described in, for example, Thomas et al., J. Immunol. 153:4016 (1994); Ferbas et al., J. Immunol. 152:4649 (1994); and O'Doherty et al., Immunology 82:487 (1994). In addition, magnetic cell separation is described in, for example, Miltenyi et al., Cytometry 11: 231-238 (1990).

Furthermore, for example, human dendritic cells may be isolated and grown using the methods described in Macatonia et al., Immunol. 74:399-406 (1991); O'Doherty et al., J. Exp. Med. 178:1067-1078 (1993); Markowicz et al., J. Clin. Invest. 85:955-961 (1990); Romani et al., J. Exp. Med. 180:83-93 (1994); Sallusto et al., J. Exp. Med. 179: 1109-1118 (1994); Berhard et al., J. Exp. Med. 55:1099-1104 (1995); and the like. Moreover, dendritic cells can be formed from $CD34^+$ cells obtained from bone marrow, cord blood, peripheral blood, or the like and from peripheral blood-derived mononuclear cells by the method described in Van Tendeloo et al., Gene Ther. 5:700-707 (1998).

In the present invention, it is preferable to mix a Sendai virus vector with a cell fraction containing a high density of immature dendritic cells as defined in the claims.

Precursor cells refer to cells that can differentiate into dendritic cells in the presence of appropriate cytokines (specifically, G-CSF, GM-CSF, TNF-alpha, IL-4, IL-13, SCF, Flt-3 ligand, or c-kit ligand, or combinations thereof). The precursor cells are preferably differentiated into dendritic cells within 4 weeks, more preferably within 20 days, even more preferably within 18 days, and still more preferably within 16 days. Such cells include $CD34^+$ stem cells. The differentiation into dendritic cells may be achieved, for example, by culturing the cells in the presence of SCF (50 ng/ml), GM-CSF (500 U/ml), and TNF-alpha (50 ng/ml) for about 3 days, followed by culturing in the presence of SCF (50 ng/ml), GM-CSF (500 U/ml), IL-4 (250 U/ml), and TNF-alpha (50ng/ml). A cell fraction refers to a group of cells obtained through cell separation (or cell fractionation). The cell fraction may be a composition including both cells and pharmaceutically acceptable carriers. Exemplary carriers include desired solutions that can be used to suspend viable cells, such as physiological saline, phosphate buffered saline (PBS), culture medium, and serum.

Furthermore, dendritic cells to be contacted with a Sendai virus vector are immature dendritic cells as defined in the claims. In the cell fraction that includes dendritic cells to be mixed with the vector, the proportion of said immature dendritic cells to the total viable cells is, for example, 10% or more, preferably 20% or more, more preferably 30% or more, more preferably 40% or more, more preferably 50% or more, more preferably 60% or more, and more preferably 70% or more.

The method of the present invention using a Sendai virus has various advantages. For example, with the Sendai virus, activated dendritic cells can be obtained by the vector infection alone, and subsequent steps for obtaining mature dendritic cells can be omitted. Since dendritic cells must be activated for their use in immunostimulation, it is advantageous that the activation can be achieved by only the vector infection. Furthermore, utilizing this property, activated T cells, in particular cytotoxic T cells or the like, which are necessary for T cell transfer therapy, can be induced *in vitro* in a short time. CTLs cannot be induced using dendritic cells that are not introduced with the Sendai virus. According to hitherto reported characteristics of other vectors, it is difficult to induce CTLs *in vitro* by gene transfer of such other vectors alone. Thus, the Sendai virus vector has the advantage that it can activate T cells (CTL induction) merely by its introduction (see Fig. 22). In addition, the Sendai virus vector is superior to other vectors, due to the fact that it possesses all the characteristics of high introduction efficiency, stability of gene transfer, convenience, safety, and maintenance of the ability of the dendritic cells to activate T cells.

Furthermore, the Sendai virus vector is also useful for differentiating stem cells after gene transfer into dendritic cells. When dendritic cell differentiation is induced after the Sendai virus vector is introduced into stem cells, the gene transfer efficiency reaches to nearly 70%. This efficiency is comparable to those of modified retroviral and lentiviral vectors. The introduction of adenoviral vector into stem cells is difficult, due to the reduction in the expression level by the episome dilution after introduction. The Sendai virus can be applied for both methods wherein dendritic cell differentiation is performed after vector introduction into stem cells and wherein the gene is introduced into dendritic cells that have been differentiated from peripheral blood mononuclear cells.

In addition, when the MOI is set high (for example, 10 or higher, preferably 20 or higher, more preferably 30 or higher, for example, 40 or higher, or 50 or higher), the Sendai virus vector can be stably introduced into cells with an introduction efficiency of nearly 100% without any significant influence on cell cytotoxicity. Moreover, since the Sendai virus does not integrate genes into the host genome, it has the advantage of a low risk of tumor development. Herein, a minus-strand RNA virus, to which the Sendai virus belongs, refers to viruses that include a minus strand (an antisense strand corresponding to a sense strand encoding viral proteins) RNA as the genome. The minus-strand RNA is also referred to as negative strand RNA. The minus-strand RNA virus used in the present invention particularly includes single-stranded minus-strand RNA viruses (also referred to as non-segmented minus-strand RNA viruses). The "single-strand negative strand RNA virus" refers to viruses having a single-stranded negative strand [*i.e.*, a minus strand] RNA as the genome. Such viruses include viruses belonging to Paramyxoviridae (including the genera *Paramyxovirus, Morbillivirus, Rubulavirus,* and *Pneumovirus*), Rhabdoviridae (including the genera *Vesiculovirus, Lyssavirus,* and *Ephemerovirus*), Filoviridae, Orthomyxoviridae, (including Influenza viruses A, B, and C, and Thogoto-like viruses), Bunyaviridae (including the genera *Bunyavirus, Hantavirus, Nairovirus,* and *Phlebovirus*), Arenaviridae, and the like.

In addition, the minus-strand RNA viral vector is a minus-strand RNA virus-based virion with infectivity and a vehicle for introducing genes into cells. Herein, "infectivity" refers to the capability of a minus-strand RNA viral vector to maintain cell-adhesion ability and introduce a gene carried by the vector to the inside of the cell to which the vector has adhered. In a preferable embodiment, the minus-strand RNA viral vector of this invention has a foreign gene incorporated into its genomic RNA for expression. The Sendai virus vector for use in this invention may have propagation ability or may be a defective-type vector with no propagation ability. "Having propagation ability" means that when a viral vector infects a host cell, the virus is replicated in the cell to produce infectious virions.

"Recombinant virus" refers to a virus produced through a recombinant polynucleotide, or an amplification product thereof. "Recombinant polynucleotide" refers to a polynucleotide in which nucleotides are not linked at one or both ends as in the natural condition. Specifically, a recombinant polynucleotide is a polynucleotide in which the linkage of the polynucleotide chain has been artificially modified (cleaved and/or linked). Recombinant polynucleotides can be produced by using gene recombination methods known in the art in combination with polynucleotide synthesis, nuclease treatment, ligase treatment, etc. A recombinant virus can be produced by expressing a polynucleotide encoding a viral genome constructed through gene manipulation and reconstructing the virus. For example, methods for reconstructing a virus from cDNA that encodes the viral genome are known (Y. Nagai, A. Kato, Microbiol. Immunol., 43, 613-624 (1999)).

In the present invention, "gene" refers to a genetic substance, a nucleic acid having a sequence to be transcribed in a sense or antisense strand. Genes may be RNAs or DNAs. In this invention, a nucleic acid encoding a protein is referred to as a gene of that protein. Further, a gene may not encode a protein. For example, a gene may encode a functional RNA, such as a ribozyme or antisense RNA. A gene may be a naturally-occurring or artificially designed sequence. Furthermore, in the present invention, "DNA" includes both single-stranded and double-stranded DNAs. Moreover, "encoding a protein" means that a polynucleotide includes an ORF that encodes an amino acid sequence of the protein in a sense or antisense strand, so that the protein can be expressed under appropriate conditions.

The Sendai virus may be derived from natural strains, wild strains, mutant strains, laboratory-passaged strains, artificially constructed strains, or the like.

Genes harbored on a minus-strand RNA viral vector are situated in the antisense direction in the genomic RNA. Genomic RNA refers to RNA that has the function to form a ribonucleoprotein (RNP) with the viral proteins of a minus-strand RNA virus. Genes contained in the genome are expressed by the RNP, genomic RNA is replicated, and daughter RNPs are formed. In general, the genome of a minus-strand RNA virus is constituted so that the viral genes are situated in an antisense orientation between the 3'-leader region and 5'-trailer region. Between the ORFs of individual genes exists a transcription ending sequence (E sequence) - intervening sequence (I sequence) - transcription starting sequence (S sequence) that allows the RNA encoding each ORF to be transcribed as a separate cistron.

Genes encoding the viral proteins of a minus-strand RNA virus include NP, P, M, F, HN, and L genes. "NP, P, M, F, HN, and L genes" refer to genes encoding nucleocapside-, phospho-, matrix-, fusion-, hemagglutinin-neuraminidase-, and large-proteins respectively. Genes in each virus belonging to Paramyxovirinae are commonly listed as follows. In general, NP gene is also listed as "N gene."

| | | | | | | | |
|---|---|---|---|---|---|---|---|
| *Respirovirus* | NP | P/C/V | M | F | HN | - | L |
| *Rubulavirus* | NP | P/V | M | F | HN | (SH) | L |
| *Morbillivirus* | NP | P/C/V | M | F | H | - | L |

For example, the database accession numbers for the nucleotide sequences of each of the Sendai virus genes are: M29343, M30202, M30203, M30204, M51331, M55565, M69046, and X17218 for NP gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30202, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene. Examples of viral genes encoded by Sendai virus are: X00087 for N gene; AB005796 for C gene; and U06433 for HN gene. However, a number of strains are known for the virus, and genes exist that include sequences other than those cited above, due to strain variation.

The ORFs encoding these viral proteins and ORFs of the foreign genes are arranged in the antisense direction in the genomic RNAs, via the above-described E-I-S sequence. The ORF closest to the 3'-end of the genomic RNAs requires only an S sequence between the 3'-leader region and the ORF, and does not require an E or I sequence. Further, the ORF closest to the 5'-end of the genomic RNA requires only an E sequence between the 5'-trailer region and the ORF, and does not require an I or S sequence. Furthermore, two ORFs can be transcribed as a single cistron, for example, by using an internal ribosome entry site (IRES) sequence. In such a case, an E-I-S sequence is not required between these two ORFs. For example, in wild type paramyxoviruses (to which Sendai virus belongs), a typical RNA genome includes a 3'-leader region, six

ORFs encoding the N, P, M, F, HN, and L proteins in the antisense direction and in this order, and a 5'-trailer region on the other end. The viral gene orientation in the genomic RNAs for use in the present invention is not restricted, but similarly to the wild type viruses, it is preferable that ORFs encoding the N, P, M, F, HN, and L proteins are arranged in this order, after the 3'-leader region, and before the 5'-trailer region. Certain types of viruses have different viral genes, but even in such cases, it is preferable that each gene be arranged as in the wild type, as described above. In general, vectors maintaining the N, P, and L genes can autonomously express genes from the RNA genome in cells, replicating the genomic RNA. Furthermore, by the action of genes such as the F and HN genes, which encode envelope proteins, and the M gene, infectious virions are formed and released to the outside of cells. Thus, such vectors become viral vectors with propagation ability. A foreign gene to be transduced into dendritic cells may be inserted into a protein-noncoding region in this genome, as described below.

Further, a Sendai virus vector for use in this invention may be deficient in any of the wild type virus genes. For example, a viral vector that excludes the M, F, or HN gene, or any combination thereof, can be preferably used in this invention. Such viral vectors can be reconstituted, for example, by externally supplying the products of the deficient genes. Similar to wild type viruses, the viral vectors thus prepared adhere to host cells and cause cell fusion, but they cannot form daughter virions that retain the same infectivity as the original vector, because the vector genome introduced into cells is deficient in viral genes. Therefore, such vectors are useful as safe viral vectors that can only introduce genes once. Examples of genes in which the genome may be deficient are the F gene and/or HN gene. For example, viral vectors can be reconstituted by transfecting host cells with a plasmid expressing a recombinant minus-strand RNA viral vector genome deficient in the F gene, along with an F protein expression vector and expression vectors for the NP, P, and L proteins (WO00/70055 and WO00/70070; Li, H.-O. et al., J. Virol. 74(14) 6564-6569 (2000)). Viruses can also be produced, for example, using host cells that have incorporated the F gene into their chromosomes. In these proteins, the amino acid sequences do not need to be the same as the viral sequences, and a mutant or homologous gene from another virus may be used as a substitute, so long as the activity in nucleic acid introduction is the same as, or greater than, that of the natural type.

Further, vectors that include an envelope protein other than that of the virus from which the vector genome was derived, may be prepared as viral vectors used in this invention. For example, when reconstituting a virus, a viral vector including a desired envelope protein can be generated by expressing an envelope protein other than the envelope protein encoded by the basic viral genome. Such proteins are not particularly limited. A desired protein that confers an ability to infect cells may be used. Examples of such proteins include the envelope proteins of other viruses, for example, the G protein of vesicular stomatitis virus (VSV-G). The VSV-G protein may be derived from an arbitrary VSV strain. For example, VSV-G proteins derived from Indiana serotype strains (J. Virology 39: 519-528 (1981)) may be used, but the present invention is not limited thereto. Furthermore, the present vector may include any arbitrary combination of envelope proteins derived from other viruses. Preferred examples of such proteins are envelope proteins derived from viruses that infect human cells. Such proteins are not particularly limited, and include retroviral amphotropic envelope proteins and the like. For example, the envelope proteins derived from mouse leukemia virus (MuLV) 4070A strain can be used as the retroviral amphotropic envelope proteins. In addition, envelope proteins derived from MuMLV 10A1 strain may also be used (for example, pCL-10A1 (Imgenex) (Naviaux, R. K. et al., J. Virol. 70:5701-5705 (1996)). The proteins of *Herpesviridae* include, for example, gB, gD, gH, and gp85 proteins of herpes simplex viruses, and gp350 and gp220 proteins of EB virus. The proteins of *Hepadnaviridae* include the S protein of hepatitis B virus. These proteins may be used as fusion proteins in which the extracellular domain is linked to the intracellular domain of the F or HN protein. As described above, the viral vectors used in this invention include pseudotype viral vectors that include envelope proteins, such as VSV-G, derived from viruses other than the virus from which the genome was derived. If the viral vectors are designed such that these envelope proteins are not encoded in RNA genomes, the proteins will never be expressed after virion infection of the cells.

Furthermore, the viral vectors used in this invention may be, for example, vectors that include on the envelope surface thereof, proteins such as adhesion factors capable of adhering to specific cells, ligands, receptors, antibodies or fragments, or vectors that include a chimeric protein with these proteins in the extracellular domain and polypeptides derived from the virus envelope in the intracellular domain. Thus, the dendritic cell specificity of the vectors can be controlled. These proteins may be encoded in the viral genome, or supplied through the expression of genes not in the viral genome (for example, genes carried by other expression vectors, or genes in the host chromosomes) at the time of viral vector reconstitution.

Further, in the viral vectors, any viral gene contained in the vector may be modified from the wild type gene in order to reduce the immunogenicity caused by viral proteins, or to enhance RNA transcriptional or replicational efficiency, for example. Specifically, for example, modifying at least one of the replication factors N, P, and L genes, is considered to enhance transcriptional or replicational function. Furthermore, although the HN protein, which is an envelope protein, has both hemagglutinin activity and neuraminidase activity, it is possible, for example, to improve viral stability in blood if the former activity is attenuated, and infectivity can be controlled if the latter activity is modified. Further, it is also possible to control membrane fusion ability by modifying the F protein. For example, the epitopes of the F protein and/or HN protein, which can be cell surface antigenic molecules, can be analyzed, and using this, viral vectors with reduced antigenicity to these proteins can be prepared.

Furthermore, the Sendai virus vector may be deficient in one or more accessory gene. For example, by knocking out the V gene, one of the SeV accessory genes, the pathogenicity of SeV toward hosts such as mice is remarkably reduced, without hindering gene expression and replication in cultured cells (Kato, A. et al., 1997, J. Virol. 71: 7266-7272; Kato, A. et al., 1997, EMBO J. 16: 578-587; Curran, J. et al., WO01/04272, EP1067179). Such attenuated vectors are particularly useful as nontoxic viral vectors for *in vivo* or *ex vivo* gene transfer.

Minus-strand RNA viruses are excellent gene transfer vectors. They do not have DNA phase and carry out transcription and replication only in the host cytoplasm, and consequently, chromosomal integration does not occur (Lamb, R.A. and Kolakofsky, D., Paramyxoviridae: The viruses and their replication. In: Fields BN, Knipe DM, Howley PM, (eds). Fields of Virology. Vol. 2. Lippincott - Raven Publishers: Philadelphia, 1996, pp. 1177-1204). Therefore, safety issues such as transformation and immortalization due to chromosomal abberation do not occur. This characteristic of minus-strand RNA viruses contributes greatly to safety when it is used as a vector. For example, results on foreign gene expression show that even after multiple continuous passages of SeV, almost no nucletide mutation is observed. This suggests that the viral genome is highly stable and the inserted foreign genes are stably expressed over long periods of time (Yu, D. et al., Genes Cells 2, 457-466 (1997)). Further, there are qualitative advantages associated with SeV not having a capsid structural protein, such as packaging flexibility and insert gene size, suggesting that minus-strand RNA viral vectors may become a novel class of highly efficient vectors for human gene therapy. SeV vectors with propagation ability are capable of introducing foreign genes of up to at least 4 kb in size, and can simultaneously express two or more kinds of genes by adding the transcriptional units.

Further, SeV is known to be pathogenic in rodents causing pneumonia, but is not pathogenic for human. This is also supported by a previous report that nasal administration of wild type SeV does not have severely harmful effects on non-human primates (Hurwitz, J.L. et al., Vaccine 15: 533-540, 1997). These SeV characteristics suggest that SeV vectors can be applied therapeutically on humans, supporting the proposition that SeV vectors are a promising choice of gene therapy that targets human dendritic cells.

Viral vectors of this invention are capable of encoding foreign genes in their genomic RNA. A recombinant viral vector harboring a foreign gene is obtained by inserting a foreign gene into an above-described viral vector genome. The foreign gene can be any desired gene that needs to be expressed in a target dendritic cell, and may be a gene that encodes a naturally-occurring protein, or protein modified from a naturally-occurring protein by deletion, substitution, or insertion of amino acid residues. The foreign gene can be inserted at any desired position in a protein-noncoding region of the virus genome, for example. The above nucleic acid can be inserted, for example, between the 3'-leader region and the viral protein ORF closest to the 3'-end; between each of the viral protein ORFs; and/or between the viral protein ORF closest to the 5'-end and the 5'-trailer region in genomic DNA. Further, in genomes deficient in the F or HN gene or the like, nucleic acids encoding the foreign genes can be inserted into those deficient regions. When introducing a foreign gene into a paramyxovirus, it is desirable to insert the gene such that the chain length of the polynucleotide to be inserted into the genome will be a multiple of six (Journal of Virology, Vol. 67, No. 8,4822-4830,1993). An E-I-S sequence should be arranged between the inserted foreign gene and the viral ORF. Two or more genes can be inserted in tandem via E-I-S sequences.

Expression levels of a foreign gene carried in a vector can be controlled using the type of transcriptional initiation sequence added upstream (to the 3'-side of the negative strand) of the gene (WO01/18223). The expression levels can also be controlled by the position at which the foreign gene is inserted in the genome: the nearer to the 3'-end of the negative strand the insertion position is, the higher the expression level; while the nearer to the 5'-end the insertion position is, the lower the expression level. Thus, to obtain a desired gene expression level, the insertion position of a foreign gene can be appropriately controlled such that the combination with genes encoding the viral proteins before and after the foreign gene is most suitable. In general, since a high foreign gene expression level is thought to be advantageous, it is preferable to link the foreign gene to a highly efficient transcriptional initiation sequence, and to insert it near the 3'-end of the negative strand genome. Specifically, a foreign gene is inserted between the 3'-leader region and the viral protein ORF closest to the 3'-end. Alternatively, a foreign gene may be inserted between the ORFs of the viral gene closest to the 3'-end and the second closest viral gene. In wild type paramyxoviruses, the viral protein gene closest to the 3'-end of the genome is the N gene, and the second closest gene is the P gene. Alternatively, when a high level of expression of the introduced gene is undesirable, the gene expression level from the viral vector can be suppressed to obtain an appropriate effect, for example, by inserting the foreign gene at a site in the vector as close as possible to the 5'-side of the negative strand, or by selecting an inefficient transcriptional initiation sequence.

To prepare a Sendai virus vector, a cDNA encoding a genomic RNA of a virus is transcribed in mammalian cells, in the presence of viral proteins (i.e., N, P, and L proteins) essential for reconstitution of an RNP, which is a component of a virus. Viral RNP can be reconstituted by producing either the negative strand genome (that is, the same antisense strand as the viral genome) or the positive strand (the sense strand encoding the viral proteins). Production of the positive strand is preferable for increased efficiency of vector reconstitution. The RNA terminals preferably reflect the terminals of the 3'-leader sequence and 5'-trailer sequence as accurately as possible, as in the natural viral genome. To accurately regulate the 5'-end of the transcript, for example, the RNA polymerase may be expressed within a cell using the recognition sequence of T7 RNA polymerase as a transcription initiation site. To regulate the 3'-end of the transcript, for example, a self-cleaving ribozyme can be encoded at the 3'-end of the transcript, allowing accurate cleavage of the 3'-end with this ribozyme (Hasan, M. K. et al., J. Gen. Virol. 78: 2813-2820, 1997; Kato, A. et al., 1997, EMBO J. 16: 578-587; and Yu, D. et al., 1997, Genes Cells 2: 457-466).

For example, a recombinant Sendai virus vector carrying a foreign gene can be constructed as follows, according to descriptions in: Hasan, M. K. et al., J. Gen. Virol. 78: 2813-2820, 1997; Kato, A. et al., 1997, EMBO J. 16: 578-587; Yu, D. et al., 1997, Genes Cells 2: 457-466; or the like.

First, a DNA sample including a cDNA sequence of an objective foreign gene is prepared. The DNA sample is preferably one that can be confirmed to be a single plasmid by electrophoresis at a concentration of 25 ng/$\mu$l or more. The following explains the case of using a *Not* I site to insert a foreign gene into a DNA encoding a viral genomic RNA, with reference to examples. When a *Not* I recognition site is included in a target cDNA nucleotide sequence, the base sequence is altered using site-directed mutagenesis or the like, such that the encoded amino acid sequence does not change, and the *Not* I site is preferably excised in advance. The objective gene fragment is amplified from this sample by PCR, and then recovered. By adding the *Not* I site to the 5' regions of a pair of primers, both ends of the amplified fragments become *Not* I sites. E-I-S sequences are designed to be included in primers such that, after a foreign gene is inserted into the viral genome, one E-I-S sequence each is placed between the ORF of the foreign gene, and either side of the ORFs of the viral genes.

For example, to guarantee cleavage with *Not* I, the forward side synthetic DNA sequence has a form in which any desired sequence of not less than two nucleotides (preferably four nucleotides not including a sequence derived from the *Not* I recognition site, such as GCG and GCC, and more preferably ACTT) is selected at the 5'-side, and a *Not* I recognition site 'gcggccgc' is added to its 3'-side. To that 3'-side, nine arbitrary nucleotides, or nine plus a multiple of six nucleotides are further added as a spacer sequence. To the further 3' of this, a sequence corresponding to about 25 nucleotides of the ORF of a desired cDNA, including and counted from the initiation codon ATG, is added. The 3'-end of the forward side synthetic oligo DNA is preferably about 25 nucleotides, selected from the desired cDNA such that the final nucleotide becomes a G or C.

For the reverse side synthetic DNA sequence, no less than two arbitrary nucleotides (preferably four nucleotides not including a sequence derived from a *Not* I recognition site, such as GCG and GCC, and more preferably ACTT) are selected from the 5'-side, a *Not* I recognition site 'gcggccgc' is added to its 3'-side, and to that 3' is further added an oligo DNA insert fragment for adjusting the length. The length of this oligo DNA is designed such that the chain length of the *Not* I fragment of the final PCR-amplified product will become a multiple of six nucleotides (the so-called "rule of six"); Kolakofski, D., et al., J. Virol. 72:891-899, 1998; Calain, P. and Roux, L., J. Virol. 67:4822-4830, 1993; Calain, P. and Roux, L., J. Virol. 67: 4822-4830, 1993). When adding an E-I-S sequence to this primer, to the 3'-side of the oligo DNA insertion fragment is added the complementary strand sequence of the Sendai virus S, I, and E sequences, preferably 5'-CTTTCACCCT-3' (SEQ ID NO: 1), 5'-AAG-3', and 5'-TTTTTCTTACTACGG-3' (SEQ ID NO: 2), respectively; and further to this 3'-side is added a complementary strand sequence corresponding to about 25 nucleotides, counted backwards from the termination codon of a desired cDNA sequence, whose length has been selected such that the final nucleotide of the chain becomes a G or C, to make the 3'-end of the reverse side synthetic DNA.

PCR can be performed according to conventional methods, using Taq polymerase or other DNA polymerases. Objective amplified fragments may be digested with *Not* I, and then inserted into the *Not* I site of plasmid vectors such as pBluescript. The nucleotide sequences of PCR products thus obtained are confirmed with a sequencer, and plasmids that include the correct sequence are selected. The inserted fragment is excised from these plasmids using *Not* I, and cloned into the *Not* I site of a plasmid composed of genomic cDNA. A recombinant Sendai virus cDNA can also be obtained by inserting the fragment directly into the *Not* I site of a genomic cDNA, without using a plasmid vector.

For example, a recombinant Sendai virus genomic cDNA can be constructed according to methods described in the literature (Yu, D. et al., Genes Cells 2: 457-466, 1997; Hasan, M. K. et al., J. Gen. Virol. 78: 2813-2820, 1997). For example, an 18 bp spacer sequence (5'-(G)-CGGCCGCAGATCTTCACG-3') (SEQ ID NO: 3), including a *Not* I restriction site, is inserted between the leader sequence and the ORF of N protein of the cloned Sendai virus genomic cDNA (pSeV(+)), obtaining plasmid pSeV18$^+$b(+), which includes an auto-cleavage ribozyme site derived from the antigenomic strand of delta hepatitis virus (Hasan, M. K. et al., 1997, J. General Virology 78: 2813-2820). A recombinant Sendai virus cDNA including a desired foreign gene can be obtained by inserting a foreign gene fragment into the *Not* I site of pSeV18$^+$b(+).

A viral vector can be reconstituted by transcribing a DNA encoding a genomic RNA of a recombinant virus thus prepared, in cells in the presence of the above-described viral proteins (L, P, and N). The present disclosure provides Sendai vectors for transfer into dendritic cells. In addition, the present disclosure relates to the use of the Sendai virus vectors in the preparation of dendritic cells introduced with a gene and in the preparation of mature dendritic cells The present disclosure also provides DNAs encoding the viral genomic RNAs of the Sendai virus vectors for manufacturing the Sendai virus vectors for transfer into dendritic cells. This disclosure also relates to the use of DNAs encoding the genomic RNAs of the vectors, in the manufacture of the vectors for use in this invention. The recombinant viruses can be reconstituted by methods known in the art (WO97/16539; WO97/16538; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404). With these methods,

Sendai virus can be reconstituted from DNA. The vectors for in this invention can be reconstituted according to these methods. When a viral vector DNA is made F gene, HN gene, and/or M gene deficient, such DNAs do not form infectious virions as is. However, infectious virions can be formed by separately introducing host cells with these deficient genes, and/or genes encoding the envelope proteins of other viruses, and then expressing these genes therein.

Specifically, the viruses can be prepared by the steps of: (a) transcribing cDNAs encoding genomic RNAs of Sendai viruses (negative strand RNAs), or complementary strands thereof (positive strands), in cells expressing N, P, and L proteins; and (b) harvesting culture supernatants thereof including the produced Sendai viruses. For transcription, a DNA encoding a genomic RNA is linked downstream of an appropriate promoter. The genomic RNA thus transcribed is replicated in the presence ofN, L, and P proteins to form an RNP complex. Then, in the presence of M, HN, and F proteins, virions enclosed in an envelope are formed. For example, a DNA encoding a genomic RNA can be linked downstream of a T7 promoter, and transcribed to RNA by T7 RNA polymerase. Any desired promoter can be used as a promoter, in addition to those including a T7 polymerase recognition sequence. Alternatively, RNA transcribed *in vitro* may be transfected into cells.

Enzymes essential for the initial transcription of genomic RNA from DNA, such as T7 RNA polymerase, can be supplied by transducing the plasmid or viral vectors that express them, or, for example, by incorporating the RNA polymerase gene into a chromosome of the cell so as to enable induction of its expression, and then inducing expression at the time of viral reconstitution. Further, genomic RNA and viral proteins essential for vector reconstitution are supplied, for example, by transducing the plasmids that express them. In supplying these viral proteins, helper viruses such as the wild type or certain types of mutant Sendai viruses are used.

Methods for transducing DNAs expressing the genomic RNAs into cells include, for example, (i) methods for making DNA precipitates which target cells can internalize; (ii) methods for making complexes including DNAs that are suitable for internalization by target cells, and have a low-cytotoxic positive charge; and (iii) methods for using electric pulses to instantaneously create holes in the target cell membrane, which are of sufficient size for DNA molecules to pass through.

In the context of method (ii), various transfection reagents can be used. For example, DOTMA (Roche), Superfect (QIAGEN #301305), DOTAP, DOPE, DOSPER (Roche #1811169), and the like can be cited. Regarding method (i), for example, transfection methods using calcium phosphate can be cited, and although DNAs transferred into cells by this method are internalized by phagosomes, a sufficient amount of DNA is known to enter the nucleus (Graham, F. L. and Van Der Eb, J., 1973, Virology 52: 456; Wigler, M. and Silverstein, S., 1977, Cell 11: 223). Chen and Okayama investigated the optimization of transfer techniques, reporting that (1) incubation conditions for cells and coprecipitates are 2 to 4% $CO_2$, 35°C, and 15 to 24 hours, (2) the activity of circular DNA is higher than linear DNA, and (3) optimal precipitation is obtained when the DNA concentration in the precipitate mixture is 20 to 30 μg/ml (Chen, C. and Okayama, H., 1987, Mol. Cell. Biol. 7: 2745). The methods of (ii) are suitable for transient transfections. Methods for performing transfection by preparing a DEAE-dextran (Sigma #D-9885 M.W. 5x $10^5$) mixture with a desired DNA concentration ratio have been known for a while. Since most complexes are decomposed in endosomes, chloroquine may also be added to enhance the effect (Calos, M. P., 1983, Proc. Natl. Acad. Sci. USA 80: 3015). The methods of (iii) are referred to as electroporation methods, and are used more in general than methods (i) or (ii) because they are not cell-selective. The efficiency of these methods is presumed to be good under optimal conditions for: the duration of pulse electric current, shape of the pulse, potency of electric field (gap between electrodes, voltage), conductivity of buffer, DNA concentration, and cell density.

Of the above three categories, the methods of(ii) are simple to operate and facilitate examination of many samples using a large amount of cells, making transfection reagents suitable for the transduction into cells of DNA for vector reconstitution. Preferably, the Superfect Transfection Reagent (QIAGEN, Cat No. 301305), or the DOSPER Liposomal Transfection Reagent (Roche, Cat No. 1811169) is used; however, the transfection reagents are not limited to these.

Specifically, virus reconstitution from cDNA can be carried out, for example, as follows:

In a plastic plate of about 6 to 24 wells, or a 100-mm Petri dish or the like, simian kidney-derived LLC-MK2 cells (ATCC CCL-7) are cultured up to about 100% confluency, using minimum essential medium (MEM) including 10% fetal calf serum (FCS) and antibiotics (100 units/ml penicillin G and 100 μg/ml streptomycin). Then they are infected with, for example, two plaque forming units (PFU)/cell of the recombinant vaccinia virus vTF7-3, which expresses T7 RNA polymerase and has been inactivated by 20-minutes of UV irradiation in the presence of 1 μg/ml psoralen (Fuerst, T. R. et al., Proc. Natl. Acad. Sci. USA 83: 8122-8126,1986; Kato, A. et al., Genes Cells 1: 569-579, 1996). The amount of psoralen added and the UV irradiation time can be appropriately adjusted. One hour after infection, 2 to 60 μg, and more preferably 3 to 20 μg, of DNA encoding the genomic RNA of a recombinant Sendai virus is transfected along with the plasmids expressing trans-acting viral proteins essential for viral RNP production (0.5 to 24 μg of pGEM-N, 0.25 to 12 μg of pGEM-P, and 0.5 to 24 μg of pGEM-L) (Kato, A. et al., Genes Cells 1: 569-579, 1996), using the lipofection method or the like with Superfect (QIAGEN). For example, the ratio of the amounts of expression vectors encoding the N, P, and L proteins is preferably 2:1:2, and the plasmid amounts are appropriately adjusted in the range of 1 to 4 μg of pGEM-N, 0.5 to 2 μg of pGEM-P, and 1 to 4 μg of pGEM-L.

The transfected cells are cultured, as desired, in serum-free MEM composed of 100 μg/ml of rifampicin (Sigma) and cytosine arabinoside (AraC), more preferably only 40 μg/ml of cytosine arabinoside (AraC) (Sigma). Optimal drug concentrations are set so as to minimize cytotoxicity due to the vaccinia virus, and to maximize virus recovery rate (Kato, A. et al., 1996, Genes Cells 1: 569-579). After culturing for about 48 to 72 hours after transfection, cells are harvested, and then disintegrated by repeating freeze-thawing three times. LLC-MK2 cells are re-infected with the disintegrated materials including RNP, and cultured. Alternatively, the culture supernatant is recovered, added to a culture solution of LLC-MK2 cells to infect them, and the cells are then cultured. Transfection can be conducted by, for example, forming a complex with lipofectamine, polycationic liposome, or the like, and transducing the complex into cells. Specifically, various transfection reagents can be used. For example, DOTMA (Roche), Superfect (QIAGEN #301305), DOTAP, DOPE, and DOSPER (Roche #1811169) may be cited. In order to prevent decomposition in the endosome, chloroquine may also be added (Calos, M. P., 1983, Proc. Natl. Acad. Sci. USA 80: 3015). In cells transduced with RNP, viral gene expression from RNP and RNP replication progress, and the vector is amplified. By diluting the viral solution thus obtained (for example, $10^6$-fold), and then repeating the amplification, the vaccinia virus vTF7-3 can be completely eliminated.

Amplification is repeated, for example, three or more times. Vectors thus obtained can be stored at -80°C. In order to reconstitute a viral vector having no propagation ability and lacking a gene encoding an envelope protein, LLC-MK2 cells expressing the envelope protein may be used for transfection, or a plasmid expressing the envelope protein may be cotransfected. Alternatively, a defective type viral vector can be amplified by culturing the transfected cells overlaid with LLK-MK2 cells expressing the envelope protein (see WO00/70055 and WO00/70070).

Titers of viruses thus recovered can be determined, for example, by measuring CIU (Cell-Infected Unit) or hemagglutination activity (HA) (WO00/70070; Kato, A. et al., 1996, Genes Cells 1: 569-579; Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). Titers of vectors carrying GFP (green fluorescent protein) marker genes and the like can be quantified by directly counting infected cells, using the marker as an indicator (for example, as GFP-CIU). Titers thus measured can be treated in the same way as CIU (WO00/70070).

So long as a viral vector can be reconstituted, the host cells used in the reconstitution are not particularly limited. For example, in the reconstitution of Sendai virus vectors cultured cells such as LLC-MK2 cells and CV-1 cells derived from monkey kidney, BHK cells derived from hamster kidney, and cells derived from humans can be used. By expressing suitable envelope proteins in these cells, infectious virions including the proteins in the envelope can also be obtained. Further, to obtain a large quantity of a Sendai virus vector, a viral vector obtained from an above-described host can be infected to embrionated hen eggs, to amplify the vector. Methods for manufacturing viral vectors using hen eggs have already been developed (Nakanishi, et al., ed. (1993), "State-of-the-Art Technology Protocol in Neuroscience Research III, Molecular Neuron Physiology", Koseisha, Osaka, pp. 153-172). Specifically, for example, a fertilized egg is placed in an incubator, and cultured for nine to twelve days at 37 to 38°C to grow an embryo. After the viral vector is inoculated into the allantoic cavity, the egg is cultured for several days (for example, three days) to proliferate the viral vector. Conditions such as the period of culture may vary depending upon the recombinant Sendai virus being used. Then, allantoic fluids including the vector are recovered. Separation and purification of a Sendai virus vector from allantoic fluids can be performed according to a usual method (Tashiro, M., "Virus Experiment Protocol," Nagai, Ishihama, ed., Medical View Co., Ltd., pp. 68-73, (1995)).

For example, the construction and preparation of Sendai virus vectors defective in F gene can be performed as described below (see WO00/70055 and WO00/70070).

<1> Construction of a genomic cDNA of an F-gene defective Sendai virus, and a plasmid expressing F gene:

A full-length genomic cDNA of Sendai virus (SeV), the cDNA of pSeV $18^+$ b (+) (Hasan, M. K. et al., 1997, J. General Virology 78: 2813-2820) ("pSeV$18^+$ b (+)" is also referred to as "pSeV$18^+$"), is digested with *Sph*I/*Kpn*I to recover a fragment (14673 bp), which is cloned into pUC18 to prepare plasmid pUC 18/KS. Construction of an F gene-defective site is performed on this pUC18/KS. An F gene deficiency is created by a combination of PCR-ligation methods, and, as a result, the F gene ORF (ATG-TGA = 1698 bp) is removed. Then, for example, 'atgcatgccggcagatga (SEQ ID NO: 4)' is ligated to construct an F gene-defective type SeV genomic cDNA (pSeV$18^+$/ΔF). A PCR product formed in PCR by using the pair of primers [forward: 5'-gttgagtactgcaagagc/SEQ ID NO: 5, reverse: 5'-tttgccggcatgcatgtttcccaaggggagagttttgcaacc/SEQ ID NO: 6] is connected upstream of F, and a PCR product formed using the pair of primers [forward: 5'-atgcatgccggcagatga/SEQ ID NO: 7, reverse: 5'-tgggtgaatgagagaatcagc/SEQ ID NO: 8] is connected downstream of the F gene with *EcoT22I.* The plasmid thus obtained is digested with *Sac*I and *Sal*I to recover a 4931 bp fragment of the region including the F gene-defective site, which is cloned into pUC18 to form pUC18/dFSS. This pUC18/dFSS is digested with *Dra*III, the fragment is recovered, replaced with the *Dra*III fragment of the region including the F gene of pSeV $18^+$, and ligated to obtain the plasmid pSeV18+/ΔF.

A foreign gene is inserted, for example, into the *Nsi* I and *Ngo* MIV restriction enzyme sites in the F gene-defective site of pUC18/dFSS. For this, a foreign gene fragment may be, for example, amplified using an *Nsi* I-tailed primer and an *Ngo* MIV-tailed primer.

<2> Preparation of helper cells that induce SeV-F protein expression:

To construct an expression plasmid of the Cre/loxP induction type that expresses the Sendai virus F gene (SeV-F), the SeV-F gene is amplified by PCR, and inserted to the unique *Swa* I site of the plasmid pCALNdlw (Arai, T. et al., J. Virology 72, 1998, p1115-1121), which is designed to enable the inducible expression of a gene product by Cre DNA recombinase, thus constructing the plasmid pCALNdLw/F.

To recover infectious virions from the F gene-defective genome, a helper cell line expressing SeV-F protein is established. The monkey kidney-derived LLC-MK2 cell line, which is commonly used for SeV proliferation, can be used as the cells, for example. LLC-MK2 cells are cultured in MEM supplemented with 10% heat-inactivated fetal bovine serum (FBS), penicillin G sodium (50 units/ml), and streptomycin (50 μg/ml) at 37°C in 5% $CO_2$. Since the SeV-F gene product is cytotoxic, the above-described plasmid pCALNdLw/F, which was designed to enable inducible expression of the F gene product with Cre DNA recombinase, is transfected to LLC-MK2 cells by the calcium phosphate method (using a mammalian transfection kit (Stratagene)), according to protocols well known in the art.

The plasmid pCALNdLw/F (10 μg) is transduced into LLC-MK2 cells grown to 40% confluency using a 10-cm plate, and the cells are then cultured in MEM (10 ml) including 10% FBS, in a 5% $CO_2$ incubator at 37°C for 24 hours. After 24 hours, the cells are detached and suspended in the medium (10 ml). The suspension is then seeded into five 10-cm dishes, 5 ml into one dish, 2 ml each into two dishes, and 0.2 ml each into two dishes, and cultured in MEM (10 ml) including G418 (GIBCO-BRL) (1200 μg/ml) and 10% FBS. The cells were cultured for 14 days, exchanging the medium every two days, to select cell lines stably transduced with the gene. The cells grown from the above medium that show G418 resistance are recovered using a cloning ring. Culture of each clone thus recovered is continued in 10-cm plates until confluent.

After the cells have grown to confluency in a 6-cm dish, F protein expression can be induced by infecting the cells with adenovirus AxCANCre, for example, at MOI = 3, according to the method of Saito, *et al.* (Saito et al., Nucl. Acids Res. 23: 3816-3821 (1995); Arai, T. et al., J. Virol 72, 1115-1121 (1998)).

<3> Reconstruction and amplification of F gene-deficient SeV virus:

The above-described plasmid pSeV18$^+$/ΔF inserted with the foreign gene is transfected into LLC-MK2 cells by the procedure described below. LLC-MK2 cells are seeded on 100-mm dishes at 5 x $10^6$ cells/dish. To transcribe the genomic RNA using T7 RNA polymerase, the cells are cultured for 24 hours, and then recombinant vaccinia virus, which expresses T7 RNA polymerase (PLWUV-VacT7: Fuerst, T.R. et al., Proc. Natl. Acad. Sci. USA 83, 8122-8126 (1986)) and is treated with psoralen and long-wavelength ultraviolet light (365 nm) for 20 minutes, is inoculated to the cells at a MOI of about 2 at room temperature for one hour. The ultraviolet light irradiation to the vaccinia virus can be achieved, for example, by using UV Stratalinker 2400 with five 15-watt bulbs (Catalog No. 400676 (100V); Stratagene, La Jolla, CA, USA). After the cells are washed with serum-free MEM, plasmid expressing the genomic RNA and expression plasmids each expressing N, P, L, F, or HN protein of the minus-strand RNA virus are transfected into the cells using an appropriate lipofection reagent. The plasmid ratio is preferably, but is not limited to, 6:2:1:2:2:2 in this order. For example, the expression plasmid for the genomic RNA, and the expression plasmids each of which expresses N, P, or L protein, or F and HN proteins (pGEM/NP, pGEM/P, pGEM/L, and pGEM/F-HN; WO00/70070, Kato, A. et al., Genes Cells 1, 569-579 (1996)) are transfected at amounts of 12, 4, 2, 4, and 4 μg/dish, respectively. After a few hours of culture, the cells are washed twice with serum-free MEM, and then cultured in MEM supplemented with 40 μg/ml cytosine β-D-arabinofuranoside (AraC: Sigma, St. Louis, MO) and 7.5 μg/ml trypsin (Gibco-BRL, Rockville, MD). The cells are recovered, and the resulting pellet is suspended in OptiMEM ($10^7$ cells/ml). The suspension is subjected to three freeze-thaw cycles, and mixed with lipofection reagent DOSPER (Boehringer Mannheim) ($10^6$ cells/25 μl DOSPER). After the mixture is allowed to stand at room temperature for 15 minutes, it is transfected to F-expressing helper cells ($10^6$ cells/well in 12-well-plate) cloned as described above. The cells are cultured in serum-free MEM (containing 40 μg/ml AraC and 7.5 μg/ml trypsin), and the supernatant is collected. Viruses deficient in genes other than F, for example, HN and M genes, can be prepared by a similar method as described above.

For the preparation of viral gene-deficient vectors, for example, two or more types of vectors which differ in the deficient viral gene on the viral genome carried by the vector are introduced into same cells. The respective deficient viral proteins are supplied through the expression from the other vector(s). Therefore, the vectors complement each other to form infectious viral particles, resulting in a complete replication cycle and amplification of the viral vectors. Specifically, when two or more types of vectors of the present invention are inoculated in combination that allows complementation of viral proteins, a mixture of the viral gene-deficient vectors can be produced on a large scale at low cost. Since such viruses lack viral genes, their genome sizes are smaller than those of viral gene-nondeficient viruses and thus can carry larger foreign genes. Furthermore, these viruses that are non-proliferative due to the lack of viral genes become diluted outside cells, which makes it difficult to maintain their coinfection. The viruses become sterile and thus are advantageous from the viewpoint of controlling their environmental release.

There is no limitation on the foreign gene to be introduced using the Sendai virus, and naturally occurring proteins include, for example, hormones, cytokines, growth factors, receptors, intracellular signaling molecules, enzymes, and peptides. The proteins may be secretory proteins, membrane proteins, cytoplasmic proteins, nuclear proteins, and the like. Artificial proteins include, for example, fusion proteins such as chimeric toxin, dominant negative proteins (including soluble receptor molecules or membrane bound dominant negative receptors), truncated cell adhesion molecules, and cell surface molecules. The proteins may also be proteins to which a secretory signal, membrane-localization signal, nuclear translocation signal, or the like has been attached. Functions of a particular gene can be suppressed by introducing and expressing antisense RNA molecule, RNA-cleaving ribozyme, or the like as the transfer gene. When a viral vector is prepared using a gene for treating diseases as the foreign gene, gene therapy can be performed through the introduction of the vector. The viral vector of the present invention is applicable to gene therapy wherein the genes are expressed by direct administration or by *ex vivo* administration, and enables expression of foreign genes for which therapeutic effect can be expected, internal genes short in *in vivo* supply, or the like from dendritic cells. In addition, the method of the present invention can also be used as a gene therapy vector in regeneration medicine.

According to the method for producing viruses as described herein, the viral vector for use in the present invention can be released into extracellular fluid of virus producing cells at a titer of, for example, 1 x $10^5$ CIU/ml or higher, preferably 1 x $10^6$ CIU/ml or higher, more preferably 5 x $10^6$ CIU/ml or higher, more preferably 1 x $10^7$ CIU/ml or higher, more preferably 5 x $10^7$ CIU/ml or higher, more preferably 1 x $10^8$ CIU/ml or higher, and more preferably 5 x $10^8$ CIU/ml or higher. The titer of virus can be determined according to methods described herein or elsewhere (Kiyotani, K. et al., Virology 177(1), 65-74 (1990); and WO00/70070).

The recovered viral vectors can be purified to be substantial pure. The purification can be achieved using known purification/separation methods, including filtration, centrifugation, adsorption, and column purification, or any combinations thereof. The phrase "substantially pure" means that the vector component constitutes a major proportion of a solution of the vector. For example, a viral vector composition can be confirmed to be substantially pure by the fact that the proportion of protein contained as the viral vector component to the total protein (excluding proteins added as carriers and stabilizers) in the solution is 10% (w/w) or greater, preferably 20% or greater, more preferably 50% or greater, preferably 70% or greater, more preferably 80% or greater, and even more preferably 90% or greater. Specific purification methods for, for example, the paramyxovirus vector includes methods using cellulose sulfate ester or cross-linked polysaccharide sulfate ester (Japanese Patent Application Kokoku Publication No. (JP-B) S62-30752 (examined, approved Japanese patent application published for opposition), JP-B S62-33879, and JP-B S62-30753) and methods including adsorbing to fucose sulfate-containing polysaccharide and/or degradation products thereof (WO97/32010), but are not limited thereto.

In the production of compositions containing the vector, the vector may be combined with desired pharmaceutically acceptable carriers or media according to needs. The "pharmaceutically acceptable carriers or media" refers to materials that can be administered together with the vector and that do not significantly inhibit the gene transfer via the vector. Such carriers and media include, for example, deionized water, sterile water, sodium chloride solution, dextrose solution, Ringer's solution containing dextrose, sodium chloride, and lactated, culture medium, serum, and phosphate buffered saline (PBS). They may be appropriately combined with the vector to formulate a composition. The composition may also include membrane stabilizers for liposome (for example, sterols such as cholesterol). The composition may also include antioxidants (for example, tocopherol or vitamin E). In addition, the composition may also include vegetable oils, suspending agents, detergents, stabilizers, biocidal agents, and the like. Furthermore, preservatives and other additives may also be added. The formula of the present composition may be aqueous solution, capsule, suspension, syrup, or the like. The vector composition of the present invention may also be in a form of solution, freeze-dried product, or aerosol. When it is a freeze-dried product, it may include sorbitol, sucrose, amino acids, various proteins, and the like as a stabilizer. The composition containing the vector of the present invention is useful as a reagent for introducing genes into dendritic cells and also as a pharmaceutical that is used in gene therapy targeting dendritic cells. Furthermore, the vector solution is useful as a vaccine (J. I. Mayordomo et al., Nature Med. 1(12), 1279-1302, (1995)). Moreover, when an antigen peptide is expressed in dendritic cells using the vector of the present invention, the cells presenting this peptide can be used as a vaccine. The vaccine compositions may include immunostimulants, such as cytokine, cholera toxin, and Salmonella toxin to improve immunogenicity. Furthermore, the vaccine may be combined with adjuvants, such as alum, incomplete Freund's adjuvant, MF59 (oil emulsion), MTP-PE (muramyl tripeptide derived from cell wall of mycobacteria), and QS-21 (derived from soapbark tree *Quilaja saponaria*).

When administering the composition, it is effective to combine them with cytokines that improve the adjuvant effect. Such genes include, for example,

(i) a combination of IL-2 and single-chain IL-12 (Proc. Natl. Acad. Sci. USA 96 (15): 8591-8596, 1999);
(ii) IL-2 and interferon-$\gamma$ (U.S. Patent No. 5,798,100);
(iii) granulocyte colony stimulating factor (GM-CSF), which is used alone; and
(iv) a combination of GM-CSF and IL-4 (J. Neurosurgery 90 (6), 1115-1124 (1999)).

An antigen to be presented by dendritic cells may be encoded by the Sendai virus vector, added to (specifically, pulsed into) dendritic cells into which the vector has been introduced, or expressed using an alternate desired vector. Such antigens include desired antigens related to infectious microorganisms, viruses, parasites, pathogens, cancers, and the like. These may be structural or non-structural proteins. Such antigens (or processed peptides thereof) are presented on the cell surface bound to MHC molecules on the surface of dendritic cells to induce immune responses.

When used as a vaccine, the antigens can be applied to, for example, tumors, infectious diseases, and other general diseases. To treat infectious diseases, for example, epitopes of an antigen protein of an infectious microorganism may be analyzed, and then expressed or presented by dendritic cells.

Antigens derived from pathogens include, for example, proteins of hepatitis A virus, hepatitis B virus, hepatitis C virus, hepatitis delta virus, papilloma virus antigen, herpes simplex virus (HSV), varicella-zoster virus (VZV), Epstein-Barr virus, Cytomegalovirus (CMV), HIV, malaria, and the like, or partial peptides thereof. The minus-strand RNA viruses encoding such antigen proteins can be used prophylactically or therapeutically. Specifically, envelopes of influenza highly-virulent strain H5N1 for influenza, envelope proteins of Japanese encephalitis virus (Vaccine, vol. 17, No. 15-16, 1869-1882 (1999)) for Japanese encephalitis, HIV and SIV gag proteins (J. Immunology (2000) vol. 164, 4968-4978), HIV envelope proteins, Nef protein, and other viral proteins for AIDS can be mentioned. In addition, for example, cholera toxin B subunit (CTB) (Arakawa T, et al., Nature Biotechnology (1998) 16(10): 934-8, Arakawa T, et al., Nature Biotechnology (1998) 16(3): 292-7) for cholera; rabies virus glycoprotein (Lodmell DL et al., 1998, Nature Medicine 4(8):949-52) for rabies; and capsid protein L1 of human papilloma virus type 6 (J. Med. Virol, 60, 200-204 (2000)) for cervical carcinoma can be mentioned. Antigen proteins of other pathogenic viruses can also be expressed from the vector. Furthermore, it is possible to use JE-E antigen protein of Japanese encephalitis (Japanese Patent Application Kokai Publication No. (JP-A) S64-74982 (unexamined, published Japanese patent application), JP-A H1-285498), gD2 protein of human herpes simplex virus (JP-A H5-252965), polypeptides derived from hepatitis C virus (JP-A H5-192160), polypeptides derived from pseudorabies virus (Japanese Patent Kohyo Publication No. (JP-A) H7-502173 (unexamined Japanese national phase publication corresponding to a non-Japanese international publication), and the like. For example, cells derived from patients infected with such pathogenic microorganisms may be analyzed to identify an epitope of an antigen protein to be presented on antigen-presenting cells (APC) for use. It is preferred to appropriately select the HLA type and identify an epitope corresponding to the desired HLA for use.

To specifically promote immune response against tumor, the Sendai virus vector expressing one or more tumor antigens is introduced into dendritic cells, or dendritic cells activated by the Sendai virus vector are pulsed with tumor antigens. The tumor antigens may be tumor cell-specific antigens (*i.e.,* existing in tumor cells but absent in non-tumor cells) or antigens that are expressed at a higher level in tumor cells than in non-tumor cells of the same type. The immune system is stimulated through the administration of the dendritic cells. When CTL acts as a major effector, a desired intercellular or extracellular tumor antigen can be used. When an antibody is reacted as the effector by using dendritic cells to activate CD4 T cells which triggers the induction of antibody production through B cell activation, it is preferred to use an antigen presented on the cell surface. For example, a cell surface receptor or cell adhesion protein can be used as the antigen. The tumor antigens include, for example, Muc-1 or Muc-1-like mucin tandem repeat peptide that induce ovarian cancer or the like (U.S. Patent No. 5,744,144); E6 and E7 proteins of human papilloma virus, which cause cervical cancer; melanoma antigens MART-1, MAGE-1, -2, -3, gp100, and tyrosinase; prostate cancer antigen PSA; as well as CEA (Kim, C. et al., Cancer Immunol. Immunother. 47 (1998) 90-96) and Her2neu (HER2p63-71, p780-788; Eur. J. Immunol. 2000; 30: 3338-3346).

Dendritic cells that are prepared according to the present invention are useful in effective immunotherapy for cancers and infectious diseases. Immunological sensitization by dendritic cells introduced with a gene of a tumor antigen or infectious disease-related antigen or T cells stimulated with such dendritic cells serves as an effective method for inducing anti-tumor or anti-infectious disease immunity in patients. The present disclosure also relates to the use of dendritic cells obtained by the present method in the induction of immune response. Specifically, the present disclosure relates to the use of dendritic cells obtained by the present method in immunotherapy, in particular, for example, in the treatment of tumors or infectious diseases. Furthermore, the present disclosure relates to the use of dendritic cells obtained by the present method in the production of immunoactivating agents. Specifically, the present disclosure relates to the use of dendritic cells obtained by the present method in the production of immunotherapeutic agents, in particular, for example, antitumor agents (tumor growth suppressants) or therapeutic agents for infectious diseases.

The cells can also be applied to general diseases. To treat diabetes, for example, a peptide of an insulin fragment can be used as an epitope in animal models of type I diabetes (Coon, B. et al., J. Clin. Invest., 1999, 104(2): 189-94).

In addition, by expressing a cytokine in dendritic cells, the cells stimulate the immune system, thereby enhancing immune responses against cancers or infectious microorganisms. Thus, dendritic cells introduced with a gene encoding a cytokine are also useful in the treatment of cancers and other diseases for which cytokine therapy is expected to be effective. A dendritic cell introduced with a Sendai virus vector carrying a gene encoding an immunostimulatory cytokine serves as an effective immune inducing agent. For example, immunostimulatory cytokines include interleukins (for example, IL-lalpha, IL-1 beta, IL-2, IL-3, IL-4, IL-6, IL-7, IL-8, IL-9, IL-10, IL-12, IL-15, IL-18, IL-19, IL-20, IL-21, IL-23, and IL-27), interferons (for example, IFN-alpha, IFN-beta, and IFN-gamma), tumor necrosis factor (TNF), transforming growth factor (TGF)-beta, granulocyte colony stimulating factor (G-CSF), macrophage colony stimulating factor (M-CSF), granulocyte macrophage colony stimulating factor (GM-CSF), insulin-like growth factor (IGF)-I, IGF-2, Flt-3 ligand, Fas ligand, c-kit ligand, and other immunomodulatory proteins (such as chemokines and costimulatory molecules).

The amino acid sequences of these cytokines are well known to those skilled in the art. One may refer to: for IL-4, for example, Arai et al. (1989), J. Immunol. 142(1) 274-282; for IL-6, for example, Yasukawa et al. (1987), EMBO J., 6(10): 2939-2945; for IL-12, for example, Wolf et al. (1991), J. Immunol. 146(9): 3074-3081; for IFN-alpha, for example, Gren et al. (1984) J. Interferon Res. 4(4): 609-617, and Weismann et al. (1982) Princess Takamatsu Symp. 12: 1-22;

for TNF, for example, Pennica et al. (1984) Nature 312: 724-729; for G-CSF, for example, Hirano et al. (1986) Nature 324:73-76; and for GM-CSF, for example, Cantrell et al. (1985) Proc. Natl. Acad. Sci. (USA) 82(18): 6250-6254. More specifically, the nucleic acid sequence encoding GM-CSF includes sequences containing the sequences from position 84 to 461 of Accession number NM_000758 (corresponding to position 18 to 144 of the amino acid sequence of NP_ 000749). The nucleic acid sequence encoding IL-4 includes sequences containing the sequences from position 443 to 829 of Accession number NM_000589 (corresponding to position 25 to 153 of the amino acid sequence of NP_000580). Vectors can be introduced into dendritic cells by designing them to include natural genes encoding these cytokines or mutant genes that still encode functional cytokines due to the degeneracy of genetic code.

Moreover, the genes may be modified to express modified forms of the cytokines. For example, a cytokine that has two forms, precursor and matured forms (for example, those producing active fragments by cleavage of their signal peptides, or by restrictive proteolysis), may be genetically modified to express either the precursor or the matured form. Other modified forms (for example, fusion proteins of an active fragment of a cytokine and a heterologous sequence (for example, heterologous signal peptide)) can also be used.

For example, as shown in Examples, dendritic cells introduced with a Sendai virus vector carrying the IFN-beta gene very strongly activate cytotoxic T lymphocytes to significantly suppress the growth of a tumor, which expresses the corresponding antigen. Since the dendritic cells are activated by the vector introduction, it is unnecessary to stimulate the dendritic cells using toxic LPS or the like. Thus, the dendritic cells introduced with the Sendai virus vector carrying the IFN-beta gene serve as an effective therapeutic agent for anti-tumor immunotherapy. The IFN-beta gene of various primates and mammals including human and mouse are well known. For example, the human IFN-beta gene is exemplified by SEQ ID NO: 12 (the mature polypeptide is from position 21 to 187 of SEQ ID NO: 13), and the mouse IFN-beta gene is exemplified by SEQ ID NO: 14 (the mature polypeptide is from position 21 to 182 of the sequence shown in SEQ ID NO: 15) (Derynck, R. et al., Nature 285, 542-547 (1980); Higashi, Y. et al., J. Biol. Chem. 258, 9522-9529 (1983); Kuga, T. et al., Nucleic Acids Res. 17, 3291 (1989)). The signal peptide may be replaced with a signal sequence of other proteins if required.

The IFN-beta gene can be identified by homology search or the like, based on the known sequences of IFN-beta cDNA and protein described above (for example, BLAST; Altschul, S. F. et al., 1990, J. Mol. Biol. 215: 403-410). Alternatively, the IFN-beta gene can be obtained by RT-PCR, using primers designed based on the known nucleotide sequence of the IFN-beta cDNA, or readily obtained by screening a cDNA library derived from a human, mouse, rat, or other mammals by hybridization using IFN-beta cDNA as a probe under stringent conditions. The hybridization condition can be determined by preparing a probe from either a nucleic acid including the coding region of IFN-beta cDNA or a nucleic acid used as the target of hybridization, and detecting whether the probe hybridizes to the other nucleic acid. The probe may be a fragment of the nucleic acid, and generally, has a length of 20 bases or more, preferably 30 bases or more, and more preferably 50 bases or more. An example of the stringent hybridization condition is wherein hybridization is performed in a solution containing 5x SSC (1x SSC contains 150 mM NaCl and 15 mM sodium citrate), 7% (w/v) SDS, 100 μg/ml denatured salmon sperm DNA, 5x Denhardt's solution (1x Denhardt's solution contains 0.2% polyvinyl pyrrolidone, 0.2% bovine serum albumin, and 0.2% Ficoll) at 48°C, preferably at 50°C, and more preferably at 52°C, followed by washing with shaking for 2 hours at the same temperature as in the hybridization, more preferably at 60°C, even more preferably at 65°C, and most preferably at 68°C in 2x SSC, preferably in 1x SSC, and more preferably in 0.5x SSC (for example, in 0.1x SSC).

Nucleotide or amino acid sequences of mammalian IFN-beta in general are composed of a sequence having high homology to a known IFN-beta sequence (for example, sequences corresponding to the mature proteins shown in SEQ ID NOs: 12 to 15). The high homology means that a sequence exhibits 70% or higher identity, preferably 75% or higher identity, more preferably 80% or higher identity, more preferably 85% or higher identity, more preferably 90% or higher identity, and more preferably 95% or higher identity. The sequence identity can be determined, for example, using BLAST program (Altschul, S. F. et al., 1990, J. Mol. Biol. 215: 403-410). Specifically, blastn program may be used to determine nucleotide sequence identity, while blastp program may be used to determine amino acid sequence identity. For example, at the BLAST web page of NCBI (National Center for Biotechnology Information), computation may be carried out using default parameters setting "OFF" the filters, such as "Low complexity" (Altschul, S.F. et al. (1993) Nature Genet. 3:266-272; Madden, T.L. et al. (1996) Meth. Enzymol. 266:131-141; Altschul, S.F. et al. (1997) Nucleic Acids Res. 25:3389-3402; Zhang, J. & Madden, T.L. (1997) Genome Res. 7:649-656). The parameters are set, for example, as follows: open gap cost is set as 5 for nucleotides or as 11 for proteins; extend gap cost is set as 2 for nucleotides or as 1 for proteins; nucleotide mismatch penalty is set as -3; reward for a nucleotide match is set as 1; expect value is set as 10; the wordsize is set as 11 for nucleotides or as 2 for proteins; Dropoff (X) for blast extensions in bits is set as 20 in blastn or as 7 in other programs; X dropoff value for gapped alignment (in bits) is set as 15 in programs other than blastn; and final X dropoff value for gapped alignment (in bits) is set as 50 in blastn or 25 in other programs. In amino acid sequence comparison, BLOSUM62 can be used as a scoring matrix. The blast2sequences program (Tatiana A et al. (1999) FEMS Microbiol Lett. 174:247-250), which compares two sequences, can be used to prepare an alignment of two sequences and thereby determine their sequence identity. The identity for the entire CDS of mature IFN-beta protein (for example, position 64 to 561 in SEQ ID NO: 12 or position 64 to 546 in SEQ ID NO: 14) or the entire amino acid sequence (for example, position 22 to 187 in SEQ ID NO: 13 or position 22 to 182 in SEQ ID NO: 15) is calculated while treating gaps as the same as mismatches and neglecting gaps outside the coding sequence (CDS) of the mature IFN-beta protein.

Polymorphisms and variants exist for IFN-beta. Variants that retain an equivalent activity to the wild-type IFN-beta can be suitably used. The equivalent activity to the wild-type IFN-beta includes antiviral activity, which can be determined, for example, by assaying the activity to inhibit the cytotoxicity of vesicular stomatitis virus. Specifically, vesicular stomatitis Indiana virus [VR-1238AF; ATCC (American Type Culture Collection)] is inoculated to WISH cells (CCL-25; ATCC, Manassas, VA, U.S.A.), and cell death caused by the virus is detected to assay the defense by IFN-beta (the assay condition is determined according to Knezic, Z., et al. (1993) Antiviral Res. 25, 215-221). The concentration at which 50% of the virus-mediated cell death is suppressed is defined as 1 international unit (IU). A polypeptide having an equivalent antiviral activity to the wild-type IFN-beta preferably has a specific activity of 1 x $10^6$ IU/mg protein or greater, more preferably 5 x $10^6$ IU/mg protein or greater, and more preferably 1 x $10^7$ IU/mg protein or greater. Further, a polypeptide having an equivalent antiviral activity to the wild-type IFN-beta preferably has a specific antiviral activity of one tenth or greater of that of wild-type IFN-beta.

A polymorphic form or variant of IFN-beta in general can include a nucleotide or amino acid sequence with a substitution, deletion, and/or insertion of one or more residues in the sequence of a certain IFN-beta molecular species (for example, SEQ ID NOs: 12 to 15). The difference from a known IFN-beta sequence is typically 30 residues or less, preferably 20 residues or less, preferably 10 residues or less, more preferably 5 residues or less, more preferably 3 residues or less, and more preferably 2 residues or less. The amino acid substitutions may be conservative substitutions. Proteins with conservative substitutions tend to retain their activities. The conservative substitutions include, for example, amino acid substitutions among members of groups, such as basic amino acids (for example, lysine, arginine, and histidine), acidic amino acids (for example, aspartic acid and glutamic acid), non-charged polar amino acids (for example, glycine, asparagine, glutamine, serine, threonine, tyrosine, and cysteine), non-polar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine).

Specifically, IFN-beta genes include the following nucleic acids:

(a) a nucleic acid encoding a polypeptide including the amino acid sequence from position 22 to 187 of SEQ ID NO: 13 or the amino acid sequence from position 22 to 182 of SEQ ID NO: 15;
(b) a nucleic acid including the sequence from position 64 to 561 of SEQ ID NO: 12, the sequence from position 64 to 546 of SEQ ID NO: 14, or a complementary sequence thereto, which encodes a polypeptide including the sequence from position 22 to 187 of SEQ ID NO: 13 or the sequence from position 22 to 182 of SEQ ID NO: 15;
(c) a nucleic acid that hybridizes under stringent conditions to the sequence from position 64 to 561 of SEQ ID NO: 12, the sequence from position 64 to 546 of SEQ ID NO: 14, or a complementary sequence thereto, which encodes a polypeptide having equivalent activity to the wild-type IFN-beta;
(d) a nucleic acid encoding a polypeptide having equivalent activity to the wild-type IFN-beta, which includes the amino acid sequence from position 22 to 187 of SEQ ID NO: 13, or the amino acid sequence from position 22 to 182 of SEQ ID NO: 15 wherein one or more amino acids are substituted, deleted, and/or inserted;
(e) a nucleic acid encoding a polypeptide having equivalent activity to the wild-type IFN-beta, which includes a sequence highly homologous to the sequence from position 64 to 561 of SEQ ID NO: 12, the sequence from position 64 to 546 of SEQ ID NO: 14, or a complementary sequence thereto; and
(f) a nucleic acid encoding a polypeptide having equivalent activity to the wild-type IFN-beta, which includes a sequence highly homologous to the sequence from position 22 to 187 of SEQ ID NO: 13, or the sequence from position 22 to 182 of SEQ ID NO: 15.

Dendritic cells genetically modified using the Sendai virus vector are useful to stimulate T cells of patients themselves *in vivo.* In addition, these genetically-modified dendritic cells are also useful to stimulate T cells *in vitro.* A sensitized T cell may be administered to patients to stimulate the patient's immune system by *ex vivo* immunotherapy.

The present disclosure relates to a method for producing T cells stimulated with dendritic cells, which includes the steps of: (a) contacting a: Sendai virus vector with an immature dendritic cell as defined in the claims; (b) differentiating the cell into mature dendritic cell; and (c) contacting the mature dendritic cell with a T cell. The antigen to be presented by the dendritic cell may be a protein (or a processed product thereof) expressed from the vector or a protein exogenously pulsed into the dendritic cell. The dendritic cell introduced with the Sendai virus vector activates T cells and induce CTLs.

The present disclosure also relates to a method for stimulating the immune system using a dendritic cell produced by the method of the present invention. For example, patients suffering from infectious disease, cancer, or the like can be treated to stimulate their immune system. This method includes the step of administering a dendritic cell or a T cell. Specifically, the method includes the step of (a) administering into a patient a therapeutically effective amount of dendritic cells introduced with the Sendai virus vector; or (b) administering into a patient a therapeutically effective amount of T cells stimulated by dendritic cells introduced with the Sendai virus vector. The Sendai virus vector may carry genes encoding one or more antigens or cytokines associated with diseases or may carry no foreign gene. Since the Sendai virus vector activates dendritic cells by infecting the cells, dendritic cells infected with the vector carrying no foreign gene can also activate patients' immune system. More highly effective dendritic cells can be obtained by pulsing dendritic cells with an antigen peptide to present the desired antigen. Alternatively, when T cells are contacted with dendritic cells *in vitro,* it is preferred to collect T cells from a patient and carry out *ex vivo* administration.

The appropriate *in vivo* dose of the vector varies depending on the disease, patient's weight, age, sex, and symptom, purpose of administration, form of administered composition, administration method, transfer gene, and the like, but can be appropriately determined by those skilled in the art. The route of administration can be appropriately selected, and includes, for example, percutaneous, intranasal, transbronchial, intramuscular, intraperitoneal, intravenous, intraarticular, and subcutaneous administration. The administration may be local or systemic. It is preferred to administer the vector at a dose within the range of preferably about $10^5$ to about $10^{11}$ CIU/ml, more preferably about $10^7$ to about $10^9$ CIU/ml, and most preferably about 1 x $10^8$ to about 5 x $10^8$ CIU/ml, in a pharmaceutically acceptable carrier. The amount per dose for human is preferably 2 x $10^5$ to 2 x $10^{11}$ CIU, which is administered once or more within a range where the side effects are clinically acceptable. The same applies to the number of doses per day. Regarding nonhuman animals, for example, a dose converted from the above-described dose based on the body weight ratio between the subject animal and human or the volume ratio (*e.g.*, mean value) of the target site for administration. In addition, when it becomes necessary to suppress the proliferation of the transmissible Sendai virus vector after administration to subjects or cells due to the completion of treatment, through the administration of an RNA-dependent RNA polymerase inhibitor the proliferation of the viral vector can be specifically suppressed without damaging the host.

For *ex vivo* administration, the vector is contacted with dendritic cells *ex vivo* (for example, in a test tube or dish). It is preferable to administer at a MOI of between 1 to 500, more preferably 2 to 300, even more preferably 3 to 200, still more preferably 5 to 100, and yet more preferably 7 to 70. The subject to which the vector is administered is not particularly limited, and includes, for example, birds and mammals (human and nonhuman mammals), including chicken, quail, mouse, rat, dog, pig, cat, bovine, rabbit, sheep, goat, monkey, and human, and other vertebrates.

When administering a dendritic cell introduced with the vector, the cell can be generally injected by intramuscular, intraperitoneal, subcutaneous, or intravenous injection, or direct injection into the lymph node. Preferably, the cell is administered into patients by subcutaneous or intraperitoneal injection, or direct injection into the lymph node. Patients can be administered typically with $10^5$ to $10^9$ transformed dendritic cells, preferably $10^6$ to $10^8$ cells, and more preferably about $10^7$ cells.

Dendritic cells introduced with the Sendai virus vector are useful as an antitumor agent. For example, tumor growth can be suppressed by administering, into tumor sites, dendritic cells introduced with the vector. The tumor site refers to tumor and its surrounding area (for example, an area within 5 mm from the tumor, preferably within 3 mm from the tumor). Although the vector is expected to cause anti-tumor effect even if it does not carry a foreign gene, a stronger effect can be obtained by letting the vector carry an IFN-beta gene. A stronger effect can be obtained by contacting a tumor antigen with the dendritic cells prior to administration into tumors. The contact of a tumor antigen with the dendritic cells can be carried out by using a method wherein a tumor cell lysate is mixed with the dendritic cells, a method wherein the dendritic cells are pulsed with a tumor antigen peptide, or a method wherein a tumor antigen gene is introduced into and expressed by the dendritic cells. Furthermore, anti-tumor effects can be achieved by directly injecting IFN-beta or a vector carrying an IFN-beta gene into tumors. For example, a Sendai virus vector carrying an IFN-beta gene is a superior antitumor agent. A greater anti-tumor effect can be exerted by combining the administration of the dendritic cells introduced with the Sendai virus vector and the injection of a vector carrying an IFN-beta gene into tumor sites.

When T cells activated with the dendritic cells are administered, for example, the T cells can be administered at a dose of about $10^5$ to $10^9$ cells, preferably $10^6$ to $10^9$ cells, and more preferably $10^8$ to $10^9$ cells per $1m^2$ body surface area by intravenous injection (see Ridell et al., 1992, Science 257: 238-241). The injection can be repeated at desired intervals (for example, monthly). After the administration, recipients may be monitored for any side effects during or after T cell injection, if required. In this case, it is preferred that T cells are obtained from the same patient from whom the dendritic cells have been derived. Alternatively, the T cells may be collected from a patient, while the dendritic cells to stimulate the T cells may be derived from an HLA-compatible healthy donor. Conversely, the dendritic cells may be collected from a patient, while the T cells may be derived from an HLA-compatible healthy donor.

Cells containing the dendritic cells as the active ingredient of vaccines that are produced according to the present invention are inoculated as therapeutic vaccines to the human body. Thus, it is safer to make them deficient in growth capacity. For example, it is known that the growth capacity of cord blood-derived monocytes is extremely reduced after the induction of differentiation. However, to use the cells as safer cell vaccines, the growth capacity can be reduced or eliminated without losing the vaccine function by treating the cells with heat, radiation, mitomycin C, or the like. For example, when X-ray irradiation is used, X-ray can be irradiated at a total radiation dose of 1000 to 3300 Rad. With regard to the mitomycin C treatment, mitomycin C can be added to the dendritic cells at a concentration of 25 to 50 μg/ml and incubated at 37°C for 30 to 60 minutes. When the cells are treated with heat, for example, the cells can be subjected to a heat treatment of 50 to 65°C for 20 minutes.

## Examples

Hereinbelow, the present invention is specifically described in the context of Examples; however, it is not to be construed as being limited thereto.

### A. Examination on introduction efficiency:

[Experiment 1]

Monocytes from healthy donors were enriched through negative selection. RosetteSep™-human monocyte enrichment cocktail (Stem Cell Technology Inc.) was used in the negative selection to enrich the monocytes. Specifically, a tetrameric antibody (consisting of two antibody molecules linked together; one is anti-glycophorin A antibody that recognizes erythrocytes, and the other an antibody that recognizes a surface antigen of mononuclear cells) was used to bind cells to be removed to erythrocytes, and the cells were removed using Ficoll Paque™ Plus (Pharmacia Biotech Inc.). Through negative selection, cells expressing CD2, CD3, CD8, CD19, CD56, and CD66b were eliminated, and the remaining cells were used as monocyte-enriched cells in the following induction of DC differentiation. At this stage, 65-80% were $CD14^+$ cells. GM-CSF (500 U/ml) and IL-4 (250 U/ml) were added to the monocyte-enriched cells, and the cells were cultured in endotoxin-free RPMI supplemented with 10% FCS to prepare DCs. After 3 to 4 days, half of the culture supernatant was exchanged with fresh culture medium having the same composition. The cells were confirmed to be positive in the expression of costimulatory molecules, and CD11c, HLA-class II (DR, DP, and DQ), and CD1a, and not to present other lineage markers (CD3, CD56, CD 19, CD15, and CD14) (Fig. 1, and data not shown). These cells were used to test the efficiency of vector introduction. At this stage, 90 to 98% of the viable cells expressed DC markers (CD11c, and HLA-class II (DR, DP, and DQ)).

Although the above-described kit was used in the selection in this Example, a similar selection can also be performed by using antibody-coated magnetic beads. The use of the beads is preferred to preparing cells on a large scale; such as to collect mononuclear cells through blood cell separation or the like.

[Experiment 2]

Sendai virus vector (SeV-GFP) (transmissible; WO 00/70070) that expresses the green fluorescent protein (GFP) was infected to the DCs obtained in Experiment 1 (7 days after differentiation induction) at various MOIs. Changes in the cell count, the expression level of GFP, and the expression levels of costimulatory molecules were investigated over time. The result showed that %GFP reached the maximal level when the MOI was 20 or greater (Figs. 2 to 5). The mean fluorescence intensity (MFI) of GFP can be further increased when the MOI is increased up to 100 (data not shown). Further, the MFI of GFP increased up to day 8. The level of costimulatory molecules (CD80 and CD86) as a whole became maximal when the MOI was 20 or greater. Regarding the decrease in cell count, less change was observed within the MOIs of 1 to 20, and a slight decrease was observed at a MOI of 50 without significant difference (Fig. 6).

[Experiment 3]

The DCs were infected with SeV-GFP at a MOI of 20, and the expression of GFP was examined over time using FACS. As a result, the expression decreased after two weeks (the cell count was also decreased) but GFP expressing cells were detectable up to two months later (Fig. 7). As described in the Example below, DCs are activated by the infection of the minus-strand RNA viral vector. Thus, gene transfer into DCs using the minus-strand RNA viral vector is clinically applicable to vaccination. The administration can be achieved *in vivo* or *ex vivo.* The gene expression can be maintained in the body for a long period by, for example, frequently administering DCs infected with the vector through *ex vivo* administration.

[Experiment 4]

The activation and infection efficiency were examined. It was examined whether the efficiency of vector infection was altered by the activation. DCs cultured for 7 days were stimulated with LPS (1 μg/ml) for two days, infected with SeV-GFP at a MOI of 30, and after 2 days GFP was analyzed by FACS. Alternatively, 2 days after SeV-GFP infection, LPS stimulation (for two days) was carried out under the same condition. (Figs. 8 and 9)

Results: human DCs were found to be nearly 60% positive in %GFP after activation with LPS. In contrast, in mouse DCs, the positivity rate was very low (data not shown). However, MFI was also very low in human, showing a drastic decrease in the efficiency of gene transfer into DCs after activation. In contrast, the efficiency of gene transfer was not altered by LPS stimulation after vector introduction. These results demonstrate that it is preferable to use immature DCs, i. e. non-activated DCs, for gene transfer into DCs using the minus-strand RNA viral vector.

[Experiment 5]

The contact time required for infection was examined (Fig. 10). The results demonstrate that gene transfer can be achieved within about 30 minutes or less.

[Experiment 6]

A previous report described success in producing gene-transferred DCs through the introduction of genes into CD34 cells and the induction of differentiation into DCs (J. Immunol. Meth. 2002; 153-165). A similar method was conducted for SeV-GFP. CD34 positive stem cells (CD34 > 90%) were separated from human cord blood using CD34 microbeads. After infection at a MOI of 0, 10, or 100, the cells were washed well. The cells were cultured in RPMI + 10% FCS supplemented with SCF (50 ng/ml), GM-CSF (500 U/ml), and TNF-alpha (50 ng/ml) for 3 days, then, passaged in a medium supplemented with SCF (50 ng/ml), GM-CSF (500 U/ml), IL-4 (250 U/ml), and TNF-alpha (50 ng/ml) (half of the medium was exchanged every 3 to 4 days), and GFP expression was examined 13 days after the vector infection. As a result, the gene transfer efficiency reached 65 to 70%, and DCs having better expression efficiency of GFP than those prepared with other vectors were prepared. By analyzing the expression of costimulatory molecules, more activated DCs were recovered from the infected DCs than uninfected DCs. (Figs. 11 and 12).

According to the Examples described above, it was demonstrated that the introduction efficiency of the minus-strand RNA virus is considerably higher than that of lentivirus or retrovirus, and an efficiency comparable to that of adenovirus can be achieved rapidly and very easily. In addition, it was found that the activation markers were not altered by using other vectors; however, DC activation can be induced by the infection of the minus-strand RNA virus.

B. Evaluation of DC function after introduction

[Experiment 1]

DCs were infected with SeV-GFP at a MOI of 30 to 50. On the following day, the cells were stimulated by LPS (for 2 days) and tested for the expression of costimulatory molecules. As controls, the conditions of LPS stimulation alone, SeV-GFP infection alone, and no LPS stimulation nor SeV-GFP infection were examined and compared.

Results: The obtained results demonstrate that DC activation occurs by SeV infection alone.

Comparable to LPS: CD80(+) HLA-DR(-) CD83(-)

Higher than LPS: CD86(+) CCR7(-)

Lower than LPS: CD40(-)

(+) indicates where synergistic effect can be obtained by using LPS and SeV. (Figs. 13 to 15)

[Experiment 2]

DCs were infected with SeV-GFP at a MOI of 30 (some groups were stimulated with LPS on the next day of the infection or 3 days after the infection). The phagocytic activity was examined in the groups similarly to those as described in Experiment 1 (1 μm PCV-RED latex-microspheres were used. The bar graphs represent the activity after subtraction of positive background at 4°C).

Results: The phagocytic activity was found to be reduced in the cells infected with SeV due to the activation as was shown by activation markers. In particular, the higher the expression level of GFP, the lower the phagocytic activity. Thus, for example, when a tumor cell lysate is used to present tumor antigens on DCs, it is preferred to co-culture DCs with the lysate before the introduction of the minus-strand RNA viral vector into DCs. (Figs. 16 to 17)

[Experiment 3]

To examine the cytokine-producing ability of dendritic cells associated with the activation of the dendritic cells by SeV, monocyte-derived dendritic cells (MoDCs) obtained by 7 days of culture were cultured in 12-well plates for 48 hours (8 x $10^5$/2ml/well: medium supplemented with X-vivo 15™, 2% autoserum, GM-CSF (500 U/ml), and IL-4 (250 U/ml)) under the conditions described below. The levels of TNF-alpha, IL-1 beta, IL-6, and IL-8 in the resulting supernatants were measured using Luminex™ system. SeV was infected at a MOI of 30 and the cells were cultured for two days.

- Unstimulated group: a group with the medium alone;
- Allantoic fluid group: a group added with 60 μl of hen egg allantoic fluid (free of SeV), suspension of SeV;
- UV-SeV-GFP group: a group added with 60 μl of SeV-GFP solution whose replication ability is deprived by ultraviolet light irradiation; and
- SeV-GFP group: a group added with 60 μl of SeV-GFP solution (replication-competent SeV).

Results: TNF-alpha, IL-1beta, and IL-6 was produced and the production of IL-8 was increased in only the dendritic cells introduced with GFP gene using the replication-competent SeV (Fig. 18). The increased expression levels of CD40, CD80, CD83, CD86, and HLA-DR on the dendritic cells were induced only by the replication-competent SeV (Figs. 19 and 20). These results suggest that the production of inflammatory cytokines, which are important in immune response, can be elicited in dendritic cells merely by introducing SeV into the dendritic cells. It also suggests that not the contact of SeV with receptors on the membrane of dendritic cells at the time of gene transfer into the dendritic cells but the process of SeV RNA amplification after SeV infection is critical to the activation of dendritic cells.

[Experiment 4]

T cell activating ability was examined using the same experimental groups by irradiating the DCs at 3000 rad. (Purified ($CD3^+$ > 95%) allo or syngenic T cells were co-cultured with DCs at various DC doses for 3 days). Syngenic T cells were used as an indicator of response to SeV-GFP.

Results: Due to the low DC ratio and the number of T cells, the differences were relatively insignificant. Nonetheless, it was found that SeV infection alone had an allo T cell-stimulating effect equivalent to LPS (Fig. 21). DCs can also be used without irradiation.

C. Induction of cancer antigen-specific CTLs

Using the method described above in subsection A, $CD14^+$ cells were enriched from human peripheral blood (healthy donors with HLA-A 0201), and immature dendritic cells were prepared using x-vivo 15™ (Cambrex) + 2% autoserum as a medium, supplemented with GM-CSF (500 U/ml), and IL-4 (250 U/ml) (half of the medium was exchanged every 3 to 4 days). The prepared immature dendritic cells were divided into the following three groups, and then further cultured for 48 hours in the presence of GM-CSF (500 U/ml) and IL-4 (250 U/ml):

Group 1: no addition;
Group 2: infected with SeV-GFP (MOI 30); and
Group 3: stimulation by cytokine cocktail (50 ng/ml IL-1β, 500 ng/ml IL-6, 2500 U/ml IFN-α, 100 ng/ml TNF-α, and 20 μM PGE2).

Next, dendritic cells were recovered and pulsed with MART-1 peptide (EAAGIGILTV (SEQ ID NO: 9); 50 μg/ml for 3 hours). T cells in peripheral blood from the same healthy donor from whom the dendritic cells had been obtained were enriched through negative selection ($CD3^+$ > 97%), and were co-cultured with peptide-pulsed dendritic cells of the above three groups for 7 days (X-vivo 15™ + 2% autologous serum). (Half of the medium was exchanged every 3 to 4 days or when the medium changed yellow. The T cells and dendritic cells were co-cultured in the absence of IL-2 at the first stimulation, and 100 U/ml IL-2 was added from the third day.) This treatment was repeated twice. The cells were recovered from each mixed culture fluid and used as effector cells in CTL assay.

T2 cells (TAP deficient cell line, a T cell-B cell hybridoma, obtained from a donor with $HLA\text{-}A2^+$) was used as target cells. These cells lack TAP (the transporter to class I), and therefore are incapable of transferring peptides produced through cytoplasmic proteolysis to class I. Thus, when a peptide is exogenously added, the peptide is loaded onto class I resulting in class I expression. The target cells were pulsed with mutant MART-1 peptide (ELAGIGILTV (SEQ ID NO: 10); a peptide with potentiated HLA-A2 binding ability without any alteration in the T cell receptor recognition site as compared to the peptide used in the above-described stimulation) or with influenza peptide (Flu; a peptide as a third party; GILGFVFTL (SEQ ID NO: 11)), and labeled with Cr. The effector T cells of the above three groups were co-cultured with each of the two types of targets at a ratio of 20:1, 10:1, 5:1, or 2.5:1 for four hours to examine the CTL activity.

The combinations used in the experiment are summarized below.

| Effector cells | Target cells | Symbols in the figure |
|---|---|---|
| Effector T cells of Group 1 | Mutant MART1 peptide + T2 cells | Solid line with closed squares |

(continued)

| Effector cells | Target cells | Symbols in the figure |
|---|---|---|
| Effector T cells of Group 2 | Mutant MART1 peptide + T2 cells | Solid line with closed triangles |
| Effector T cells of Group 3 | Mutant MART1 peptide + T2 cells | Solid line with closed inverted triangles |
| Effector T cells of Group 1 | Flu peptide + T2 cells | Dotted line with closed diamonds |
| Effector T cells of Group 2 | Flu peptide + T2 cells | Dotted line with closed circles |
| Effector T cells of Group 3 | Flu peptide + T2 cells | Dotted line with open squares |

Results: MART-1 specific CTL cannot be induced when the T cells are stimulated by the non-activated DCs (MART1 peptide +) among the three groups described above. However, as a positive control, when T cells were stimulated using dendritic cells that were activated by cytokines (a method which most intensively activates cells among the current dendritic cell therapy for anti-tumor immunity), MART-1 specific CTLs could be induced (a similar result was obtained when, instead of the mutant MART-1 peptide, the MART-1 peptide as used in the stimulation was used to pulse the target). When dendritic cells introduced with genes using SeV were used, a CTL activity comparable to the positive control was obtained (Fig. 22). Specifically, when determined by the CTL assay, it was shown that dendritic cells were activated by SeV infection alone, and that they can induce CTLs *in vitro* to the same level as dendritic cells activated by cytokines. When SeV is used for T cell activation, the activation can be achieved simultaneously with the introduction of the target gene, which makes it unnecessary to add activation factors, such as cytokines, and thus contributes to cost reduction, time saving, and retaining cell viability.

D. Introduction effects of immunostimulatory cytokine genes

It was examined *in vivo* whether dendritic cells activated by SeV can exert anti-tumor immunity. A B16 melanoma-transplanted model that expresses MHC class I at only a very low level and exhibits poor immunogenicity was used as a tumor model. C57BL/6 mice (6- to 8-week-old; female) (CHARLES RIVER JAPAN, INC.) were used as the tumor model mice, and dendritic cells were collected from C57BL/6 mice (8-week-old; female) (CHARLES RIVER JAPAN, INC.). The dendritic cells were obtained by collecting bone marrow from thigh bones of C57BL/6 mice; removing T cells using SpinSep™, murine hematopoietic progenitor enrichment cocktail (anti-CD5 antibody, anti-CD45R antibody, anti-CD11b antibody, anti-Gr-1 antibody, anti-TER119 antibody, anti-7/4 antibody; Stem Cell technology); then culturing the cells for one week with the addition of IL-4 and GM-CSF. On day 0, 1 x $10^5$/100 μL of B16 melanoma cells were subcutaneously (s.c.) injected into the abdominal are of the mice. On days 10, 17, and 24, dendritic cells without stimulation for activation, dendritic cells activated with LPS (LPS DC), or dendritic cells activated by introducing SeV-GFP or SeV-IFNβ expressing mouse interferon β (SeV GFP DC and SeV IFNβ DC, respectively) were administered in the area surrounding the tumor. Simultaneously, another experiment was carried out, wherein the dendritic cells were administered after the pulsing with tumor antigens (tumor lysate obtained by freeze and thaw of B16). In addition to these experiments, SeV-IFNβ was directly injected intratumorally 10 days after tumor injection (day 10) to examine the anti-tumor effect.

SeV was introduced into dendritic cells by infecting dendritic cells cultured for one week as described above with SeV-IFNβ at a MOI of 40, and culturing the cells for 8 hours. When pulsing dendritic cells with tumor antigens, dendritic cells cultured for one week as described above were recovered and pulsed with tumor lysate as the tumor antigens (DC : tumor lysate = 1:3), cultured for 18 hours, infected with SeV-IFNβ at a MOI of 40, and cultured for 8 hours. Then, these dendritic cells were recovered and administered at a cell number of 5 x $10^5$ to 10 x $10^5$ cells in an area surrounding the tumor of the mice.

As shown in Fig. 23, when SeV-IFNβ was directly injected intratumorally, tumor growth was suppressed for 2 weeks after the injection. However, thereafter, the regrowth of tumors was apparent. When DC/SeV-GFP was used, significant anti-tumor effects could be observed, with the strongest tumor suppression being observed in mice treated with DC/LPS and mice treated with DC/SeV-IFNβ.

The anti-tumor effect in each of the therapeutic groups described above was closely examined. To assay natural killer (NK) cell activity, spleens were excised from mice of each of the therapeutic groups described above after 7 days from the end of three rounds of DC therapy to prepare effector cells. $^{51}Cr$ release assay was performed using Yac-1 as the target. Further, to assay the cytotoxicity of T lymphocytes, the residual spleen cells from the NK cell activity assay described above were cultured for 5 days with TRP-2 peptide, a B16 tumor antigen, to use them as effector cells. The effector cells were co-cultured with EL-4 target cells pulsed with mTRP-2 peptide, and then $^{51}Cr$ release assay was performed. The rate of specific $^{51}Cr$ release was calculated as follows:

$$[(\text{sample (cpm)} - \text{spontaneous emission (cpm)}) / (\text{maximum emission (cpm)} - \text{spontaneous emission (cpm)})] \times 100$$

where the maximum emission was determined using target cells incubated with 1% triton X, while spontaneous emission was determined using target cells incubated with culture medium alone.

The activation of natural killer (NK) cells was detected only in mice that were directly injected with vectors, and not in the dendritic cell injection group (Fig. 24). In contrast, the activation of cytotoxic T lymphocytes (CTLs) was maximal in the DC/LPS treated group and mice treated with DC/SeV-IFNβ, slightly lower in the DC/SeV-GFP treated group, and was not detected in the group of SeV-IFNβ direct injection (Fig. 25). The tumor lysate pulsing had no significant influence on tumor growth nor on CTL response. Thus, it was demonstrated that anti-tumor therapeutic effects were exerted by tumor immunotherapy using dendritic cells introduced with immunostimulatory cytokine genes by SeV. Although there was a slight difference in the CTL activities between the DC/LPS-treated group and the DC/SeV-IFNβ-treated group, their anti-tumor effects were found to be comparable. Since the mechanism of anti-tumor effect induced by direct injection of the IFNβ expression vector is different from that induced by IFNβ expression via dendritic cells, treatments combining them are expected to be more effective.

Industrial Applicability

The present invention facilitates the efficient introduction of genes into dendritic cells. Dendritic cells produced according to the present invention are preferably used to induce protective immunity against viruses, bacteria, and the like, and also in anti-cancer immunotherapy and the like. Since dendritic cells have strong ability to induce immunity, DC vaccine that induces antigen-specific cellular immunity can be produced by introducing a desired antigen gene or immune-activating gene into dendritic cells using the method of the present invention.

**Claims**

**1.** A method for producing a mature dendritic cell, consisting of contacting a Sendai virus vector with a CD11c$^+$, HLA-class II$^+$, CD1a$^+$, CD3$^-$, CD19$^-$, CD20$^-$, CD56$^-$, CD 14$^-$ and CD 15$^-$ immature myeloid dendritic cell, thereby inducing maturation of the immature dendritic cell only by contacting with the Sendai virus vector.

**2.** The method of claim 1, wherein the produced mature dendritic cell is a gene transferred mature dendritic cell.

**3.** The method of claim 1, wherein the vector comprises a cytokine gene.

**4.** The method of claim 3, wherein the cytokine is interferon β.

**5.** The method of claim 1, wherein the cell is a human cell.

## Patentansprüche

**1.** Verfahren zur Herstellung einer reifen dendritischen Zelle, bestehend aus dem Inkontaktbringen eines Sendai-Virus-Vektors mit einer unreifen myeloiden CD11c$^+$-, HLA-Klasse II$^+$-, CD1a$^+$-, CD3$^-$-, CD19$^-$-, CD20$^-$-, CD56$^-$-, CD14$^-$- und CD15$^-$- dendritischen Zelle, wodurch die Reifung der unreifen dendritischen Zelle nur durch das Inkontaktbringen mit dem Sendai-Virus-Vektor induziert wird.

**2.** Verfahren nach Anspruch 1, wobei die erzeugte reife dendritische Zelle eine mit einem Gen transferierte reife dendritische Zelle ist.

**3.** Verfahren nach Anspruch 1, wobei der Vektor ein Cytokingen umfasst.

**4.** Verfahren nach Anspruch 3, wobei das Cytokin Interferon-β ist.

**5.** Verfahren nach Anspruch 1, wobei die Zelle eine menschliche Zelle ist.

## Revendications

**1.** Procédé pour produire une cellule dendritique mature, consistant à mettre en contact un vecteur du virus de Sendai avec une cellule dendritique myéloïde immature CD11c$^+$, HLA-classe II$^+$, CD1a$^+$, CD3$^-$, CD19$^-$, CD20$^-$, CD56$^-$, CD14$^-$ et CD15$^-$, induisant de cette manière la maturation de la cellule dendritique immature uniquement par mise en contact avec le vecteur du virus de Sendai.

**2.** Procédé selon la revendication 1, dans lequel la cellule dendritique mature produite est une cellule dendritique mature dans laquelle un gène a été transféré.

**3.** Procédé selon la revendication 1, dans lequel le vecteur comprend un gène codant pour une cytokine.

**4.** Procédé selon la revendication 3, dans lequel la cytokine est l’interféron β.

**5.** Procédé selon la revendication 1, dans lequel la cellule est une cellule humaine.

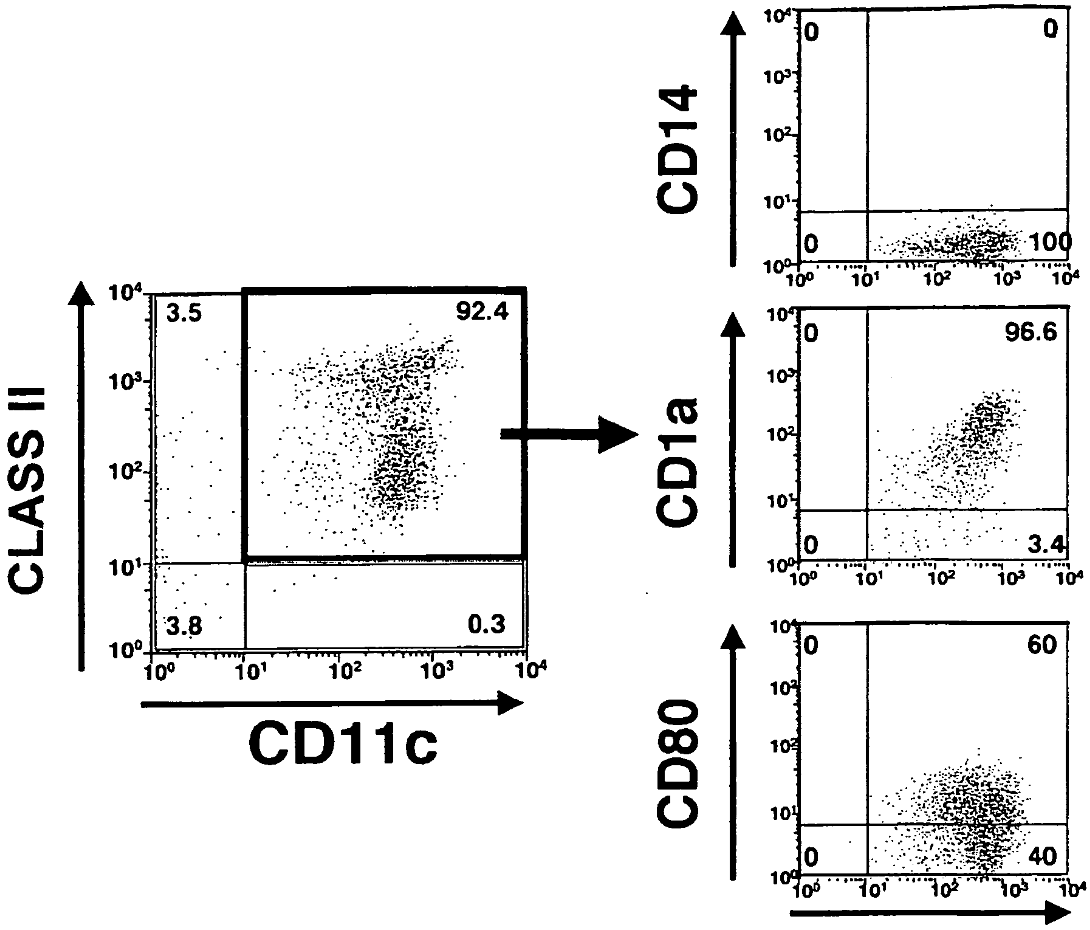
CLASS II
CD11c
3.5
92.4
3.8
0.3
CD14
0
0
0
100
CD1a
0
96.6
0
3.4
CD80
0
60
0
40

FIG. 1

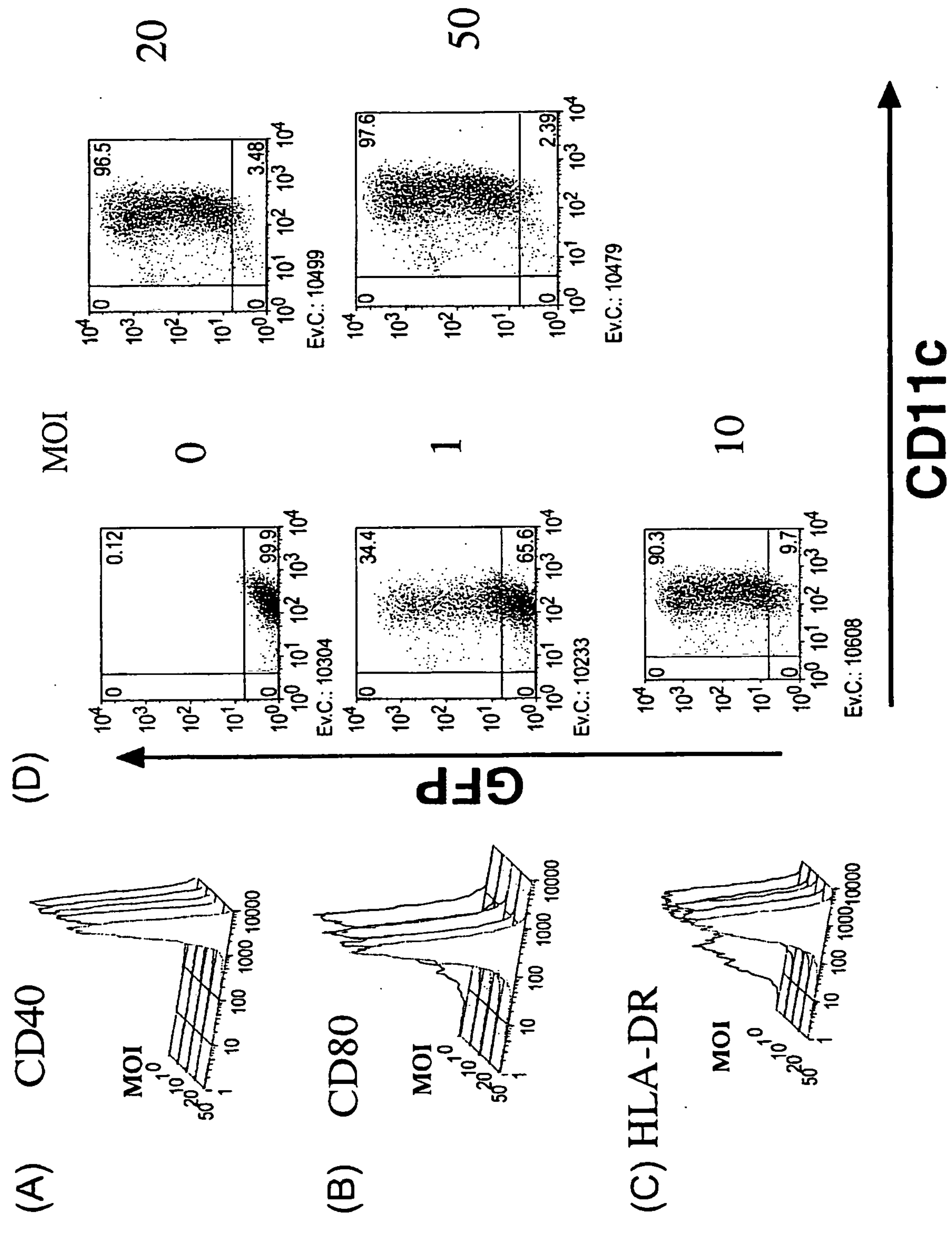
(A) CD40
(B) CD80
(C) HLA-DR
MOI
(D)
GFP
CD11c
MOI
0
1
10
20
50
0.12
99.9
34.4
65.6
90.3
9.7
96.5
3.48
97.6
2.39
Ev.C.: 10304
Ev.C.: 10233
Ev.C.: 10608
Ev.C.: 10499
Ev.C.: 10479

FIG. 2

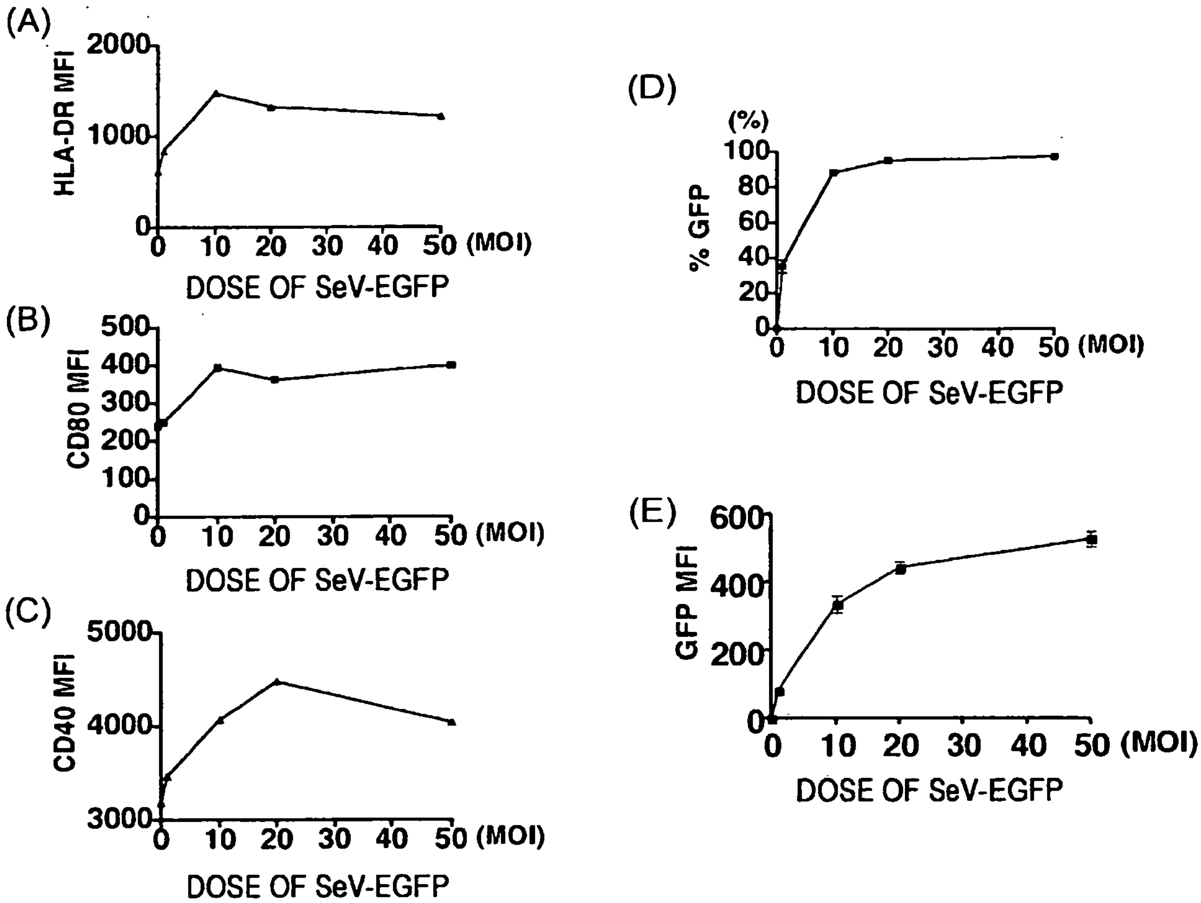
(A)
HLA-DR MFI
2000
1000
0
0 10 20 30 40 50 (MOI)
DOSE OF SeV-EGFP
(B)
CD80 MFI
500
400
300
200
100
0
0 10 20 30 40 50 (MOI)
DOSE OF SeV-EGFP
(C)
CD40 MFI
5000
4000
3000
0 10 20 30 40 50 (MOI)
DOSE OF SeV-EGFP
(D)
(%)
% GFP
100
80
60
40
20
0
0 10 20 30 40 50 (MOI)
DOSE OF SeV-EGFP
(E)
GFP MFI
600
400
200
0
0 10 20 30 40 50 (MOI)
DOSE OF SeV-EGFP

FIG. 3

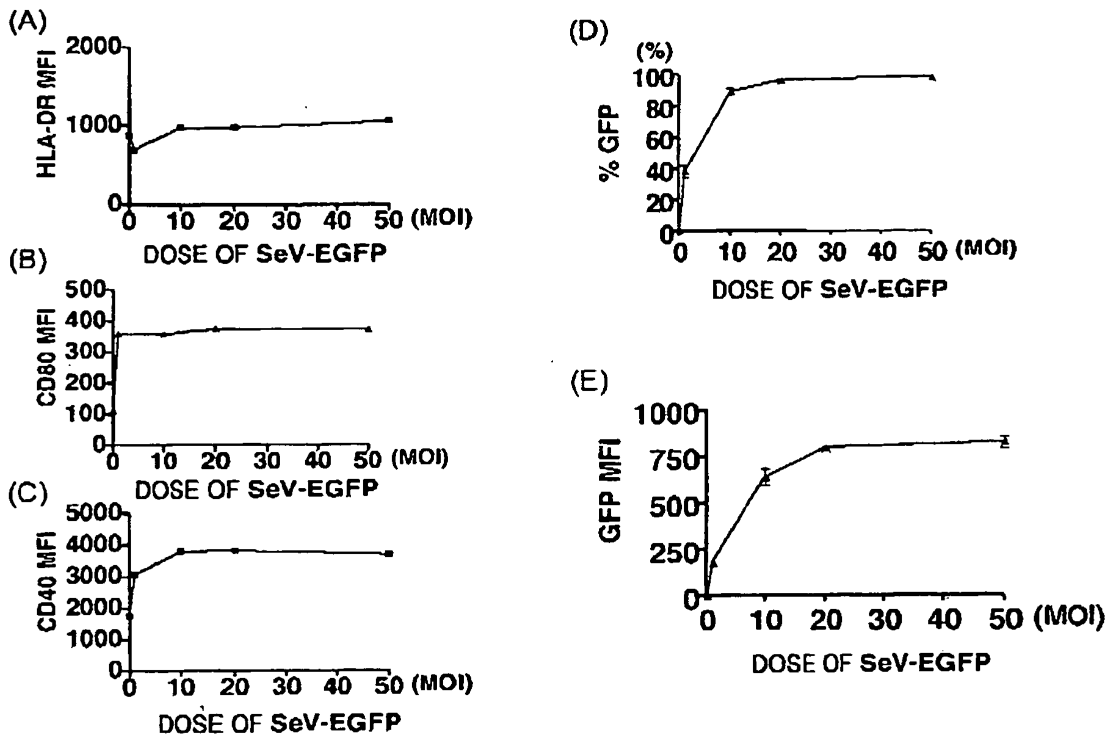
(A)
HLA-DR MFI
2000
1000
0
0 10 20 30 40 50 (MOI)
DOSE OF SeV-EGFP
(B)
CD80 MFI
500
400
300
200
100
0
0 10 20 30 40 50 (MOI)
DOSE OF SeV-EGFP
(C)
CD40 MFI
5000
4000
3000
2000
1000
0
0 10 20 30 40 50 (MOI)
DOSE OF SeV-EGFP
(D)
(%)
% GFP
100
80
60
40
20
0
0 10 20 30 40 50 (MOI)
DOSE OF SeV-EGFP
(E)
GFP MFI
1000
750
500
250
0
0 10 20 30 40 50 (MOI)
DOSE OF SeV-EGFP

FIG. 4

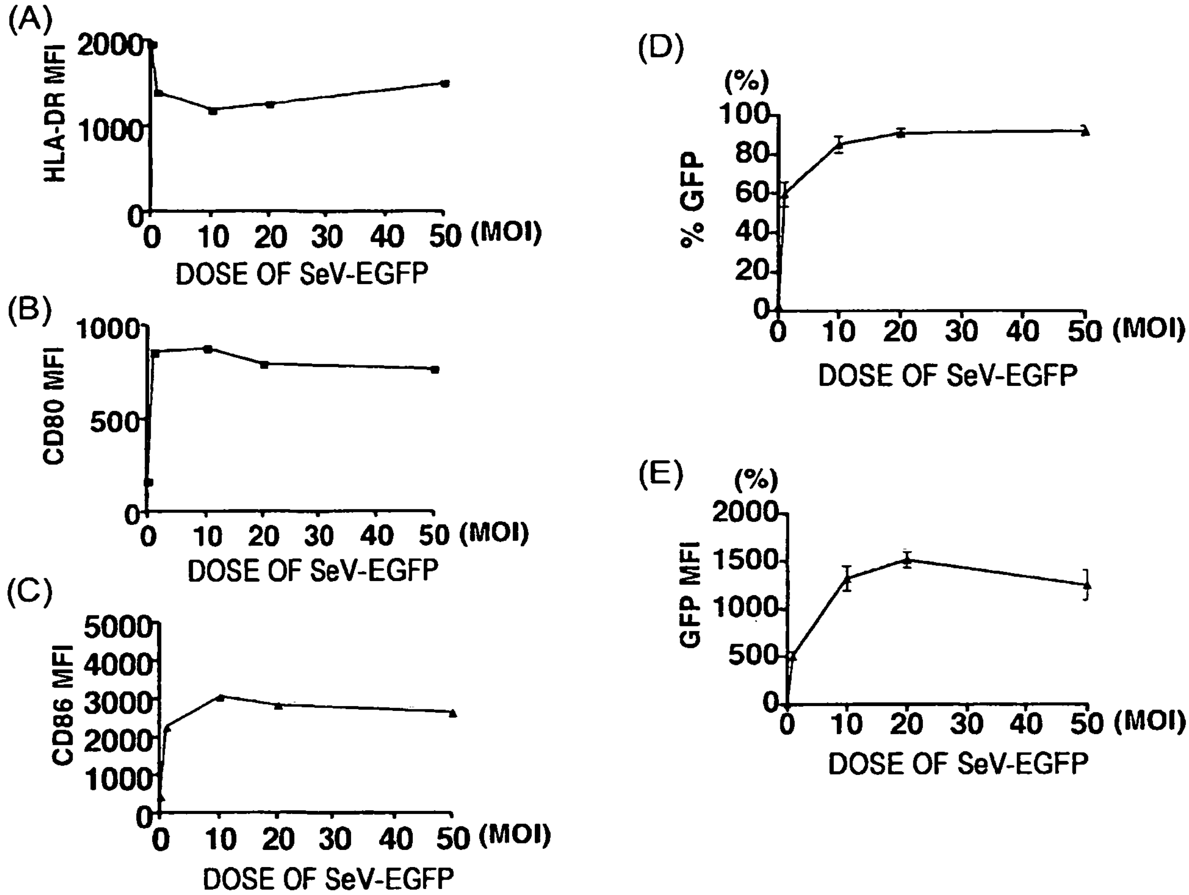
(A)
HLA-DR MFI
2000
1000
0
0 10 20 30 40 50 (MOI)
DOSE OF SeV-EGFP
(B)
CD80 MFI
1000
500
0
0 10 20 30 40 50 (MOI)
DOSE OF SeV-EGFP
(C)
CD86 MFI
5000
4000
3000
2000
1000
0
0 10 20 30 40 50 (MOI)
DOSE OF SeV-EGFP
(D)
(%)
% GFP
100
80
60
40
20
0
0 10 20 30 40 50 (MOI)
DOSE OF SeV-EGFP
(E)
(%)
GFP MFI
2000
1500
1000
500
0
0 10 20 30 40 50 (MOI)
DOSE OF SeV-EGFP

FIG. 5

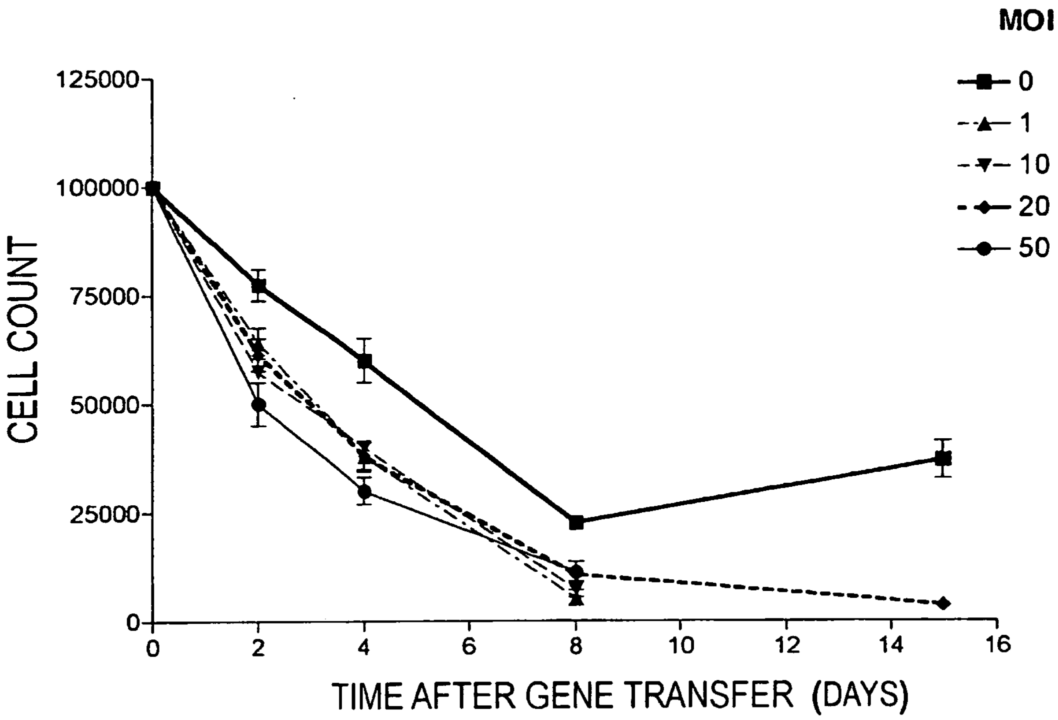
MOI
0
1
10
20
50
125000
100000
75000
50000
25000
0
CELL COUNT
0
2
4
6
8
10
12
14
16
TIME AFTER GENE TRANSFER (DAYS)

FIG. 6

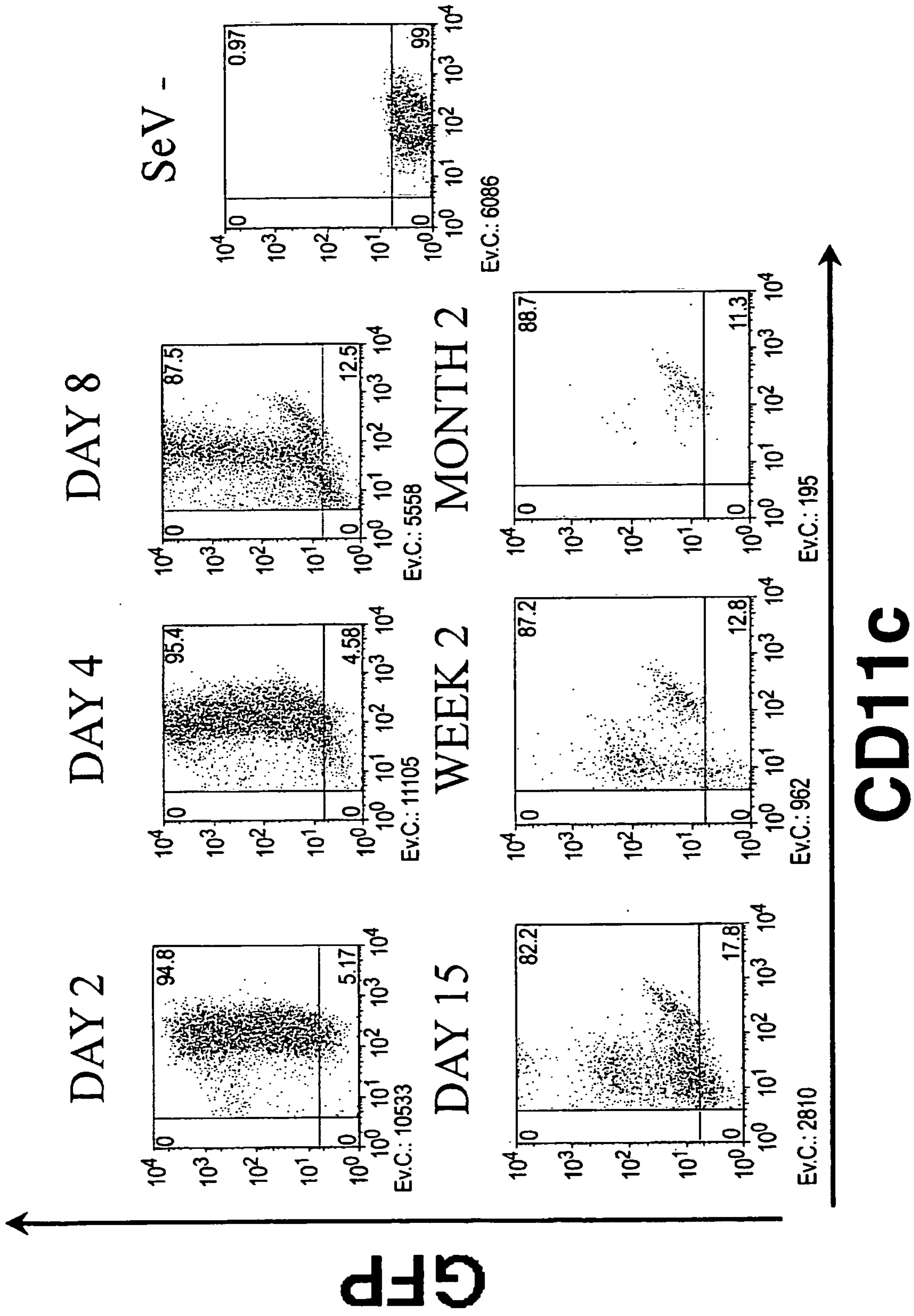

DAY 2
DAY 4
DAY 8
SeV -
DAY 15
WEEK 2
MONTH 2
94.8
5.17
Ev.C.: 10533
95.4
4.58
Ev.C.: 11105
87.5
12.5
Ev.C.: 5558
0.97
99
Ev.C.: 6086
82.2
17.8
Ev.C.: 2810
87.2
12.8
Ev.C.: 962
88.7
11.3
Ev.C.: 195
CD11c
GFP


FIG. 7

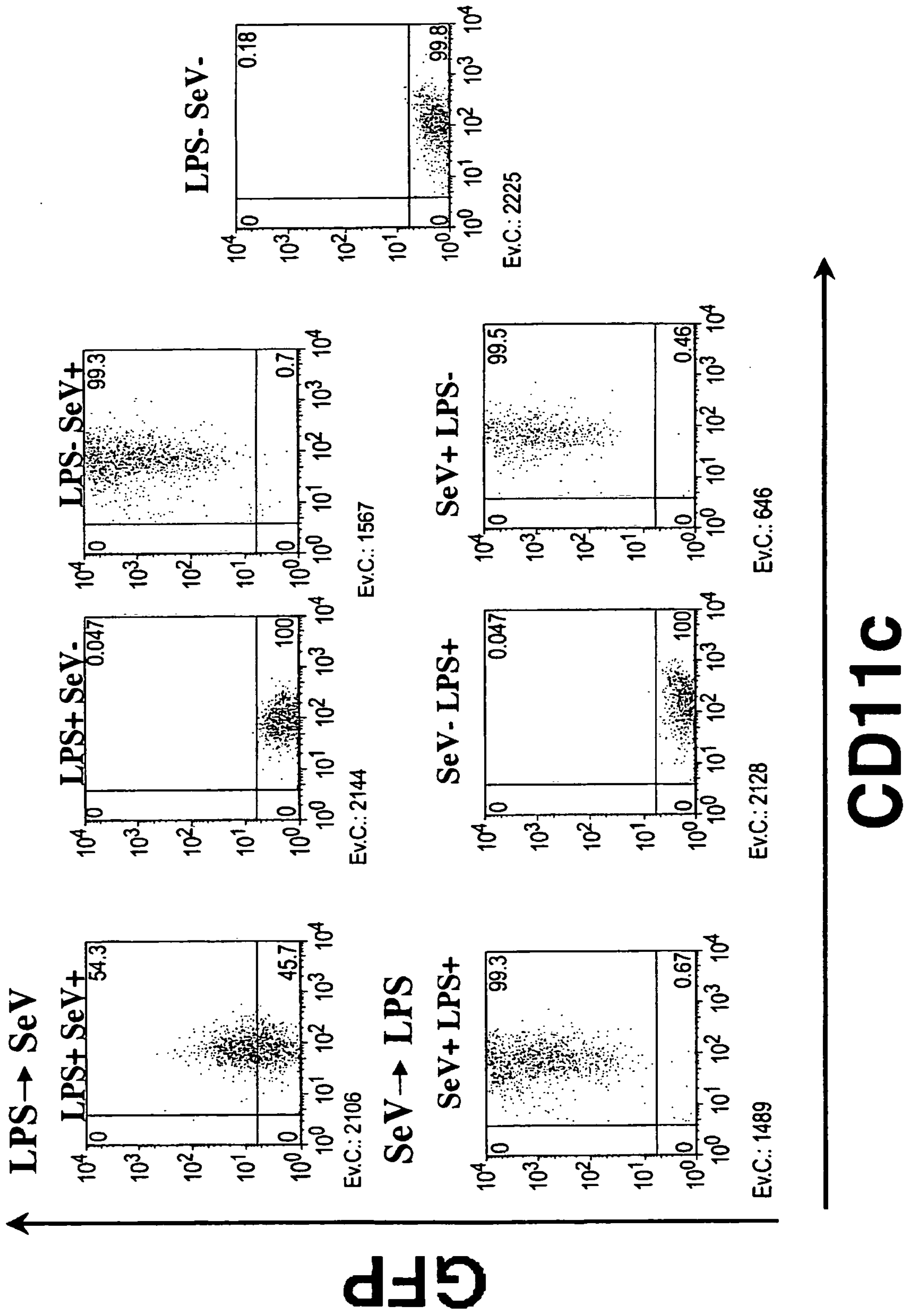
LPS→ SeV
LPS+ SeV+
Ev.C.: 2106
SeV→ LPS
SeV+ LPS+
Ev.C.: 1489
LPS+ SeV-
Ev.C.: 2144
SeV- LPS+
Ev.C.: 2128
LPS- SeV+
Ev.C.: 1567
SeV+ LPS-
Ev.C.: 646
LPS- SeV-
Ev.C.: 2225
GFP
CD11c

FIG. 8

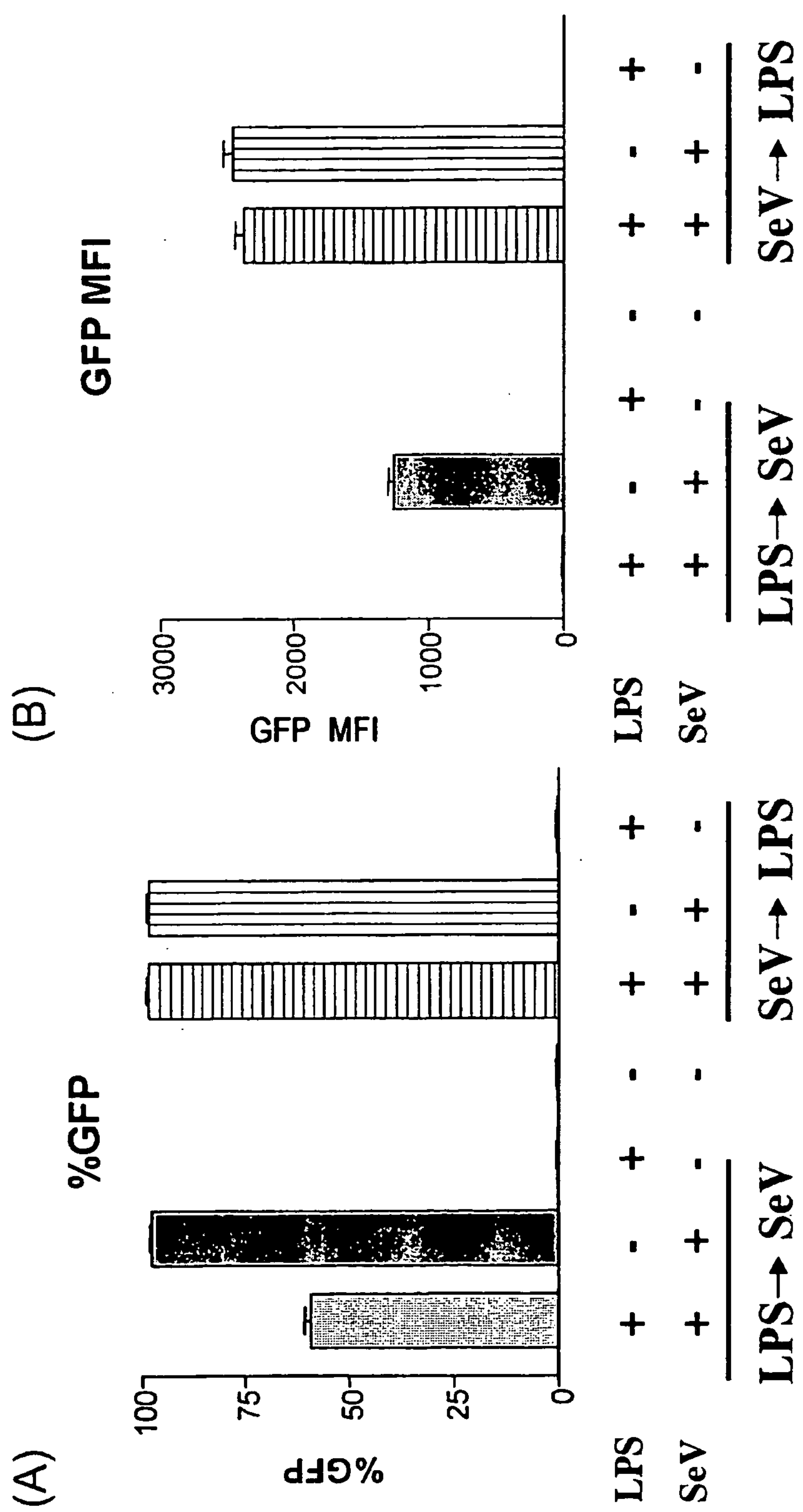
(A)
%GFP
%GFP
100
75
50
25
0
LPS
SeV
LPS→ SeV
SeV→ LPS
(B)
GFP MFI
GFP MFI
3000
2000
1000
0
LPS
SeV
LPS→ SeV
SeV→ LPS

FIG. 9

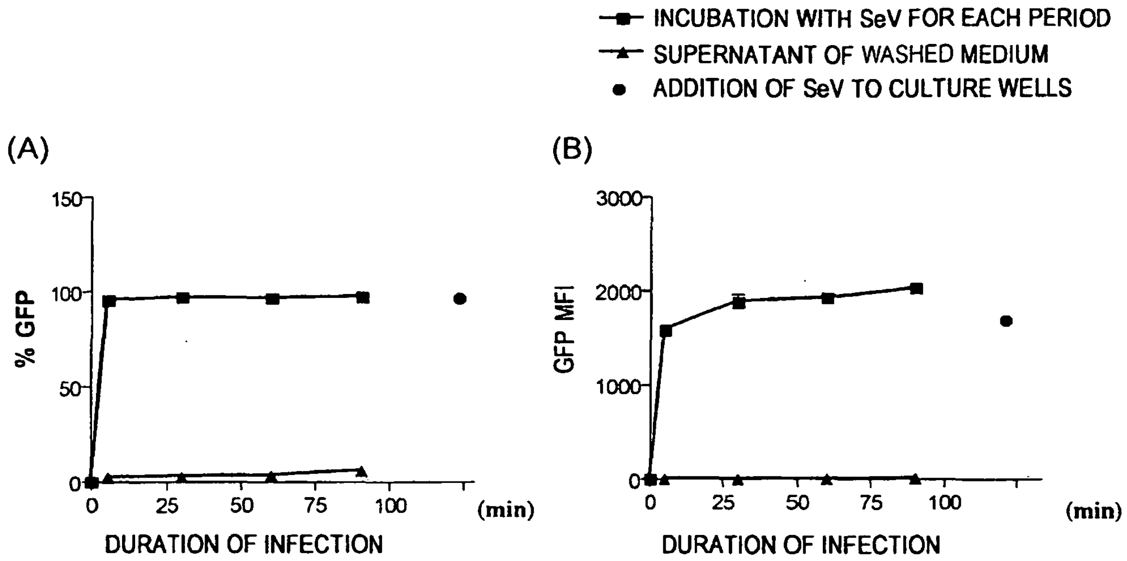
INCUBATION WITH SeV FOR EACH PERIOD
SUPERNATANT OF WASHED MEDIUM
ADDITION OF SeV TO CULTURE WELLS
(A)
150
100
50
0
% GFP
0
25
50
75
100
(min)
DURATION OF INFECTION
(B)
3000
2000
1000
0
GFP MFI
0
25
50
75
100
(min)
DURATION OF INFECTION

FIG. 10

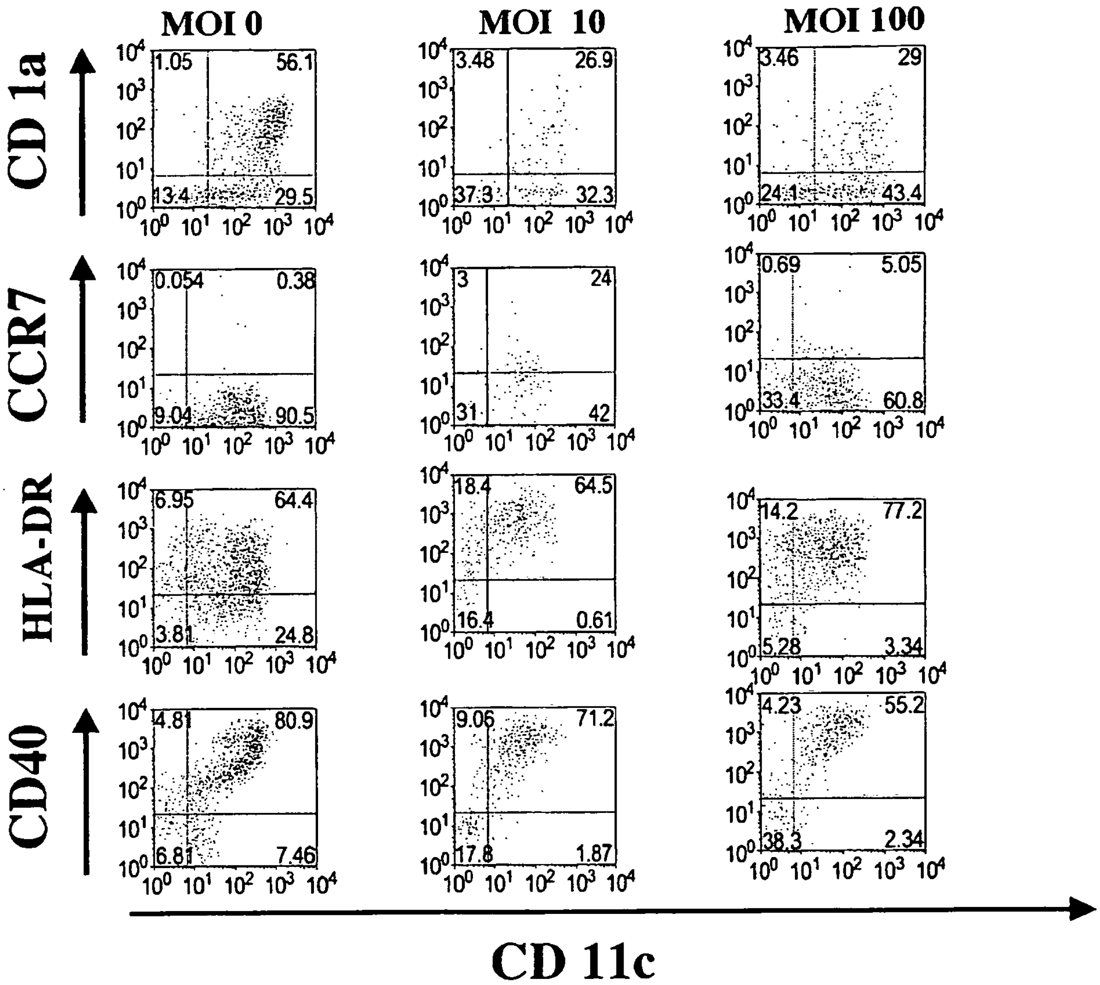
MOI 0
MOI 10
MOI 100
CD 1a
CCR7
HLA-DR
CD40
CD 11c
1.05
56.1
13.4
29.5
3.48
26.9
37.3
32.3
3.46
29
24.1
43.4
0.054
0.38
9.04
90.5
3
24
31
42
0.69
5.05
33.4
60.8
6.95
64.4
3.81
24.8
18.4
64.5
16.4
0.61
14.2
77.2
5.28
3.34
4.81
80.9
6.81
7.46
9.06
71.2
17.8
1.87
4.23
55.2
38.3
2.34

FIG. 11

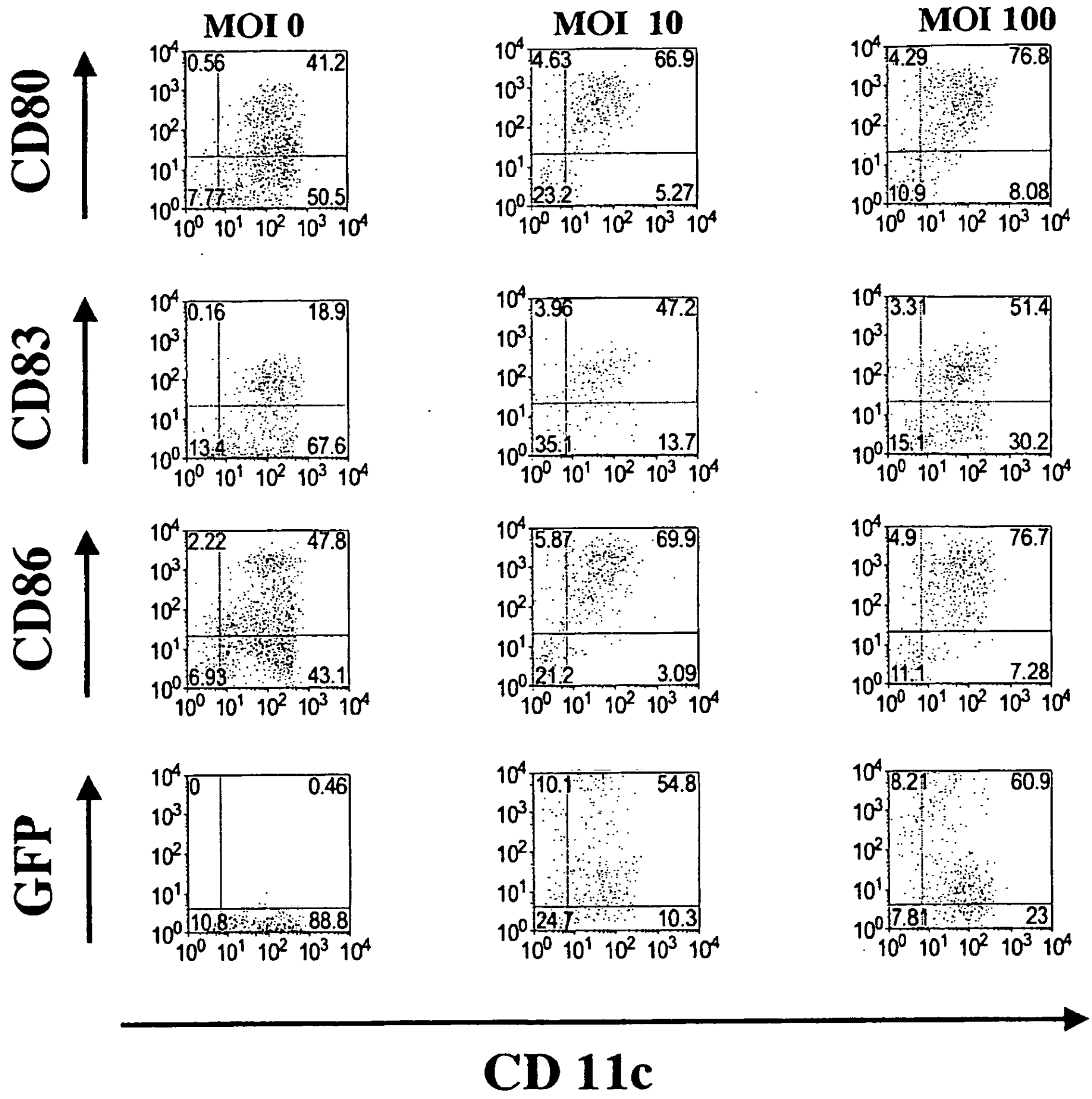
MOI 0
MOI 10
MOI 100
CD80
CD83
CD86
GFP
CD 11c
0.56
41.2
7.77
50.5
4.63
66.9
23.2
5.27
4.29
76.8
10.9
8.08
0.16
18.9
13.4
67.6
3.96
47.2
35.1
13.7
3.31
51.4
15.1
30.2
2.22
47.8
6.93
43.1
5.87
69.9
21.2
3.09
4.9
76.7
11.1
7.28
0
0.46
10.8
88.8
10.1
54.8
24.7
10.3
8.21
60.9
7.81
23

FIG. 12

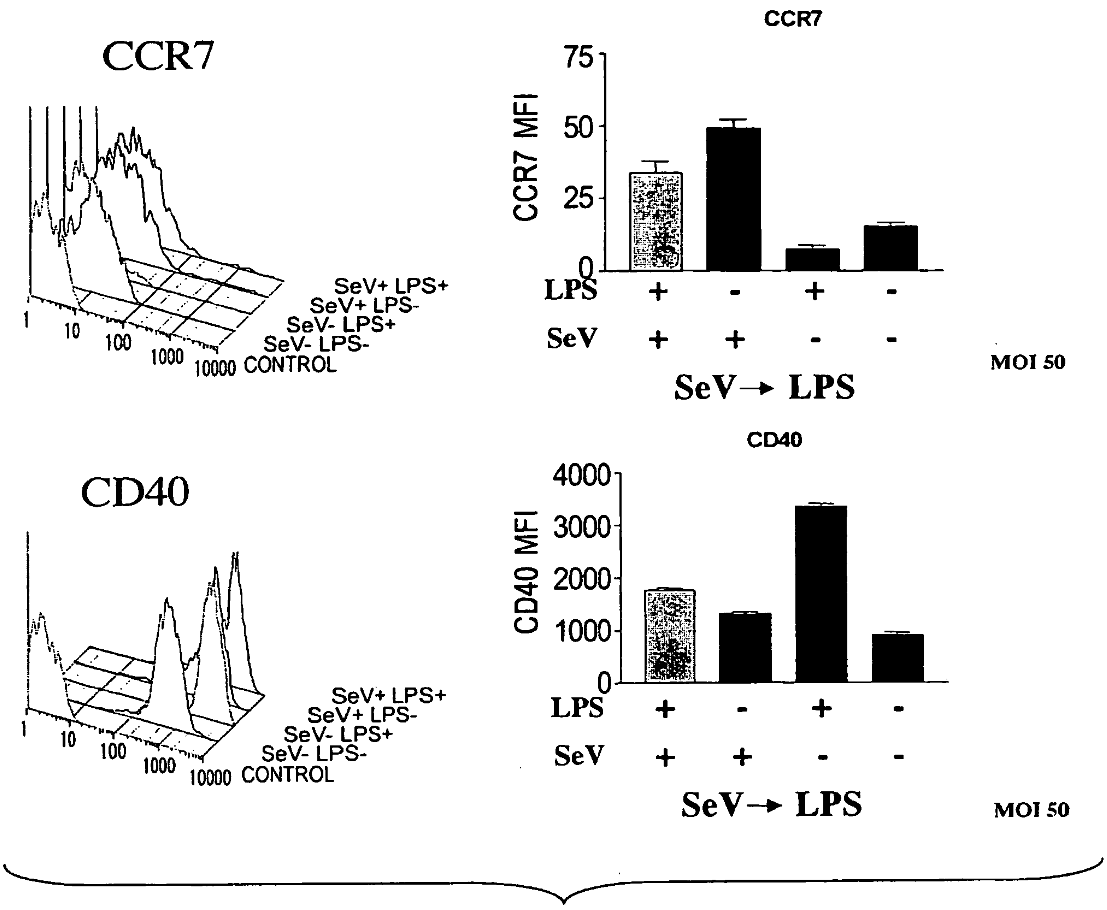
CCR7
1
10
100
1000
10000
SeV+ LPS+
SeV+ LPS-
SeV- LPS+
SeV- LPS-
CONTROL
CCR7
CCR7 MFI
75
50
25
0
LPS + - + -
SeV + + - -
MOI 50
SeV→ LPS
CD40
1
10
100
1000
10000
SeV+ LPS+
SeV+ LPS-
SeV- LPS+
SeV- LPS-
CONTROL
CD40
CD40 MFI
4000
3000
2000
1000
0
LPS + - + -
SeV + + - -
SeV→ LPS
MOI 50

FIG. 13

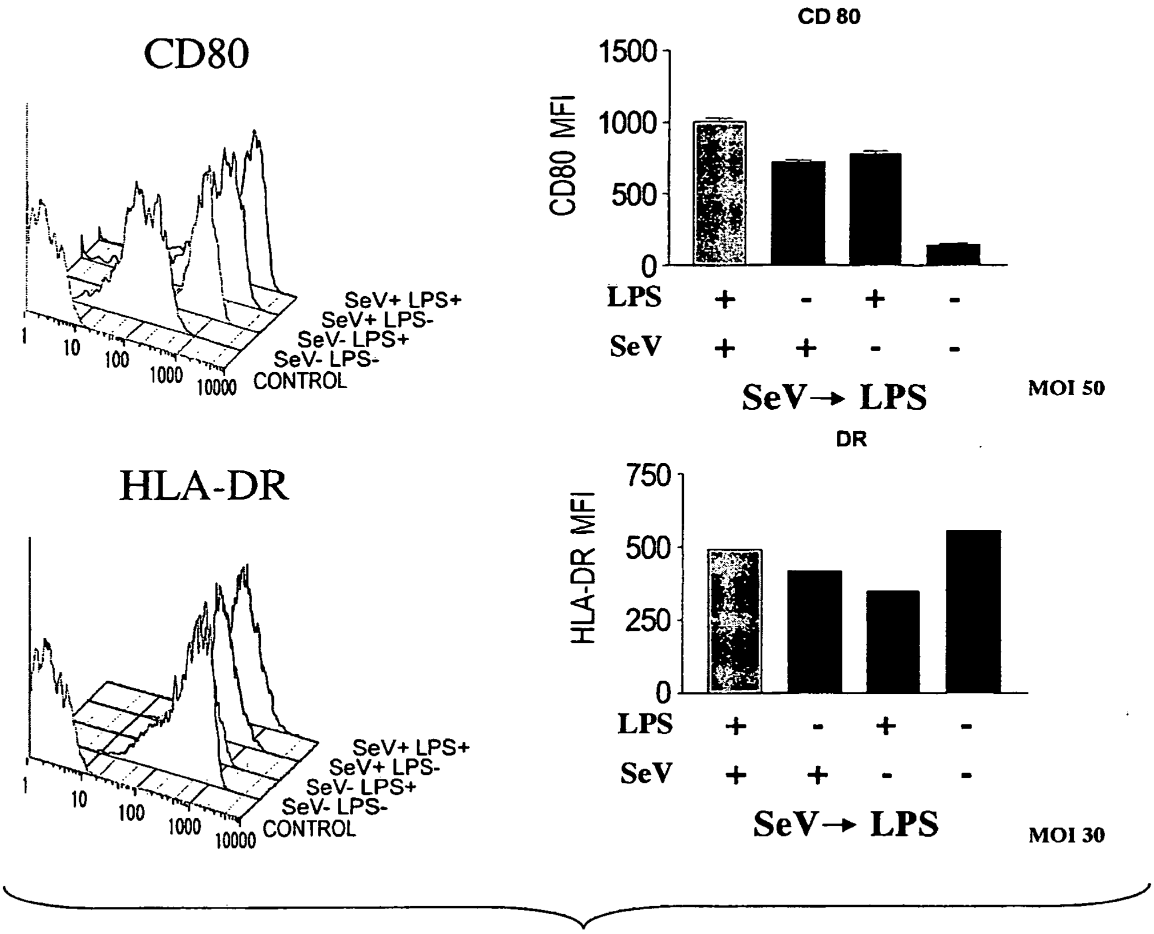
CD80
SeV+ LPS+
SeV+ LPS-
SeV- LPS+
SeV- LPS-
CONTROL
1
10
100
1000
10000
CD 80
CD80 MFI
1500
1000
500
0
LPS + - + -
SeV + + - -
SeV→ LPS
MOI 50
HLA-DR
SeV+ LPS+
SeV+ LPS-
SeV- LPS+
SeV- LPS-
CONTROL
1
10
100
1000
10000
DR
HLA-DR MFI
750
500
250
0
LPS + - + -
SeV + + - -
SeV→ LPS
MOI 30

FIG. 14

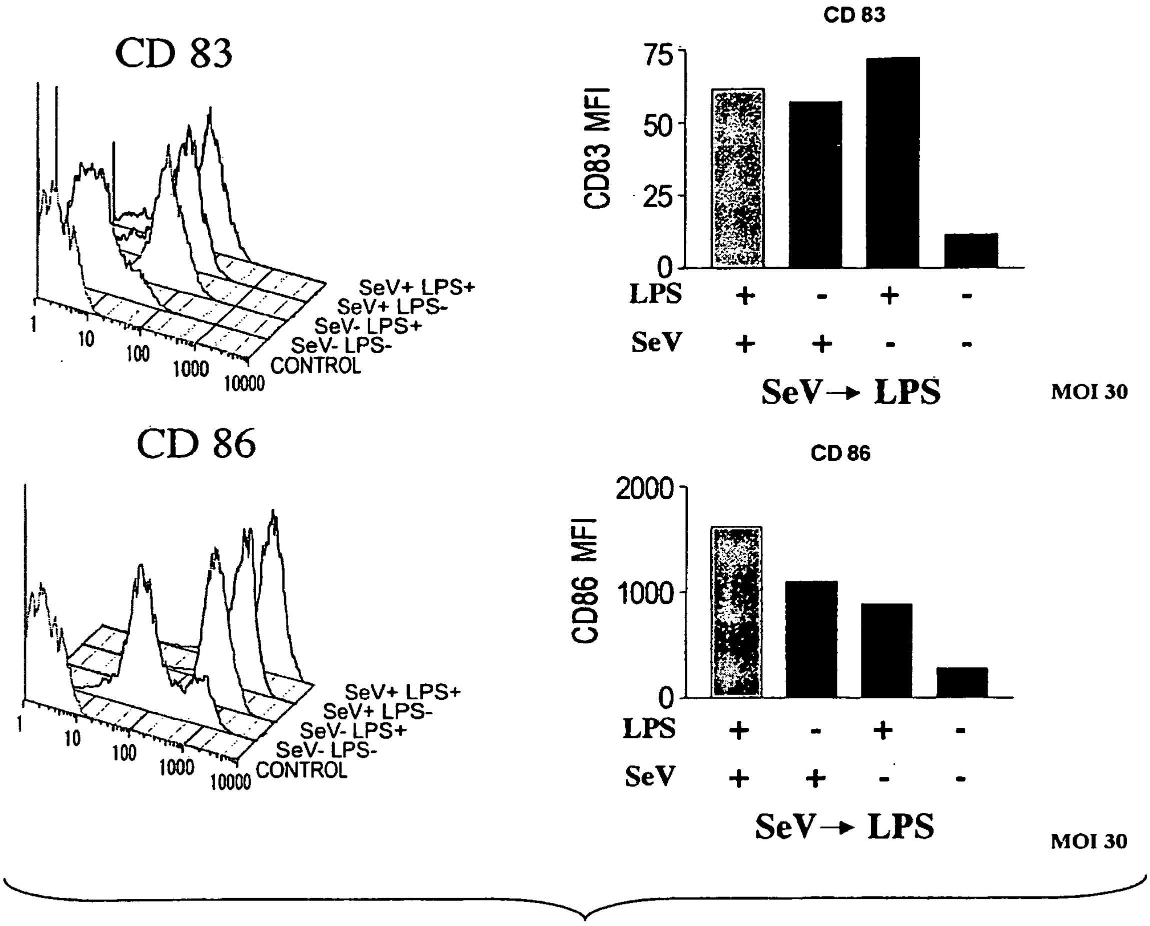
CD 83
SeV+ LPS+
SeV+ LPS-
SeV- LPS+
SeV- LPS-
CONTROL
1
10
100
1000
10000
CD 83
CD83 MFI
75
50
25
0
LPS + - + -
SeV + + - -
SeV→ LPS
MOI 30
CD 86
SeV+ LPS+
SeV+ LPS-
SeV- LPS+
SeV- LPS-
CONTROL
1
10
100
1000
10000
CD 86
CD86 MFI
2000
1000
0
LPS + - + -
SeV + + - -
SeV→ LPS
MOI 30

FIG. 15

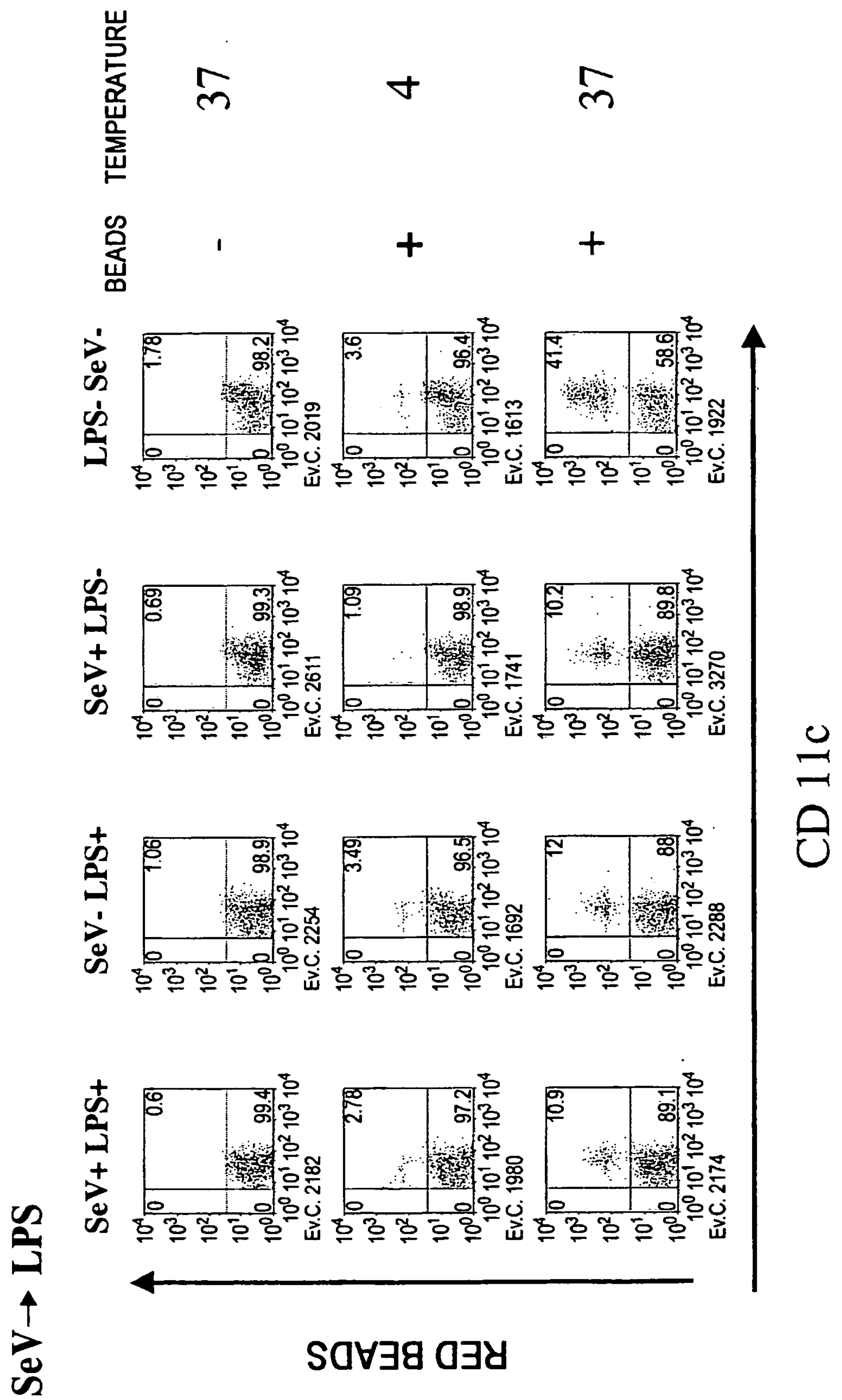
BEADS
TEMPERATURE
-
37
+
4
+
37
LPS- SeV-
SeV+ LPS-
SeV- LPS+
SeV+ LPS+
1.78
98.2
Ev.C. 2019
3.6
96.4
Ev.C. 1613
41.4
58.6
Ev.C. 1922
0.69
99.3
Ev.C. 2611
1.09
98.9
Ev.C. 1741
10.2
89.8
Ev.C. 3270
1.06
98.9
Ev.C. 2254
3.49
96.5
Ev.C. 1692
12
88
Ev.C. 2288
0.6
99.4
Ev.C. 2182
2.78
97.2
Ev.C. 1980
10.9
89.1
Ev.C. 2174
CD 11c
RED BEADS
SeV→ LPS

FIG. 16

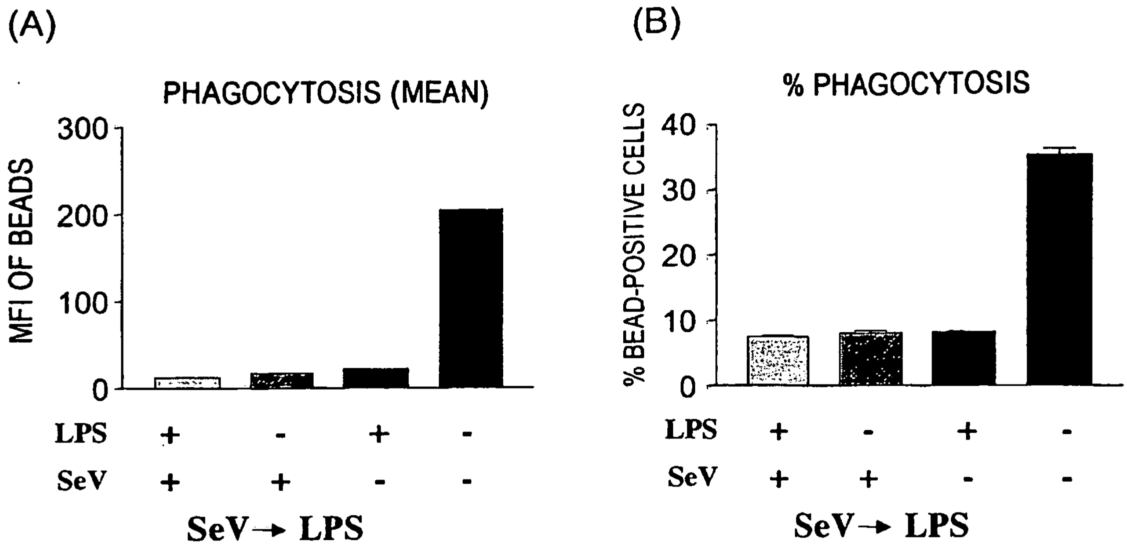
(A)
PHAGOCYTOSIS (MEAN)
MFI OF BEADS
300
200
100
0
LPS + - + -
SeV + + - -
SeV→ LPS
(B)
% PHAGOCYTOSIS
% BEAD-POSITIVE CELLS
40
30
20
10
0
LPS + - + -
SeV + + - -
SeV→ LPS

FIG. 17

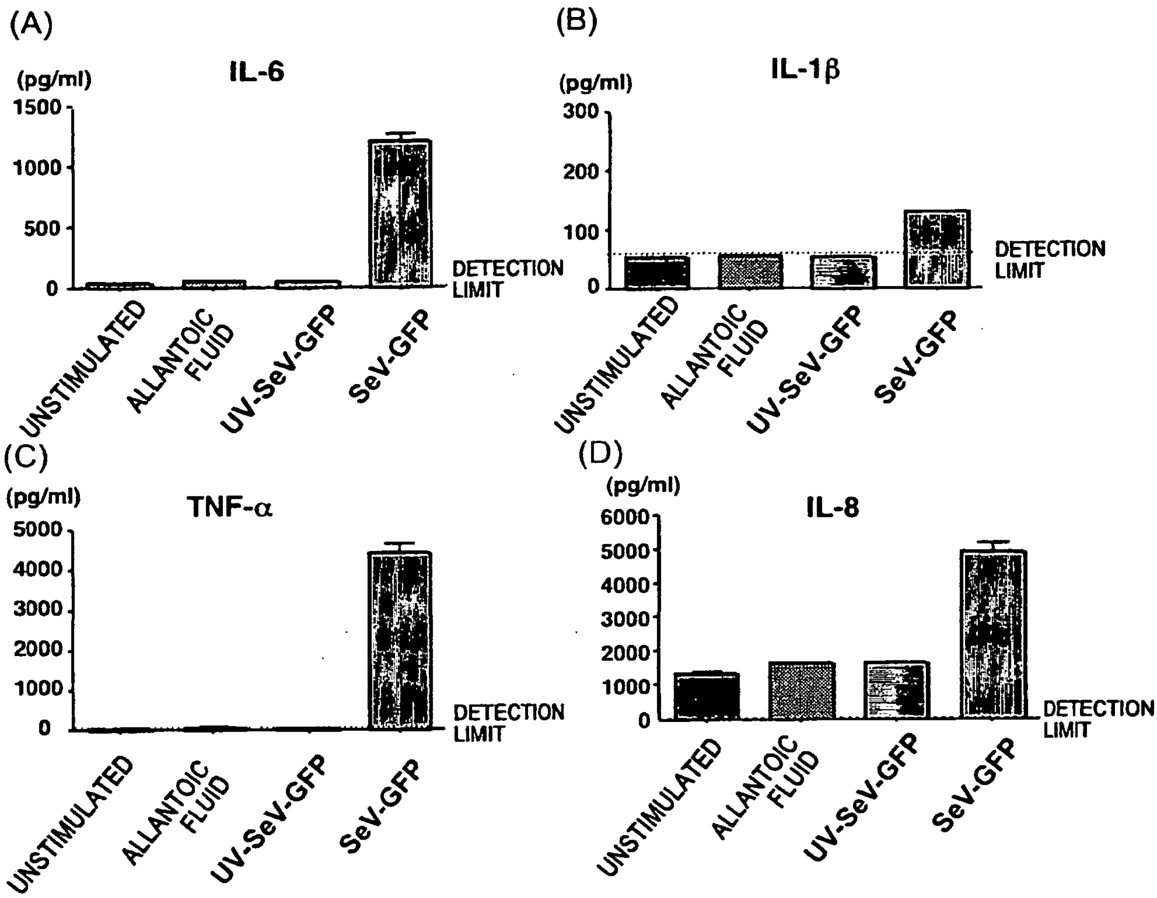
(A)
IL-6
(pg/ml)
1500
1000
500
0
DETECTION LIMIT
UNSTIMULATED
ALLANTOIC FLUID
UV-SeV-GFP
SeV-GFP
(B)
IL-1β
(pg/ml)
300
200
100
0
DETECTION LIMIT
UNSTIMULATED
ALLANTOIC FLUID
UV-SeV-GFP
SeV-GFP
(C)
TNF-α
(pg/ml)
5000
4000
3000
2000
1000
0
DETECTION LIMIT
UNSTIMULATED
ALLANTOIC FLUID
UV-SeV-GFP
SeV-GFP
(D)
IL-8
(pg/ml)
6000
5000
4000
3000
2000
1000
0
DETECTION LIMIT
UNSTIMULATED
ALLANTOIC FLUID
UV-SeV-GFP
SeV-GFP

FIG. 18

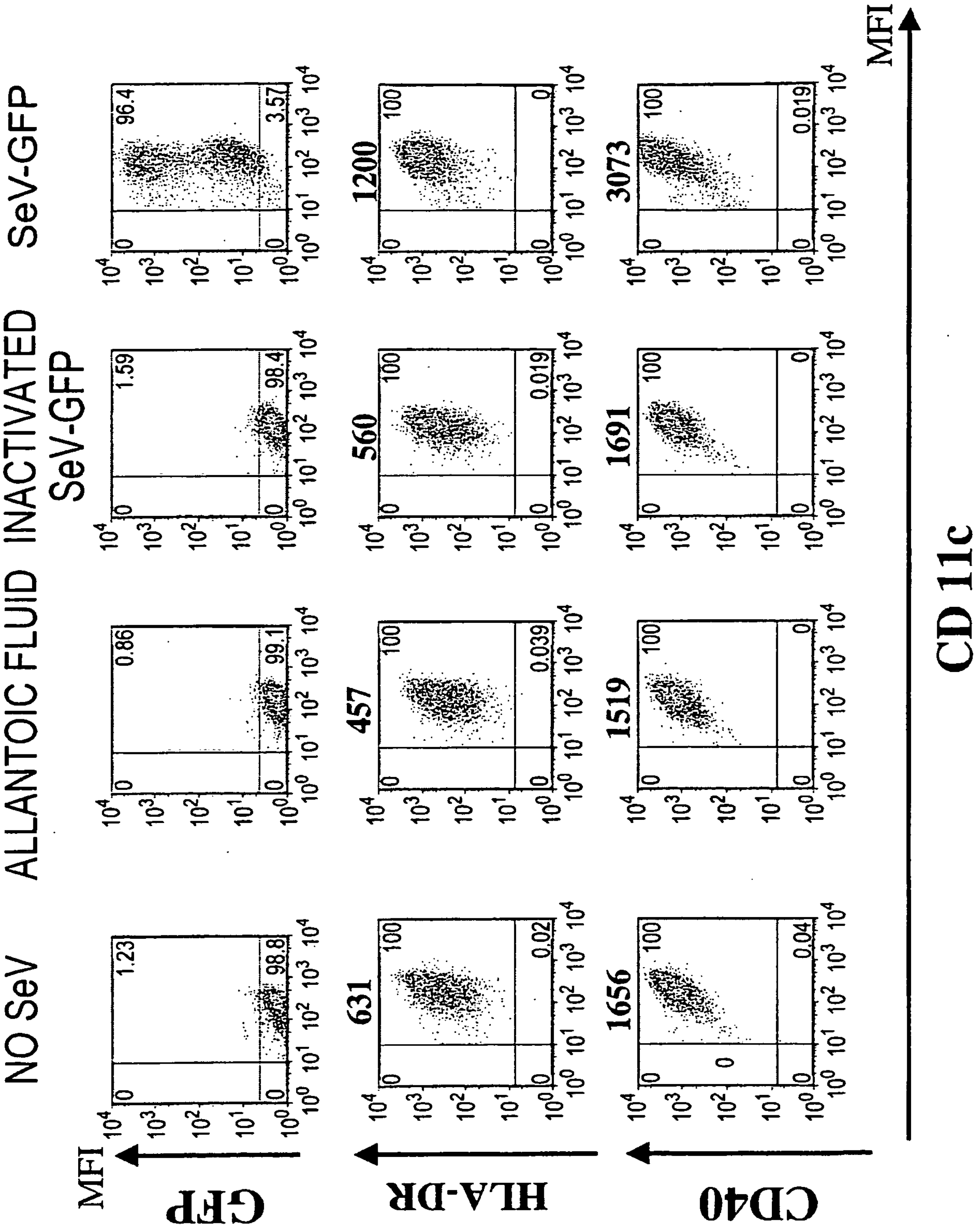
NO SeV
ALLANTOIC FLUID
UV-INACTIVATED SeV-GFP
SeV-GFP
GFP
HLA-DR
CD40
MFI
CD 11c
MFI
631
457
560
1200
1656
1519
1691
3073

FIG. 19

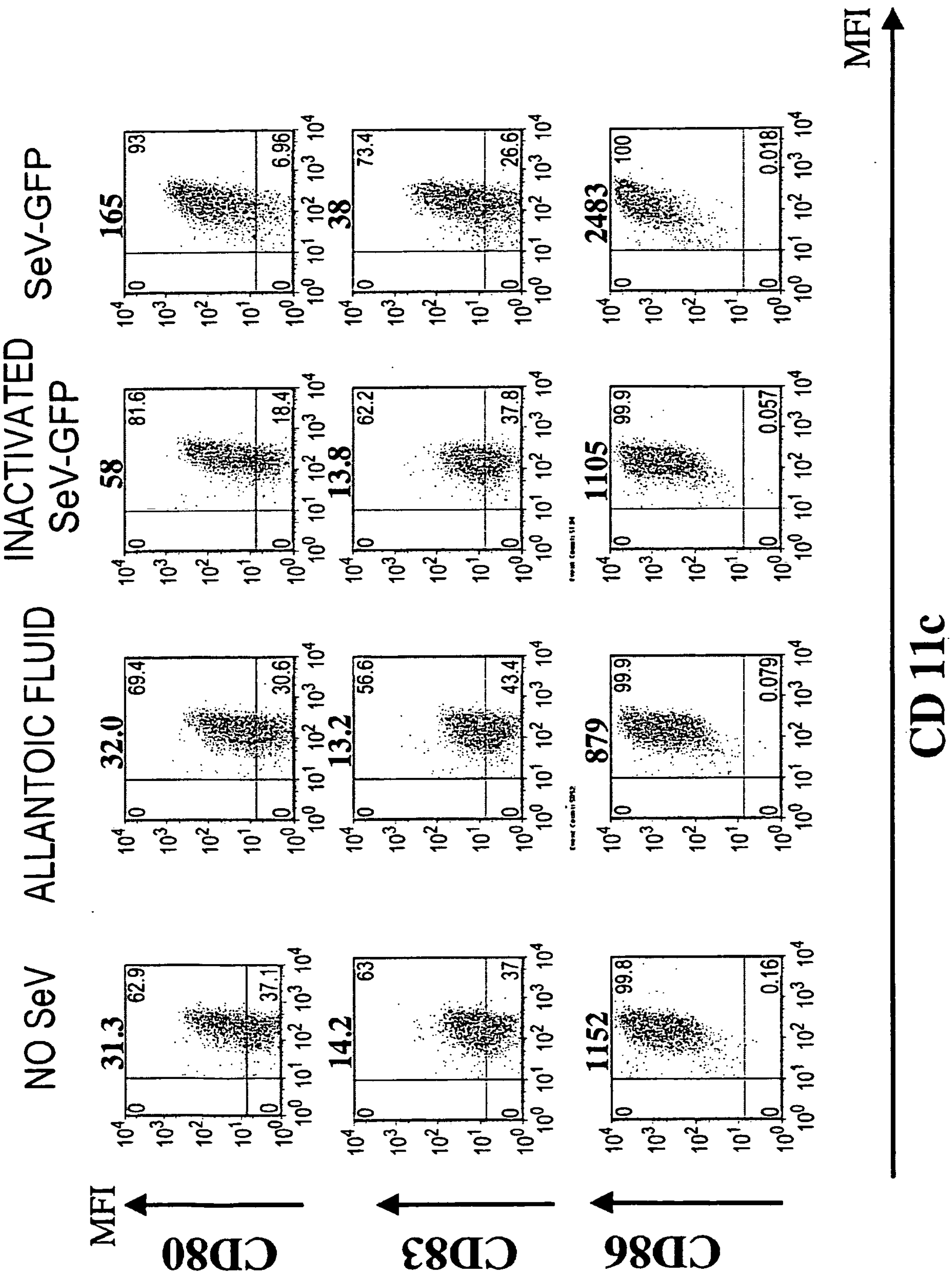
NO SeV
ALLANTOIC FLUID
UV-INACTIVATED SeV-GFP
SeV-GFP
CD80
CD83
CD86
MFI
CD 11c
MFI
31.3
14.2
1152
32.0
13.2
879
58
13.8
1105
165
38
2483

FIG. 20

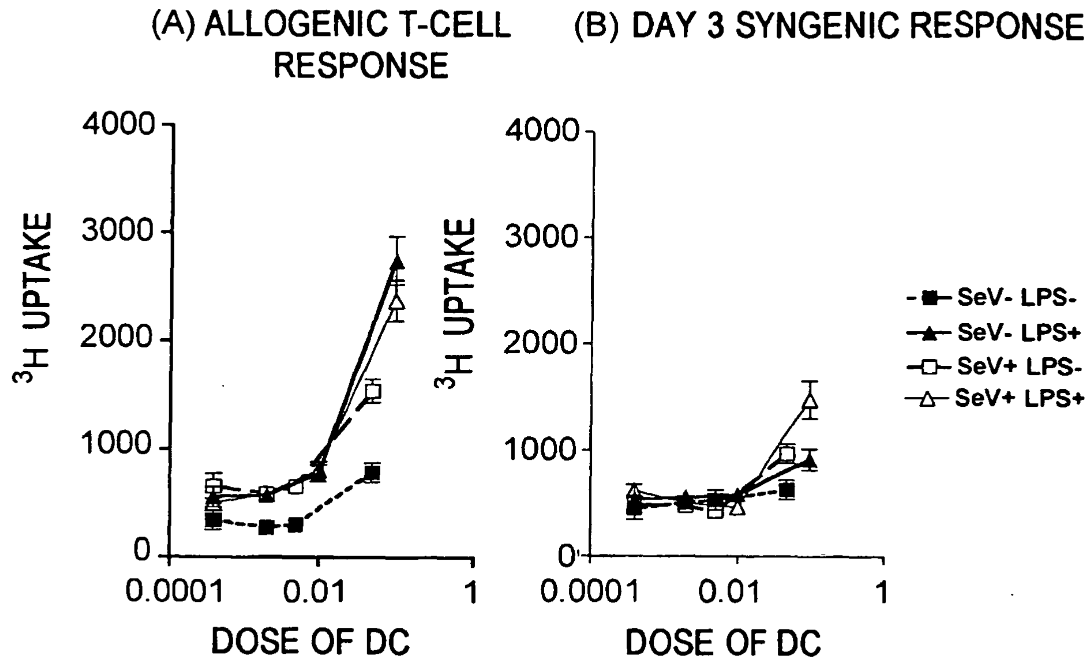
(A) ALLOGENIC T-CELL RESPONSE
(B) DAY 3 SYNGENIC RESPONSE
4000
3000
2000
1000
0
$^3$H UPTAKE
0.0001
0.01
1
DOSE OF DC
SeV- LPS-
SeV- LPS+
SeV+ LPS-
SeV+ LPS+

FIG. 21

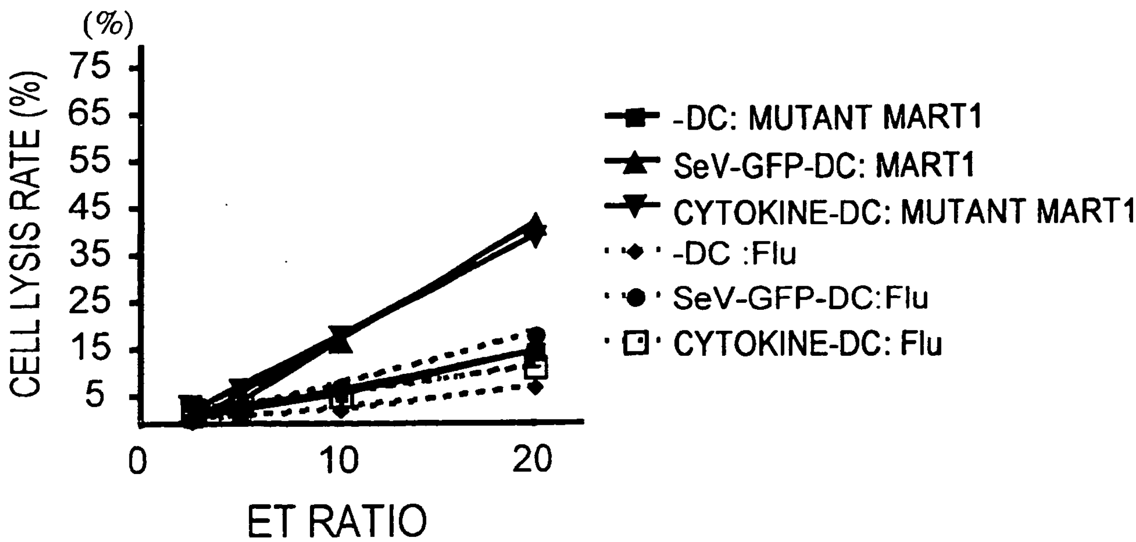
(%)
CELL LYSIS RATE (%)
75
65
55
45
35
25
15
5
0
10
20
ET RATIO
-DC: MUTANT MART1
SeV-GFP-DC: MART1
CYTOKINE-DC: MUTANT MART1
-DC :Flu
SeV-GFP-DC:Flu
CYTOKINE-DC: Flu

FIG. 22

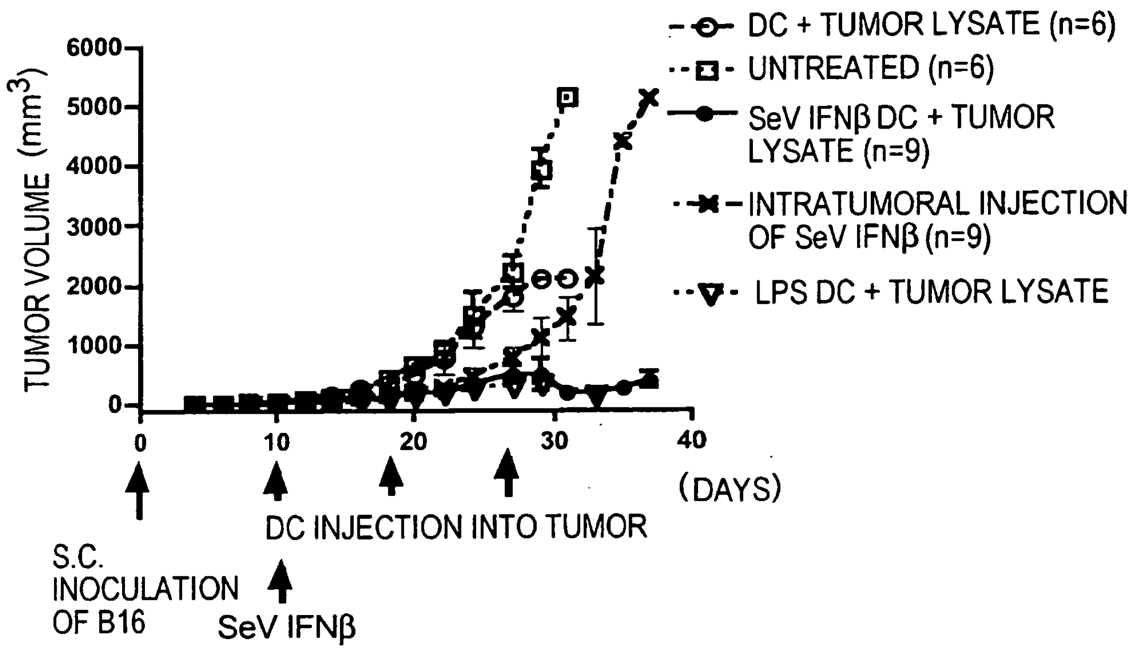
TUMOR VOLUME (mm3)
6000
5000
4000
3000
2000
1000
0
0
10
20
30
40
(DAYS)
DC + TUMOR LYSATE (n=6)
UNTREATED (n=6)
SeV IFNβ DC + TUMOR LYSATE (n=9)
INTRATUMORAL INJECTION OF SeV IFNβ (n=9)
LPS DC + TUMOR LYSATE
DC INJECTION INTO TUMOR
S.C. INOCULATION OF B16
SeV IFNβ

FIG. 23

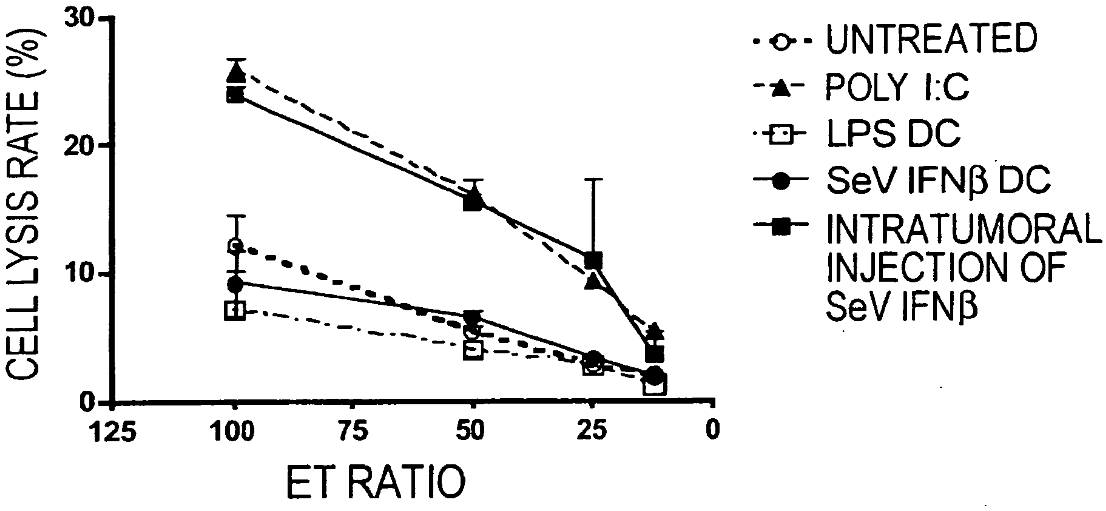
CELL LYSIS RATE (%)
30
20
10
0
125
100
75
50
25
0
ET RATIO
UNTREATED
POLY I:C
LPS DC
SeV IFNβ DC
INTRATUMORAL INJECTION OF SeV IFNβ

FIG. 24

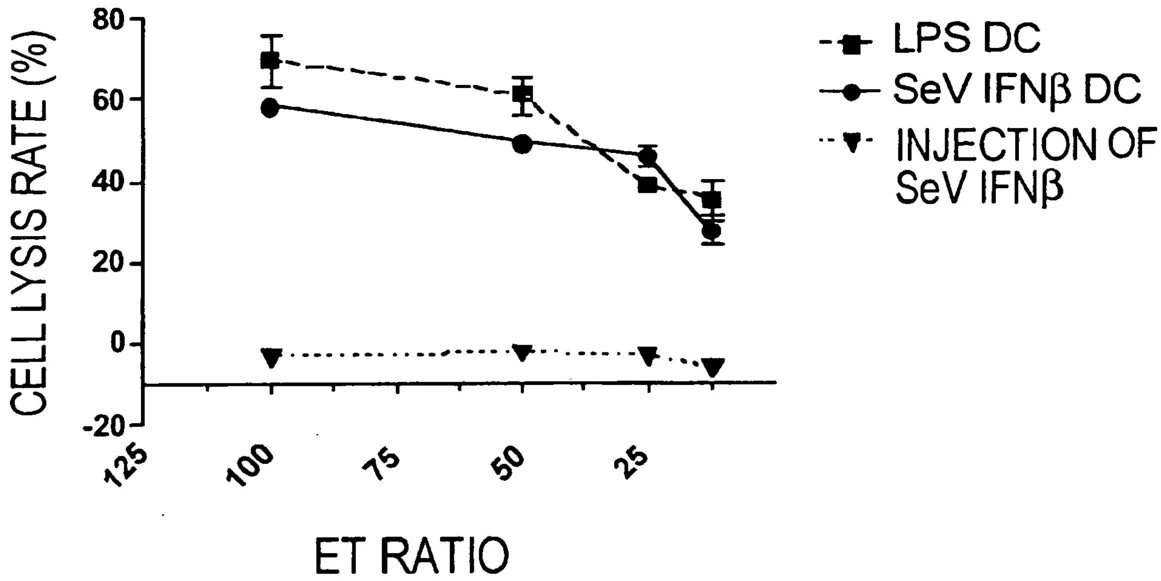
CELL LYSIS RATE (%)
80
60
40
20
0
-20
125
100
75
50
25
ET RATIO
LPS DC
SeV IFNβ DC
INJECTION OF SeV IFNβ

FIG. 25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0070055 A
- WO 0070070 A
- WO 0104272 A, Curran, J.
- EP 1067179 A
- WO 0118223 A
- WO 9716539 A
- WO 9716538 A
- JP S6230752 B
- JP S6233879 B
- JP S6230753 B
- WO 9732010 A
- US 5798100 A
- JP S6474982 A
- JP H1285498 A
- JP H5252965 A
- JP H5192160 A
- JP H7502173 A
- US 5744144 A
- JP 2003374808 A


### Non-patent literature cited in the description


- **STEINMAN, R. M.** *Ann. Rev. Immunol.,* 1991, vol. 9, 271
- **BANCHEREAU, J.B. ; STEINMAN R.M.** *Nature,* 1998, vol. 392, 245
- *Gene Therapy,* 2000, vol. 7, 2113-2121
- *J Immunol.,* 2000, vol. 164, 161-167
- **BANCHEREAU, J.B. ; R.M. STEINMAN.** *Nature,* 1998, vol. 392, 245
- **AKIYAMA, Y. et al.** *Gene Therapy,* 2000, vol. 7, 2113-2121
- **MIN, W.P.** *J. Immunol.,* 2000, vol. 164, 161-167
- **HSU, F.J. et al.** *Nat. Med.,* 1996, vol. 2, 52-58
- **NESTLE, F.O. et al.** *Nat. Med.,* 1998, vol. 4, 328-332
- **CAMPOREALE, A. et al.** *Cancer. Res.,* 2003, vol. 63, 3688-3694
- **BON, L. A. et al.** *Nat. Immunol.,* 2003, vol. 4, 1009-1015
- **XIA, D.J. et al.** *Gene Therapy,* 2002, vol. 9, 592-601
- **MULLINS, D.W. et al.** *J. Exp. Med.,* 2003, vol. 198, 1023-1034
- **OKADA, T. et al.** *Gene Therapy,* 2003, vol. 10, 1891-1902
- **NAKAHARA, S. et al.** *Cancer Res.,* 2003, vol. 63, 4112-4118
- **TEITZ-TENNENBAUM, S. et al.** *Cancer Res.,* 2003, vol. 63, 8466-8475
- **IMBODEN, M. et al.** *Cancer Res.,* 2001, vol. 61, 1500-1507
- **GOLDSZMID, R. S. et al.** *J. Immunol.,* 2003, vol. 171, 5940-5947
- **STROME, S.E. et al.** *Cancer Res.,* 2002, vol. 62, 1884-1889
- *Cancer Gene Ther,* 1997, vol. 4, 17-25
- *J. Immunotherapy,* 2002, vol. 25, 445-454
- *Gene Therapy,* 2000, vol. 7, 249-254
- *J. Leuko. Biol.,* 1999, 263-267
- *Br. J. Haematol.,* 2000, vol. 108, 817-824
- *J. Gene Med.,* 2001, vol. 3, 311-320
- *J. Immunol. Meth.,* 2002, 153-165
- *Mol. Ther.,* 2002, 283-290
- *Cancer Gene Therapy,* 2002, vol. 9, 715-724
- *J. Virol.,* vol. 75, 5448-5456
- *Arch Dermatol Res,* 2000, vol. 292, 325-332
- Leucocyte Typing IV: White Cell Differentiation Antigens. Oxford University Press, 1989
- The Leucocyte Antigen Facts Book, CD11 Section. Academic Press Inc, 1993, 124
- **STACKER, S.A. ; T.A. SPRINGER.** *J. Immunol.,* 1991, vol. 146, 648
- Leucocyte Typing V: White Cell Differentiation Antigens. Oxford University Press, 1995
- **HANAU, D. et al.** *J. Investigative Dermatol.,* 1990, vol. 95, 503
- **CALABI, F. ; A. BRADBURY.** *Tissue Antigens,* 1991, vol. 37, 1
- Leucocyte Typing III: White Cell Differentiation Antigens. Oxford University Press, 1987
- **WRIGHT, S.D. et al.** *Science,* 1990, vol. 249, 1434
- **GALY, A.H.M. ; H. SPITS.** *J. Immunol.,* 1992, vol. 149, 775
- **CLARK, E.A.** *Proc. Natl. Acad. Sci.,* 1986, vol. 83, 4494

- **ITOH, H. et al.** *Cell,* vol. 66, 233
- **BARCLAY, N.A. et al.** The Leucocyte Antigen Facts Book. Academic Press, 1993
- **SCHWARTS, R.H.** *Cell,* 1992, vol. 71, 1065
- **AZUMA, M. et al.** *J. Exp. Med.,* 1993, vol. 177, 845
- **KOULOVA, L. et al.** *J. Exp. Med.,* 1991, vol. 173, 759
- **FREEMAN, G.J. et al.** *J. Immunol.,* 1998, vol. 161, 2708
- **BEHRENS, L. et al.** *J. Immunol.,* 1998, vol. 161 (11), 5943
- **GUESDON, J.-L. et al.** *J. Histochem. Cytochem.,* 1979, vol. 27, 1131-1139
- **ZHOU, L-J. ; T.F. TEDDER.** *J. Immunol.,* 1995, vol. 154, 3821
- **ZHOU, L-J. et al.** *J. Immunol.,* 1992, vol. 149, 735
- **SUMMERS, K.L. et al.** *Clin Exp. Immunol.,* 1995, vol. 100, 81
- **WEISSMAN, D. et al.** *Proc. Natl. Acad. Sci USA.,* 1995, vol. 92, 826
- **HART, D.N.J.** *Blood,* 1997, vol. 90, 3245
- **AZUMA M. et al.** *Nature,* 1993, vol. 366, 76
- **NOZAWA Y et al.** *J. Pathology,* 1993, vol. 169, 309
- **ENGLE, P. et al.** *Blood,* 1994, vol. 84, 1402
- CD86 Workshop Report. **ENGEL, P. et al.** Leukocyte Typing V. Oxford University Press, 1994
- **YANG, X.F. et al.** Upregulation of CD86 antigen on TPA stimulated U937 cells. American Society of Hematology, 1994
- **SALLUSTO, F. et al.** *Nature,* 1999, vol. 401, 708-12
- **LIPP, M. et al.** *Curr. Top. Microbiol. Immunol.,* 2000, vol. 251, 173-9
- **BIRKENBACH, M. et al.** *J. Virol.,* 1993, vol. 67, 2209-20
- **SCHWEICKART, V. L. et al.** *Genomics,* 1994, vol. 23, 643-50
- **BURGSTAHLER, R. et al.** *Biochem. Biophys. Res. Commun.,* 1995, vol. 215, 737-43
- **YOSHIDA, R. et al.** *J. Biol. Chem.,* 1997, vol. 272, 13803-9
- **YOSHIDA, R. et al.** *J. Biol. Chem.,* 1998, vol. 273, 7118-22
- **YOSHIDA, R. et al.** *Int. Immunol.,* 1998, vol. 10, 901-10
- **KIM, C. H. et al.** *J. Immunol.,* 1998, vol. 161, 2580-5
- **YANAGIHARA, S. et al.** *J. Immunol.,* 1998, vol. 161, 3096-102
- **PAWELEC, G. et al.** *Human Immunology,* 1985, vol. 12, 165
- **ZIEGLER, A. et al.** *Immunobiol,* 1986, vol. 171, 77
- **BARCLAY, N.A. et al.** The Leucocyte Antigen Facts Book. Academic Press, 1993, 376
- **KIERTSCHER SM ; ROTH MD.** Human CD14+ leukocytes acquire the phenotype and function of antigen-presenting dendritic cells when cultured in GM-CSF and IL-4. *J. Leukoc. Biol.,* 1996, vol. 59 (2), 208-18
- **OEHLER, L. et al.** Neutrophil granulocyte-committed cells can be driven to acquire dendritic cell characteristics. *J. Exp. Med.,* 1998, vol. 187 (7), 1019-28
- **OKANO, S. et al.** Recombinant Sendai virus vectors for activated T lymphocytes. *Gene Ther.,* 2003, vol. 10 (16), 1381-91
- **STITES, D. et al.** Flow cytometric analysis of lymphocyte phenotypes in AIDS using monoclonal antibodies and simultaneous dual immunofluorescence. *Clin. Immunol. Immunopathol.,* 1986, vol. 38, 161-177
- *J. Exp. Med.,* 1998, vol. 187, 1019-1028
- *Blood,* 1996, vol. 87, 4520-4530
- **HSU et al.** *Nature Med.,* 1996, vol. 2, 52
- **CAMERON et al.** *Science,* 1992, vol. 257, 383
- **LANGHOFF et al.** *Proc. Natl. Acad. Sci. USA,* 1991, vol. 88, 7998
- **CHEHIMI et al.** *J. Gen. Virol.,* 1993, vol. 74, 1277
- **CAMERON et al.** *Clin. Exp. Immunol.,* 1992, vol. 88, 226
- **THOMAS et al.** *J. Immunol.,* 1993, vol. 150, 821
- **KARHUMAKI et al.** *Clin. Exp. Immunol.,* 1993, vol. 91, 482
- **THOMAS et al.** *J. Immunol.,* 1994, vol. 153, 4016
- **FERBAS et al.** *J. Immunol.,* 1994, vol. 152, 4649
- **O'DOHERTY et al.** *Immunology,* 1994, vol. 82, 487
- **MILTENYI et al.** *Cytometry,* 1990, vol. 11, 231-238
- **MACATONIA et al.** *Immunol.,* 1991, vol. 74, 399-406
- **O'DOHERTY et al.** *J. Exp. Med.,* 1993, vol. 178, 1067-1078
- **MARKOWICZ et al.** *J. Clin. Invest.,* 1990, vol. 85, 955-961
- **ROMANI et al.** *J. Exp. Med.,* 1994, vol. 180, 83-93
- **SALLUSTO et al.** *J. Exp. Med.,* vol. 179, 1109-1118
- **BERHARD et al.** *J. Exp. Med.,* 1995, vol. 55, 1099-1104
- **VAN TENDELOO et al.** *Gene Ther.,* 1998, vol. 5, 700-707
- **Y. NAGAI ; A. KATO.** *Microbiol. Immunol.,* 1999, vol. 43, 613-624
- **LI, H.-O. et al.** *J. Virol.,* 2000, vol. 74 (14), 6564-6569
- *J. Virology,* 1981, vol. 39, 519-528

- **NAVIAUX, R. K. et al.** *J. Virol.,* 1996, vol. 70, 5701-5705
- **KATO, A. et al.** *J. Virol.,* 1997, vol. 71, 7266-7272
- **KATO, A. et al.** *EMBO J.,* 1997, vol. 16, 578-587
- Paramyxoviridae: The viruses and their replication. **LAMB, R.A. ; KOLAKOFSKY, D.** Fields of Virology. Lippincott - Raven Publishers, 1996, vol. 2, 1177-1204
- **YU, D. et al.** *Genes Cells,* 1997, vol. 2, 457-466
- **HURWITZ, J.L. et al.** *Vaccine,* 1997, vol. 15, 533-540
- *Journal of Virology,* 1993, vol. 67 (8), 4822-4830
- **HASAN, M. K. et al.** *J. Gen. Virol.,* 1997, vol. 78, 2813-2820
- **KOLAKOFSKI, D. et al.** *J. Virol.,* 1998, vol. 72, 891-899
- **CALAIN, P. ; ROUX, L.** *J. Virol.,* 1993, vol. 67, 4822-4830
- **HASAN, M. K. et al.** *J. General Virology,* 1997, vol. 78, 2813-2820
- **DURBIN, A. P. et al.** *Virology,* 1997, vol. 235, 323-332
- **WHELAN, S. P. et al.** *Proc. Natl. Acad. Sci. USA,* 1995, vol. 92, 8388-8392
- **SCHNELL. M. J. et al.** *EMBO J.,* 1994, vol. 13, 4195-4203
- **RADECKE, F. et al.** *EMBO J.,* 1995, vol. 14, 5773-5784
- **LAWSON, N. D. et al.** *Proc. Natl. Acad. Sci. USA,* vol. 92, 4477-4481
- **GARCIN, D. et al.** *EMBO J.,* 1995, vol. 14, 6087-6094
- **KATO, A. et al.** *Genes Cells,* 1996, vol. 1, 569-579
- **BARON, M. D. ; BARRETT, T.** *J. Virol.,* 1997, vol. 71, 1265-1271
- **BRIDGEN, A. ; ELLIOTT, R. M.** *Proc. Natl. Acad. Sci. USA,* 1996, vol. 93, 15400-15404
- **GRAHAM, F. L. ; VAN DER EB, J.** *Virology,* 1973, vol. 52, 456
- **WIGLER, M. ; SILVERSTEIN, S.** *Cell,* 1977, vol. 11, 223
- **CHEN, C. ; OKAYAMA, H.** *Mol. Cell. Biol.,* 1987, vol. 7, 2745
- **CALOS, M. P.** *Proc. Natl. Acad. Sci. USA,* 1983, vol. 80, 3015
- **FUERST, T. R. et al.** *Proc. Natl. Acad. Sci. USA,* 1986, vol. 83, 8122-8126
- Hemaggulutinating virus of Japan-liposome-mediated gene delivery to vascular cells. **YONEMITSU, Y. ; KANEDA, Y.** Molecular Biology of Vascular Diseases. Method in Molecular Medicine. Humana Press, 1999, 295-306
- State-of-the-Art Technology Protocol in Neuroscience Research III, Molecular Neuron Physiology. Koseisha, Osaka. 1993, 153-172
- **TASHIRO, M.** Virus Experiment Protocol. Medical View Co., Ltd, 1995, 68-73
- **HASAN, M. K et al.** *J. General Virology,* 1997, vol. 78, 2813-2820
- **ARAI, T. et al.** *J. Virology,* 1998, vol. 72, 1115-1121
- **SAITO et al.** *Nucl. Acids Res.,* 1995, vol. 23, 3816-3821
- **ARAI, T. et al.** *J. Virol,* 1998, vol. 72, 1115-1121
- **FUERST, T.R. et al.** *Proc. Natl. Acad. Sci. USA,* 1986, vol. 83, 8122-8126
- **KIYOTANI, K. et al.** *Virology,* 1990, vol. 177 (1), 65-74
- **J. I. MAYORDOMO et al.** *Nature Med.,* 1995, vol. 1 (12), 1279-1302
- *Proc. Natl. Acad. Sci. USA,* 1999, vol. 96 (15), 8591-8596
- *J. Neurosurgery,* 1999, vol. 90 (6), 1115-1124
- *Vaccine,* 1999, vol. 17 (15-16), 1869-1882
- *J. Immunology,* 2000, vol. 164, 4968-4978
- **ARAKAWA T et al.** *Nature Biotechnology,* vol. 16 (10), 934-8
- **ARAKAWA T et al.** *Nature Biotechnology,* 1998, vol. 16 (3), 292-7
- **LODMELL DL et al.** *Nature Medicine,* 1998, vol. 4 (8), 949-52
- *J. Med. Virol,* 2000, vol. 60, 200-204
- **KIM, C. et al.** *Cancer Immunol. Immunother.,* 1998, vol. 47, 90-96
- *Eur. J. Immunol.,* 2000, vol. 30, 3338-3346
- **COON, B. et al.** *J. Clin. Invest.,* 1999, vol. 104 (2), 189-94
- **ARAI et al.** *J. Immunol.,* 1989, vol. 142 (1), 274-282
- **YASUKAWA et al.** *EMBO J.,* 1987, vol. 6 (10), 2939-2945
- **WOLF et al.** *J. Immunol.,* 1991, vol. 146 (9), 3074-3081
- **GREN et al.** *J. Interferon Res.,* 1984, vol. 4 (4), 609-617
- **WEISMANN et al.** *Princess Takamatsu Symp.,* 1982, vol. 12, 1-22
- **PENNICA et al.** *Nature,* 1984, vol. 312, 724-729
- **HIRANO et al.** *Nature,* 1986, vol. 324, 73-76
- **CANTRELL et al.** *Proc. Natl. Acad. Sci. (USA,* 1985, vol. 82 (18), 6250-6254
- **DERYNCK, R. et al.** *Nature,* 1980, vol. 285, 542-547
- **HIGASHI, Y. et al.** *J. Biol. Chem.,* 1983, vol. 258, 9522-9529
- **KUGA, T. et al.** *Nucleic Acids Res.,* 1989, vol. 17, 3291

- **ALTSCHUL, S. F. et al.** *J. Mol. Biol.,* 1990, vol. 215, 403-410
- **ALTSCHUL, S.F. et al.** *Nature Genet.,* 1993, vol. 3, 266-272
- **MADDEN, T.L. et al.** *Meth. Enzymol.,* 1996, vol. 266, 131-141
- **ALTSCHUL, S.F. et al.** *Nucleic Acids Res.,* 1997, vol. 25, 3389-3402
- **ZHANG, J. ; MADDEN, T.L.** *Genome Res.,* 1997, vol. 7, 649-656
- **TATIANA A et al.** *FEMS Microbiol Lett.,* 1999, vol. 174, 247-250
- **KNEZIC, Z. et al.** *Antiviral Res.,* 1993, vol. 25, 215-221
- **RIDELL et al.** *Science,* 1992, vol. 257, 238-241

SEQUENCE LISTING

<110> DNAVEC RESEARCH INC.

<120> Method of Constructing Transgenic Dendritic cell

<130> D3-A0307Y1P

<140>
<141>

<150> JP 2003-374808
<151> 2003-11-04

<160> 15

<170> PatentIn version 3.1

<210> 1
<211> 10
<212> DNA
<213> Artificial

<220>
<223> artificially synthesized sequence

<400> 1
ctttcaccct 10

<210> 2
<211> 15

<212> DNA
<213> Artificial

<220>
<223> artificially synthesized sequence

<400> 2 15
tttttcttac tacgg 15

<210> 3
<211> 18
<212> DNA
<213> Artificial

<220>
<223> artificially synthesized sequence

<400> 3 18
cggccgcaga tcttcacg 18

<210> 4
<211> 18
<212> DNA
<213> Artificial

<220>
<223> artificially synthesized sequence

<400> 4 18
atgcatgccg gcagatga 18

<210> 5
<211> 18
<212> DNA
<213> Artificial

<220>
<223> artificially synthesized sequence

<400> 5 18
gttgagtact gcaagagc 18

<210> 6
<211> 42
<212> DNA
<213> Artificial

<220>
<223> artificially synthesized sequence

<400> 6 42 tttgccggca tgcatgtttc ccaaggggag agttttgcaa cc 42

<210> 7
<211> 18
<212> DNA
<213> Artificial

<220>

<223> artificially synthesized sequence

<400> 7 18 atgcatgccg gcagatga 18

<210> 8
<211> 21
<212> DNA
<213> Artificial

<220>
<223> artificially synthesized sequence

<400> 8
tgggtgaatg agagaatcag c 21

<210> 9
<211> 10
<212> PRT
<213> Artificial

<220>
<223> an artificially synthesized peptide

<400> 9

Glu Ala Ala Gly Ile Gly Ile Leu Thr Val
1 5 10

<210> 10
<211> 10
<212> PRT
<213> Artificial

<220>
<223> an artificially synthesized peptide

<400> 10

Glu Leu Ala Gly Ile Gly Ile Leu Thr Val
1 5 10

<210> 11
<211> 9
<212> PRT
<213> Artificial

<220>
<223> an artificially synthesized peptide

<400> 11

Gly Ile Leu Gly Phe Val Phe Thr Leu
1 5

<210> 12

<211> 561
<212> DNA
<213> Homo sapiens

<220>
<221> CDS
<222> (1).. (561)
<223>

<220>
<221> sig_peptide
<222> (1).. (21)
<223>

<400> 12

```
atg acc aac aag tgt ctc ctc caa att gct ctc ctg ttg tgc ttc tcc      48
Met Thr Asn Lys Cys Leu Leu Gln Ile Ala Leu Leu Leu Cys Phe Ser
1               5                   10                  15

act aca gct ctt tcc atg agc tac aac ttg ctt gga ttc cta caa aga      96
Thr Thr Ala Leu Ser Met Ser Tyr Asn Leu Leu Gly Phe Leu Gln Arg
            20                  25                  30

agc agc aat ttt cag tgt cag aag ctc ctg tgg caa ttg aat ggg agg     144
Ser Ser Asn Phe Gln Cys Gln Lys Leu Leu Trp Gln Leu Asn Gly Arg
        35                  40                  45

ctt gaa tat tgc ctc aag gac agg atg aac ttt gac atc cct gag gag     192
```

Leu Glu Tyr Cys Leu Lys Asp Arg Met Asn Phe Asp Ile Pro Glu Glu
50 55 60

att aag cag ctg cag cag ttc cag aag gag gac gcc gca ttg acc atc 240
Ile Lys Gln Leu Gln Gln Phe Gln Lys Glu Asp Ala Ala Leu Thr Ile
65 70 75 80

tat gag atg ctc cag aac atc ttt gct att ttc aga caa gat tca tct 288
Tyr Glu Met Leu Gln Asn Ile Phe Ala Ile Phe Arg Gln Asp Ser Ser
85 90 95

agc act ggc tgg aat gag act att gtt gag aac ctc ctg gct aat gtc 336
Ser Thr Gly Trp Asn Glu Thr Ile Val Glu Asn Leu Leu Ala Asn Val
100 105 110

tat cat cag ata aac cat ctg aag aca gtc ctg gaa gaa aaa ctg gag 384
Tyr His Gln Ile Asn His Leu Lys Thr Val Leu Glu Glu Lys Leu Glu
115 120 125

aaa gaa gat ttt acc agg gga aaa ctc atg agc agt ctg cac ctg aaa 432
Lys Glu Asp Phe Thr Arg Gly Lys Leu Met Ser Ser Leu His Leu Lys
130 135 140

aga tat tat ggg agg att ctg cat tac ctg aag gcc aag gag tac agt 480
Arg Tyr Tyr Gly Arg Ile Leu His Tyr Leu Lys Ala Lys Glu Tyr Ser
145 150 155 160

cac tgt gcc tgg acc ata gtc aga gtg gaa atc cta agg aac ttt tac 528
His Cys Ala Trp Thr Ile Val Arg Val Glu Ile Leu Arg Asn Phe Tyr
165 170 175

ttc att aac aga ctt aca ggt tac ctc cga aac 561
Phe Ile Asn Arg Leu Thr Gly Tyr Leu Arg Asn
180 185

<210> 13
<211> 187
<212> PRT
<213> Homo sapiens

<400> 13

Met Thr Asn Lys Cys Leu Leu Gln Ile Ala Leu Leu Leu Cys Phe Ser
1 5 10 15

Thr Thr Ala Leu Ser Met Ser Tyr Asn Leu Leu Gly Phe Leu Gln Arg
20 25 30

Ser Ser Asn Phe Gln Cys Gln Lys Leu Leu Trp Gln Leu Asn Gly Arg
35 40 45

Leu Glu Tyr Cys Leu Lys Asp Arg Met Asn Phe Asp Ile Pro Glu Glu
50 55 60

Ile Lys Gln Leu Gln Gln Phe Gln Lys Glu Asp Ala Ala Leu Thr Ile
65 70 75 80

Tyr Glu Met Leu Gln Asn Ile Phe Ala Ile Phe Arg Gln Asp Ser Ser
85 90 95

Ser Thr Gly Trp Asn Glu Thr Ile Val Glu Asn Leu Leu Ala Asn Val
100 105 110

Tyr His Gln Ile Asn His Leu Lys Thr Val Leu Glu Glu Lys Leu Glu
115 120 125

Lys Glu Asp Phe Thr Arg Gly Lys Leu Met Ser Ser Leu His Leu Lys
130 135 140

Arg Tyr Tyr Gly Arg Ile Leu His Tyr Leu Lys Ala Lys Glu Tyr Ser
145 150 155 160

His Cys Ala Trp Thr Ile Val Arg Val Glu Ile Leu Arg Asn Phe Tyr
165 170 175

Phe Ile Asn Arg Leu Thr Gly Tyr Leu Arg Asn
180 185

<210> 14
<211> 546
<212> DNA
<213> Mus musculus

<220>
<221> CDS
<222> (1).. (546)
<223>

<220>
<221> sig_peptide
<222> (1).. (21)
<223>

<400> 14

```
atg aac aac agg tgg atc ctc cac gct gcg ttc ctg ctg tgc ttc tcc      48
Met Asn Asn Arg Trp Ile Leu His Ala Ala Phe Leu Leu Cys Phe Ser
1               5                   10                  15

acc aca gcc ctc tcc atc aac tat aag cag ctc cag ctc caa gaa agg      96
Thr Thr Ala Leu Ser Ile Asn Tyr Lys Gln Leu Gln Leu Gln Glu Arg
            20                  25                  30

acg aac att cgg aaa tgt cag gag ctc ctg gag cag ctg aat gga aag      144
Thr Asn Ile Arg Lys Cys Gln Glu Leu Leu Glu Gln Leu Asn Gly Lys
        35                  40                  45

atc aac ctc acc tac agg gcg gac ttc aag atc cct atg gag atg acg      192
Ile Asn Leu Thr Tyr Arg Ala Asp Phe Lys Ile Pro Met Glu Met Thr
    50                  55                  60
```

gag aag atg cag aag agt tac act gcc ttt gcc atc caa gag atg ctc 240
Glu Lys Met Gln Lys Ser Tyr Thr Ala Phe Ala Ile Gln Glu Met Leu
65 70 75 80

cag aat gtc ttt ctt gtc ttc aga aac aat ttc tcc agc act ggg tgg 288
Gln Asn Val Phe Leu Val Phe Arg Asn Asn Phe Ser Ser Thr Gly Trp
85 90 95

aat gag act att gtt gta cgt ctc ctg gat gaa ctc cac cag cag aca 336
Asn Glu Thr Ile Val Val Arg Leu Leu Asp Glu Leu His Gln Gln Thr
100 105 110

gtg ttt ctg aag aca gta cta gag gaa aag caa gag gaa aga ttg acg 384
Val Phe Leu Lys Thr Val Leu Glu Glu Lys Gln Glu Glu Arg Leu Thr
115 120 125

tgg gag atg tcc tca act gct ctc cac ttg aag agc tat tac tgg agg 432
Trp Glu Met Ser Ser Thr Ala Leu His Leu Lys Ser Tyr Tyr Trp Arg
130 135 140

gtg caa agg tac ctt aaa ctc atg aag tac aac agc tac gcc tgg atg 480
Val Gln Arg Tyr Leu Lys Leu Met Lys Tyr Asn Ser Tyr Ala Trp Met
145 150 155 160

gtg gtc cga gca gag atc ttc agg aac ttt ctc atc att cga aga ctt 528
Val Val Arg Ala Glu Ile Phe Arg Asn Phe Leu Ile Ile Arg Arg Leu
165 170 175

acc aga aac ttc caa aac 546
Thr Arg Asn Phe Gln Asn
180

<210> 15
<211> 182
<212> PRT
<213> Mus musculus

<400> 15

Met Asn Asn Arg Trp Ile Leu His Ala Ala Phe Leu Leu Cys Phe Ser
1 5 10 15

Thr Thr Ala Leu Ser Ile Asn Tyr Lys Gln Leu Gln Leu Gln Glu Arg
20 25 30

Thr Asn Ile Arg Lys Cys Gln Glu Leu Leu Glu Gln Leu Asn Gly Lys
35 40 45

Ile Asn Leu Thr Tyr Arg Ala Asp Phe Lys Ile Pro Met Glu Met Thr
50 55 60

Glu Lys Met Gln Lys Ser Tyr Thr Ala Phe Ala Ile Gln Glu Met Leu
65 70 75 80

Gln Asn Val Phe Leu Val Phe Arg Asn Asn Phe Ser Ser Thr Gly Trp
85 90 95

Asn Glu Thr Ile Val Val Arg Leu Leu Asp Glu Leu His Gln Gln Thr
100 105 110

Val Phe Leu Lys Thr Val Leu Glu Glu Lys Gln Glu Glu Arg Leu Thr
115 120 125

Trp Glu Met Ser Ser Thr Ala Leu His Leu Lys Ser Tyr Tyr Trp Arg
130 135 140

Val Gln Arg Tyr Leu Lys Leu Met Lys Tyr Asn Ser Tyr Ala Trp Met
145 150 155 160

Val Val Arg Ala Glu Ile Phe Arg Asn Phe Leu Ile Ile Arg Arg Leu
165 170 175

Thr Arg Asn Phe Gln Asn
180